# EUROPEAN PATENT APPLICATION

(11) **EP 2 199 399 A1**
(43) Date of publication of application: **23.06.2010**
(21) Application number: 08171888.4
(22) Date of filing: 17.12.2008
(51) Int. Cl.: C12N 15/82

(54) **Production of ketocarotenoids in plants**

(71) Applicant: BASF Plant Science GmbH, 67056 Ludwigshafen (DE)
(72) Inventor: Fachmann, Ralf, Dr., 06484 Quedlinburg (DE); Wenderoth, Irina, Dr., 67112 Mutterstadt (DE); Schopfer, Christel, Renate, Dr., 67061 Ludwigshafen (DE); Sauer, George, Mather, Dr., 06484 Quedlinburg (DE); Tschiersch, Bettina, Dr., 06484 Quedlinburg (DE); Bridg-Giannakopoulos, Hannia, Dr., 06484 Quedlinburg (DE); Leps, Michael, Dr., 38820 Halberstadt (DE)
(74) Representative: Popp, Andreas

(57) **Abstract**

The present invention relates to optimized ketolase coding sequences and corresponding genetic constructs comprising the same, their use for the expression in plants, in particular in plants of the genus Tagetes, to such genetically modified plants and to a process for the preparation of carotenoid products , in particular ketocarotenoid products by culturing the genetically modified plants.

## Description

The present invention relates to optimized ketolase coding sequences and corresponding genetic constructs comprising the same, their use for the expression in plants, in particular in plants of the genus Tagetes, to such genetically modified plants and to a process for the preparation of carotenoid products , in particular ketocarotenoid products by culturing the genetically modified plants.

### Technical Background

Carotenoids are synthesized de novo in bacteria, algae, fungi and plants. In recent years, it has increasingly been attempted also to utilize plants as production organisms for fine chemicals, in particular for vitamins and carotenoids.

A natural mixture of the carotenoids lutein and zeaxanthin is extracted, for example, from the flowers of marigold plants (Tagetes plants) as "oleoresin". This oleoresin is used both as an ingredient of food supplements and in the feed sector.

Lycopene from tomatoes is likewise used as a food supplement, while phytoene is mainly used in the cosmetic sector.

Ketocarotenoids, that is carotenoids which comprise at least one keto group, such as, for example, astaxanthin, canthaxanthin, echinenone, 3-hydroxyechinenone, 3'-hydroxyechinenone, adonirubin and adonixanthin are natural antioxidants and pigments which are produced by some algae, plants and microorganisms as secondary metabolites.

On account of their color-imparting properties, the ketocarotenoids and in particular astaxanthin are used as pigmenting aids in animal nutrition, in particular in trout, salmon and shrimp farming.

An economical biotechnological process for the production of natural, biosynthetic products and in particular carotenoids is therefore of great importance.

WO 98/18910 describes the synthesis of ketocarotenoids in nectar glands of tobacco flowers by introducing a ketolase gene into tobacco

WO 00/32788 describes some carotenoid biosynthesis genes from plants of the genus Tagetes and discloses how genetically modified plants of the genus Tagetes could be produced in order to obtain various carotenoid profiles in the petals and thus to produce certain carotenoids selectively. To this end, it was necessary to over-express some biosynthesis genes and to suppress others.

For the over expression of the newly found carotenoid biosynthesis genes in plants of the genus Tagetes, WO 00/32788 postulates the petal-specific promoter of the ketolase from Adonis vernalis.

Methods for the production of ketocarotenoids in plants with ketolase activity in petals are disclosed in W0 2004/018693. This previous application describes the expression of a beta carotene ketolase from different species in lutein-containing Tagetes plants. Lutein is the major carotenoid but is not being considered as the primary and desired substrate for beta-carotene ketolases to synthesize astaxanthin. Via silencing of the epsilon-cyclase gene expression, an early step in the lutein biosynthetic pathway, lutein concentrations were reduced and beta-carotenoids as substrates for ketolases increased, but only slightly. Those plants showing reduced lutein concentrations were crossed with plants accumulating low ketocarotenoid amounts. Thereby the accumulation of ketocarotenoids could be further increased.

There is a constant need to make available further improved methods for the production of carotenoids, in particular ketocarotenoids, in plants, as for example plants of the genus Tagetes.

### Summary of the invention

The above-mentioned problem could be solved by the present invention, which makes use of optimized carotene ketolase coding sequences, which result in a surprisingly favorable production of carotenoids, in particular ketocarotenoids, in plants, in particular flowers of plants, like those of the genus Tagetes.

### Detailed description of the invention:

### a) General definitions:

"Carotenoids": any carotenoid, in particular alpha- and beta-carotenoids and ketocarotenoids, in particular ketocarotenoids and mixtures thereof with alpha- and/or beta-carotenoids. For example, alpha carotenoids are selected from alpha-carotene, alphacryptoxanthin and/or lutein; beta-carotenoids are selected from beta-carotene, beta-cryptoxanthin, zeaxanthin, antheraxanthin, violaxanthin and/or neoxanthin; ketocarotenoids are selected from astaxanthin, adonixanthin, adonirubin, echinenone, 3'-hydroxyechinenone, 3-hydroxyechinenone and/or canthaxanthin without being restricted thereto.

A preferred group of carotenoids are ketocarotenoids, especially astaxanthin.

"Carotenoids" also comprises precursors thereof as for example geranylgeranylpyrophosphate, phytoenediphosphate and phytoene.

"Carotenoids" also comprise derivatives thereof, for example derivatives of ketocarotenoids, such as esters, for example ketocarotenoid esters. The "ester" of a carotenoid means any ester, for example mono-, di- and polyester, in particular diester or a mixture of various esters. Di- or polyesters may be derived from identical or different carboxylic acids.

Esters are, in particular, esters of fatty acids. Fatty acid esters are for example composed of straight-chain or branched, mono- or polyunsaturated, optionally substituted C₆-C₃₀ monocarboxylic acids. Examples of saturated unbranched fatty acids are caproic acid, enanthic acid, caprylic acid, pelargonic acid, capric acid, undecanoic acid, lauric acid, tridecanoic acid, myristic acid, pentadecanoic acid, palmitic acid, margaric acid, stearic acid, nonadecaonic acid, arachic acid, behenic acid, lignoceric acid, cerotitic acid and melissic acid. Examples of monounsaturated fatty acids are palmitoleic acid, oleic acid and erucic acid. Examples of disaturated fatty acids are sorbic acid and linoleic acid. Examples of triunsaturated fatty acids are linolenic acid and elaeostearic acid. Examples of tetra- and polyunsaturated fatty acids are arachidonic acid, clupanodonic acid and docosahexaeonic acid. Unbranched, saturated fatty acids are preferred. Also preferred are monobasic, saturated or mono-, di- or triunsaturated C₁₀₋₂₄, preferably C₁₂₋₂₀ or C₁₄₋₂₀ fatty acids.

Carotenoids such as ketocarotenoids and their derivatives, such as for example esters as defined above include these compounds both in isomerically pure form and in the form of mixtures of stereoisomers.

"Total carotenoids": the amount of all carotenoids and carotenoid esters as defined above.

"Ketolase enzyme": protein with the enzymatic activity of introducing a keto group at the optionally substituted beta-ionone ring of carotenoids, in particular, a protein with the enzymatic activity of converting beta-carotene into canthaxanthin.

"Ketolase activity": enzymatic activity of a ketolase enzyme. Accordingly, "ketolase activity" is understood as meaning the amount of beta-carotene converted, or the amount of canthaxanthin formed, by the protein ketolase within a certain period of time.

"Expression improved" or "expression optimized" or "optimized" coding sequence: sequence modified compared to the parent sequence in order to increase the expression of the encoded protein or enzyme in specific plants or part of plants such as plant plastids in comparison with the expression observed for the corresponding parent coding sequence encoding the same (i.e. substantially identical on the amino acid sequence level) protein or enzyme in the same plants or part of plants under substantially identical conditions. In particular, the parent coding sequence originates from a different, in particular non-plant, organism, and is therefore also called "heterologous coding sequence". An "expression improved" coding sequence is codon optimized with respect to the host organism or compartment such as a tissue or plastid. Additionally for example cryptic splice sites and/or cryptic polyadenylation signals may have been avoided in the codon optimization process or removed if present in the parent sequence. Secondary structures in the transcript interfering with the translational efficiency may have also been avoided and/or removed from the sequence.

"Codon optimized": the codon usage of the host plants, part of the host plants such as tissues or plastids is determined. Subsequently at least one codon of the non-improved sequence is changed into a codon encoding the same amino acid and having a higher abundance than the replaced codon in the host plants or plant compartments such as tissues or plastids. Preferentially, the codon with the highest abundance in the host plants or plant compartments such as tissues or plastids for the respective amino acid is chosen. More preferentially all codons of a non-improved sequence are optimized to the codon usage of the respective plants or plant compartments such as tissues or plastids.

"Non-improved" or "wild type" coding sequence, amino acid sequence or sequence: the sequence of a gene, protein or both as identified in any organism being unaltered in terms of the nucleic acid and/or amino acid sequence.

"Parent sequence": a sequence being used as a template for any sequence alteration such as for example deletions, insertions and/or substitutions, in particular for expression improvement such as for example codon optimization. A "parent sequence" usually is a "non-improved" or "wild type" sequence but may also mean a changed for example improved sequence which is used as a template for further improvement of the coding region.

"Expression improved heterologous carotene ketolase coding sequence" or, as used herein as a synonymous phrase, "improved ketolase sequence": a carotene ketolase coding sequence identified in a non host organism which codon usage has been optimized for the respective host plants or plant compartments such as tissues or plastids codon usage and/or where cryptic splice sites, polyadenylation signals and/or secondary structures of the transcript have been removed or avoided.

"Signals and/or structures negatively interfering with expression efficiency": any sequence interfering with the expression efficiency, for example cryptic polyadenylation signals, cryptic splice sites and/or secondary structures in the transcript that are hindering the efficient translation of the protein from the transcript or negatively influence transcript stability.

"Plant tissue": any tissue of plants, for example specialized tissues such as mesophyll, phloem or apical meristem. Additionally it covers plant organs such as for example leaf, root, flower or seed.

"Plastid": any type of plastids present in plants such as for example proplastids, chloroplasts and/or chromoplasts.

"Plant" or "plants" as used herein are interchangeable. A method applied to a plant or an effect detectable in a plant is also applicable or detectable in a plurality of plants. The use of the word "plants" is not excluding that the respective information holds also true for a single plant.

"Part of a plant": any part of a plant such as a single cell or a plant organ such as leaf or root. For example those parts of a plant used in transformation processes such as explants, cotyledons and cuttings. The phrase also covers harvested parts of a plant such as for example flowers, fruits, tubers and/or seeds.

"Wild type plant": any plant that is not genetically modified, for example that is not transgenic or mutagenized.

"Starting plant": a plant used in total or as donor plant for explants or seeds for example for transformation processes or mutagenisation. The "starting plant" could be a "wild type plant" likewise it may also be a genetically modified plant used for supertransformation.

"Reference plant": any plant that is used as a reference for genetically modified plants, for example transgenic or mutagenized plants. A reference plant preferentially is substantially identical to, more preferential a clone of the starting plant used in the respective process for transformation or mutagenization as defined above. A reference plant may also be a transgenic plant comprising an expression construct which itself comprises a parent sequence. This plant may be used as reference for a transgenic plant comprising the respective expression improved sequence comprised in a corresponding expression construct.

"Expression construct": a coding region under control of and physically linked to at least those regulatory elements necessary and sufficient for expression of the coding region in plants.

"Regulatory element": any element able to regulate transcription and/or translation of a sequence and comprises for example promoters, enhancers, polyadenylation signals, terminators and/or regulatory introns. A person skilled in the art is aware of other regulatory regions described in the state of the art useful for regulating expression of a coding region in an appropriate way.

"Plant specific promoter": any promoter able to generate expression in plants irrespective of the origin of the promoter. It could be derived for example from a plant, an alga, a bacteria, a plant virus, a plastid or could be a synthetic sequence.

"Tissue specific promoter": a plant specific promoter as defined above generating expression for example specifically, predominantly or preferably in specific plant tissues.

"Plastid specific promoter": any promoter capable of generating expression in plant plastids irrespective of the origin of the promoter. It could be derived for example from a plant, an alga, a bacteria, a plant virus, a plastid or could be a synthetic sequence.

The ketolase activity in genetically modified plants of the invention and in wild type or reference plants is determined under the following conditions: The ketolase activity in plant materials is determined as described in Frazer et al., (J. Biol. Chem. 272(10): 6128-6135, 1997). The ketolase activity in plant extracts is determined using the substrates beta-carotene and canthaxanthin in the presence of lipid (soya lecithin) and detergent (sodium cholate). Substrate/product ratios from the ketolase assays are determined by HPLC.

In the case of an "increased" ketolase activity, the amount of beta-carotene converted, or the amount of canthaxanthin formed by the protein ketolase within a certain period of time is higher than that converted or formed by a protein ketolase in the reference plant. Preferably the "increase" of the ketolase activity amounts to at least 5%, furthermore preferably at least 20%, furthermore preferably at least 50%, furthermore preferably at least 100%, more preferably at least 300%, even more preferably at least 500%, in particular at least 600% of the ketolase activity of the respective reference plant.

"Essentially quantitative hydrolytic ester cleavage": at least one of the carotenoid esters present, in particular at least one of the ketocarotenoid esters present, is at least about 85%, in particular at least about 88%, hydrolyzed by enzymatic activity according to the invention so that ester groups are no longer present in the molecule.

"Hydrolysis rate": the percentage decrease in the amount of (e.g. extracted) carotenoid esters in a reactant. The hydrolysis rate can be determined in particular by determining the carotenoid ester content in the reactant before and after the hydrolytic treatment, e.g. by chromatography as described in the examples, and determining the content of hydrolyzed esters therefrom. The hydrolysis rates may be 100% or less, that is no carotenoid esters remain or a certain amount remain. For example the hydrolysis rate may range from 88% to 99% or from 95% to 99%.

"Carotenoid content" is defined as the amount of total carotenoids determined by HPLC.

"Carotenoid ester content": the amount of carotenoids esterified by a saturated or mono- or di- or triunsaturated C₁₀₋₂₄, preferably C₁₂₋₂₀ or C₁₄₋₂₀ monocarboxylic acid. The content of carotenoid esters can be measured inter alia by chromatography because carotenoid esters usually have longer retention times on suitable reverse phase support materials such as, for example, long-chain polymer-bound C30 phases than do unbound carotenoids. A suitable C₃₀ support material and suitable separation conditions are mentioned by way of example in example 3.

"Carotenoid profile": the relative amount of different carotenoids compared to each other determined in plants or part of plants.

"Microorganisms": bacteria, yeasts, algae or fungi.

"Expression activity": the amount of transcript formed in a certain time from a gene or the amount of protein formed in a certain time from a transcript or both.

"Increased expression activity" or "increased expression rate": the formation of an increased transcript and/or protein amount formed from a gene in modified plants during a certain period of time, in comparison with reference plants, e.g. wild type.

### b) Particular aspects of the present invention

One aspect of the present invention relates to a process for the preparation of at least one carotenoid, in particular at least one ketocarotenoid as defined above, in genetically modified plants, which method comprises expressing in plants at least one carotene ketolase enzyme encoded by an expression improved heterologous carotene ketolase coding sequence (improved ketolase sequence). In particular, an "increased expression activity" or "increased expression rate" of the coding sequence or gene is observed, resulting in the increased formation of at least one carotenoid as defined above in the plants. In particular, the content of at least one ketocarotenoid selected from astaxanthin, adonixanthin, adonirubin, echinenone, 3'-hydroxyechinenone, 3-hydroxyechinenone, canthaxanthin, and/or the total content of ketocarotenoids in genetically modified plants or parts thereof is statistically significantly increased, if compared to reference plants. Statistically significantly increased means that at least three independent transgenic lines and three reference plants, e.g. wild type plants, are compared for their total carotenoid content and that the amount of total carotenoids in transgenic lines are significantly higher using a statistical test such as the t-test. In the context of ketocarotenoids that are not detectable in most plants for example Tagetes plants the respective detection limit is defined by measuring a series of dilutions of the respective compound with the respective method applied to the plant according to the invention. The detection limit is taken as the value for the total ketocarotenoid content of the reference plants, e.g. wild type plants and the statistical test is accordingly applied to test the significance of the total ketocarotenoid content increase.

According to the process of the invention the ketolase activity may also be increased by applying at least one of the following methods:
The ketolase activity can be further increased in various ways, for example by eliminating inhibiting regulatory mechanisms at the translation and protein level, or by increasing the gene expression of a nucleic acid encoding a ketolase in comparison with the reference plant, e.g. wild type, for example by inducing the ketolase gene by activators.

An increase of the gene expression of a nucleic acid encoding a ketolase is also understood as meaning the manipulation of the expression of the plants' homologous endogenous ketolases. This can be achieved for example by modifying the promoter DNA sequence of ketolase-encoding genes. Such a modification, which results in a modified, preferentially increased expression rate of at least one endogenous ketolase gene, can be a deletion, insertion or substitution of DNA sequences.

It is also possible to modify the expression of at least one endogenous ketolase by applying exogenous stimuli. This can be carried out by specific physiological conditions, i.e. by the application of foreign substances.

Moreover, an increased expression of at least one endogenous ketolase gene can be achieved by a regulator protein, which does not occur in the reference plant, e.g. wild type, or which is modified, and which interacts with the promoter of these genes.

Such a regulator can constitute a chimeric protein, which consists of a DNA binding domain and a transcription activator domain such as described, for example, in WO 96/06166.

In addition, there may be at least one further ketolase gene present in the genetically modified plants according to the invention in comparison with the reference plants, e.g. wild type. In this aspect, the genetically modified plants according to the invention, accordingly, have at least one exogenous (= heterologous) nucleic acid encoding a ketolase, or at least two endogenous nucleic acids encoding ketolases.

There may also be that the starting plants used are plants, which show no ketolase activity in the plants, in particular in petals.

Preferably, the content of at least one of the ketocarotenoids, or the total ketocarotenoid content as defined above, is increased by at least 1%, as for example from 2 to 100 %, or by a factor of 1 to 10, as for example 1, 2, 3, 4 or 5.

Preferably, the plants are of one of the following families: Ranunculaceae, Berberidaceae, Begoniaceae, Papaveraceae, Cannabaceae, Chenopodiaceae, Cruciferae, Rosaceae, Fabaceae, Linaceae, Vitaceae, Brassiceae, Cucurbitaceae, Primulaceae, Caryophyllaceae, Amaranthaceae, Apocynaceae, Balsaminaceae, Gentianaceae, Geraniaceae, Graminae, Euphorbiaceae, Labiatae, Leguminosae, Caprifoliaceae, Oleaceae, Tropaeolaceae, Solanaceae, Lobeliaceae, Scrophulariaceae, Compositae, Asteraceae, Plumbaginaceae, Liliaceae, Amaryllidaceae, Rubiaceae, Poaceae, Polemoniaceae, Orchidaceae, Umbelliferae, Verbenaceae, Violaceae, Malvaceae, Illiaceae and Lamiaceae.

In particular plants are selected from the genus Tagetes, Acacia, Aconitum, Adonis, Arnica, Aqulegia, Aster, Astragalus, Bignonia, Calendula, Caltha, Campanula, Canna, Centaurea, Cheiranthus, Chrysanthemum, Citrus, Crepis, Crocus, Curcurbita, Cytisus, Delonia, Delphinium, Dianthus, Dimorphotheca, Doronicum, Eschscholtzia, Forsythia, Fremontia, Gazania, Gelsemium, Genista, Gentiana, Geranium, Gerbera, Geum, Grevillea, Helenium, Helianthus, Hepatica, Heracleum, Hisbiscus, Heliopsis, Hypericum, Hypochoeris, Impatiens, Iris, Jacaranda, Kerria, Laburnum, Lathyrus, Leontodon, Lilium, Linum, Lotus, Lycopersicon, Lysimachia, Maratia, Medicago, Mimulus, Narcissus, Oenothera, Osmanthus, Petunia, Photinia, Physalis, Phyteuma, Potentilla, Pyracantha, Ranunculus, Rhododendron, Rosa, Rudbeckia, Senecio, Silene, Silphium, Sinapsis, Sorbus, Spartium, Tecoma, Torenia, Tragopogon, Trollius, Tropaeolum, Tulipa, Tussilago, Ulex, Viola and Zinnia.

Preferably the plants are selected from the genus Tagetes (as for example Tagetes erecta and Tagetes patula).

Likewise it is also possible to perform the claimed process with plants showing a low lutein (an alpha-carotenoid) content. This may be achieved by directed or non-directed mutagenesis in combination with screening for mutants with the desired carotenoid profile. Non-directed mutagenesis, for example, may be achieved by making use of chemical mutagens, as for example EMS (ethyl methane sulfonate). Corresponding methods for obtaining such plants, in particular lutein-depleted plants (lutein content 0 to about 90% based on the total carotenoid content, see US 6.784.351), are known in the art, and one specific method is referred to in the experimental part.

Preferably the process of the invention is performed such that the expression of the ketolase gene is, primarily or specifically, observed in flowers of the plants, in particular in petals, especially in plastids like chromoplasts, of the plants. The latter can be achieved for example by targeting the ketolase protein to the respective organelle or by expressing the ketolase in the respective organelle for example by plastid transformation.

The improved ketolase sequence may preferably be modified by adapting its codon usage to the codon usage of the plants or compartments of the plants such as tissues or plastids such as for example chromoplasts. In addition other methods for improving translational efficiency (i.e. number of proteins translated from one transcript per time) may be applied to the parent sequence such as reducing the number, the length and/or the binding energy of potential secondary structures in the transcript, avoiding or removing cryptic splice sites and/or the number of cryptic polyadenylation signals.

In order to adapt the codon usage of a coding sequence to the codon usage of a host organism, the respective host's codon usage may be determined. A list of codon usages for a large range of organisms and organelles may for example be found in the resources of the Japanese "Kazusa DNA Research Institute" provided in the internet under http://www.kazusa.or.jp/codon/ . The person skilled in the art is aware of methods of how to define the codon usage of any given organism or organelle which in addition is exemplified for the determination of the codon usage of Tagetes in the example 4 below. This procedure may be applied to any other organism.

In particular, the adaptation of the ketolase coding sequence to the codon usage of the plants or compartments of the plants such as tissues or plastids comprise replacing at least one parent sequence codon by a different replacement codon encoding the same amino acid, resulting in an "increased expression activity" or "increased expression rate" of the ketolase gene.

Preferably the replacement codon (to be introduced) is present in the genome or transcriptome of the plants or compartments of the plants such as tissues or plastids such as for example chromoplasts in an abundance, which is identical to or higher than the abundance of the parent sequence codon (to be replaced).

In particular, at least one codon of the parent ketolase coding sequence is adapted to a higher abundant codon, preferentially the most abundant codon for the respective amino acid.

The codon usage may be adapted such that the least abundant codons of any amino acid are adapted to higher abundant codons for the respective amino acid. Preferentially the codon of the parent sequence is adapted to the most abundant codon for the respective amino acid.

The codon usage of the parent sequence may also be adapted in a way that the codon usage of the respective expression improved sequence resembles the host's codon usage. For example, the expression improved sequence comprises codons for the individual amino acid in abundance similar to the average codon usage of the host.

The codon usage of the parent sequence may also be adapted in a way that all codons of any amino acid in the parent sequence are adapted to the codon with the highest abundance for the respective amino acid in the hosts' codon usage.

The codon usage may be adapted such that 10 to 100%, as for example 50 to 100%, or 90 to 100% of the codons of the ketolase gene are adapted to the codon usage of the plants or plant plastids such as for example chromoplasts. In particular, the most abundant codon for the respective amino acid may be used for the adaptation.

In addition, the expression of the ketolases may be further improved by removing or avoiding signals and/or structures in the sequence negatively interfering with expression efficiency in the respective host organism such as higher plants or plant plastids such as for example chromoplasts, for example by removing cryptic splice sites, cryptic polyadenylation signals or sequences able to form secondary structures inhibiting expression in particular translation. The person skilled in the art is aware of methods on how to identify and avoid or remove the respective signals such as cryptic splice sites (Haseloff et al. (1997) PNAS 94, 2122-2127), cryptic polyadenylation signals (Grec et al. (2000) Gene 242, 87-95; Rutherford et al. (2005) Plant Journal 43, 769-788) or secondary structures interfering with expression (Wang and Wessler (2001) Plant Phys. 125, 1380-1387) with no or only minor changes of the protein sequence encoded by the respective coding sequence, for example only leading to exchange of similar amino acids as for example shown in the table given in chapter g). Preferentially the respective signals and structures are removed or avoided without changing the amino acid sequence of the protein encoded by the respective coding sequence.

The expression-improved heterologous carotene ketolase coding sequence may be derived from a corresponding parent sequence, e.g. wild type coding sequence of prokaryotic or eukaryotic origin, as for example derived from a corresponding wild type sequence of bacteria, yeast or algae. In particular the coding sequence is derived from the corresponding wild type sequences of algae, in particular algae of the genus Haematococcus, Chlamydomonas, Scenedesmus, or Chlorella.

Non-limiting examples of preferred algal species are Haematococcus pluvialis, Chlamydomonas reinhardtii, Scenedesmus vacuolatus or Chlorella zoofingiensis.

Preferably, a ketolase enzyme is expressed having an amino acid sequence encoded by the corresponding coding portions comprised a nucleotide sequence selected from SEQ ID NO: 3, 4, 7, 8, 10, 11, 13 and 14; or coding sequences derived therefrom by nucleic acid substitution, addition, deletion or insertion, and encoding a ketolase enzyme having a sequence identity of at least 50%, as for example at least 60%, at least 70%, at least 80% or 90% to 100% or 95% to 99%, with respect to the parent sequence and retaining ketolase activity.

Preferably, the expression-improved ketolase coding sequence is selected from the corresponding coding portions comprised in a nucleotide sequence selected from SEQ ID NO: 3, 4, 7, 8, 10, 11, 13 and 14; or coding sequences derived therefrom by nucleic acid substitution, addition, deletion or insertion, and encoding a ketolase enzyme having a sequence identity of at least 50%, as for example at least 60%, at least 70%, at least 80% or 90% to 100% or 95% to 99%, with respect to the parent sequence and retaining ketolase activity.

The genetically modified plants of the process may carry expression constructs encompassing at least one improved ketolase sequence under the control of suitable regulatory elements.

Preferably, the improved ketolase sequence is under the control of a plant specific, in particular plant plastid or plant tissue specific promoter.

In particular, the promoter directs the flower specific, in particular petal specific expression.

The expression of the expression improved ketolase may alternatively take place in the plastids. In that case a promoter functional in plant plastids especially chromoplasts is preferred for controlling expression of the ketolase enzyme.

The expression construct may further comprise the coding sequence for a transit peptide, operably linked to the coding sequence of the ketolase. The transit peptide may originate, as the ketolase, from a different organism, and the coding sequence of such a heterologous transit peptide preferably may be adapted to the codon usage of the plants or compartments of the plants such as tissues or plastids such as for example chromoplasts as explained above for the ketolase coding sequence.

The present invention also relates to expression constructs as defined above, recombinant vectors and/or microorganisms comprising at least one of the expression constructs.

The present invention also relates to genetically modified plants carrying at least one improved ketolase sequence as defined above, or at least one expression construct as defined above, or at least one vector as defined above.

The present invention further relates to genetically modified plants expressing carotene ketolase activity in at least one plant tissue or plastid, and, in particular, genetically modified plants expressing carotene ketolase activity in their flowers, flower petals and/or chromoplasts.

A further aspect of the invention relates to genetically modified plants having an altered carotenoid profile, in particular in their flowers, preferably in their petals, in particular in their plastids.

A further aspect of the invention relates to genetically modified plants containing a detectable amount of at least one ketocarotenoid in at least one part of the plants, in particular in their flowers, preferably petals. The genetically modified plants are selected from plants of the families and the genus as defined above.

A further aspect of the invention also relates to parts or seeds of genetically modified plants as defined above.

A further aspect of the invention relates to a process of preparing genetically modified plants as defined above, which process comprises introducing into the plants at least one expression construct as defined above into starting plants.

A further aspect of the invention relates to a process of preparing parts or seeds of genetically modified plants as defined above, which process comprises introducing into the plants at least one expression construct as defined above into starting plants, growing the so obtained plants and obtaining parts or seeds thereof.

A further aspect of the invention relates to a process of preparing at least one carotenoid, in particular ketocarotenoid, which process comprises cultivating genetically modified plants as defined above under conditions which allow the expression of improved ketolase activity for a sufficient time to produce a detectable amount of at least one ketocarotenoid or derivative thereof within the plants, and isolating the ketocarotenoid or derivative thereof.

A further aspect of the invention relates to the use of genetically modified plants as defined above for preparing carotenoids, in particular ketocaroteonids.

Another aspect of the invention relates to an expression-improved carotene ketolase coding sequence as defined above.

In another aspect, the present invention relates to a process of chemically hydrolyzing carotenoid esters obtained by extracting carotenoid ester containing plants for example such as described above, which process comprises hydrolyzing the carotenoid esters under substantially anaerobic conditions. Preferably the substantially anaerobic hydrolysis is performed in the presence of a base and/or at a reduced reaction temperature. Substantially anaerobic means the absence of oxygen from the reaction medium or that essentially no oxygen is present in the reaction medium. In general an oxygen content of the reaction medium in the range of 1 to 50 ppm, as for example 1 to 10 ppm is acceptable.

In particular, the base is used in a concentration in the range of 0,01 to 0,5 M, as for example 0,1 to 0,4 M. Non-limiting examples of suitable bases are alkali metal salts, as for example sodium or potassium alkoxides, as for example sodium methoxide.

The reaction temperature is in the range from minus 10 to plus 20 °C, as for example from 0 to plus 10 °C or from plus 5 to plus 9 °C.

The carotenoid esters to be hydrolyzed may be obtained by extracting carotenoid ester containing plants or parts thereof, as for example flowers of parts of the flowers, with organic solvents for example acetone, and optionally removing the solvent.

In another aspect the present invention provides a process of analyzing carotenoids in plants, which process comprises obtaining a sample of plant material, as for example of specific parts of the plants, like flowers or parts thereof, like petals, isolating a carotenoid ester containing sample therefrom, performing a chemical hydrolysis of the carotenoid esters as defined above and determining the carotenoid content of the hydrolyzed product in a manner known per se, as for example chromatographically.

### c) Ketolase coding sequences

Suitable ketolase coding sequences which may be used as parent sequence and therefore expression improved according to the invention and applied in a process of the invention are summarized in Annex 1 and described via their data base entries.

### d) Promoter sequences

Numerous plant specific promoters are well known in the art.

As a specific example of a suitable plant specific promoter there may be mentioned the Brassica napus plastid-associated protein X (PAPX) promoter. This promoter substantially corresponds to the nucleotide sequence of position 1734 to 2764 of SEQ ID NO:4. Further suitable promoters are functionally equivalent sequences as derived therefrom which direct the expression of a coding sequence with comparable efficiency and/or specificity. Functionally equivalent promoter sequences may be derived therefrom or may have a sequence homology or identity determinable as defined below, and being in the range of 40 to below 100%, or at least 50%, at least 60%, at least 70%, at least 80%, or at least 90%, as for example 91% to 99% or 94% to 98% if compared to the nucleotide sequence of position 1734 to 2764 of SEQ ID NO:4. Further suitable PAP promoters are disclosed in PCT/EP2007/055756, filed June 12, 2007, the disclosure of which document is herein incorporated by reference. In particular, page 4 line 22 to page 8 line 10 and the sequences referred to therein are herewith incorporated by reference.

Suitable promoters are plant promoters or promoters derived from plant viruses. Specifically mentioned is the promoter of the CaMV cauliflower mosaic virus 35S transcript (Franck et al. (1980) Cell 21:285-294; Odell et al. (1985) Nature 313:810-812; Shewmaker et al. (1985) virology 140:281-288; Gardner et al. (1986) Plant Mol Biol 6:221-228) or the 19S CaMV promoter (U.S. Pat. No. 5,352,605; WO 84/02913; Benfey et al. (1989) EMBO J 8:2195-2202).

Further suitable plant promoters are the fruit specific pds promoter (Pecker et al. (1992) Proc. Natl. Acad. Sci USA 89: 4962-4966), the leaf preferential "Rubisco small subunit (SSU)" promoter (U.S. Pat. No. 4,962,028) or the seed specific legumin B promoter (GenBank Acc. No. X03677). Further suitable are constitutive promoters as the promoter of the Agrobacterium nopaline synthase, the TR dual promoter, the OCS (octopine synthase) promoter from Agrobacterium, the ubiquitin promoter (Holtorf S et al. (1995) Plant Mol Biol 29:637-649), the ubiquitin 1 promoter (Christensen et al. (1992) Plant Mol Biol 18:675-689; Bruce et al. (1989) Proc Natl Acad Sci USA 86:9692-9696), the mas promoter (Fox et al. (1992) Plant Molecular Biology 20 (2) 219-233), the cinnamyl alcohol dehydrogenase promoter (U.S. Pat. No. 5,683,439), the promoters of the vacuolar ATPase subunits, the promoter of a proline-rich protein from wheat (WO 91/13991), the P-nit promoter (Y07648.L, Hillebrand et al. (1998), Plant. Mol. Biol. 36, 89-99, Hillebrand et al. (1996), Gene, 170, 197-200), the ferredoxin NADPH oxidoreductase promoter (database entry AB011474, position 70127 to 69493), the TPT promoter (WO 03006660), the "superpromoter" (US 5,955,646), the 34S promoter (US 6,051,753), and further promoters of genes whose constitutive expression in plants is known to the skilled worker.

The expression cassettes may also comprise a chemically inducible promoter (review paper: Gatz et al. (1997) Annu Rev Plant Physiol Plant Mol Biol 48:89-108), by means of which the expression of the ketolase gene in plants can be controlled at a particular point in time. Such promoters such as, for example, the PRP1 promoter (Ward et al. (1993) Plant Mol Biol 22:361-366), salicylic-acid-inducible promoter (WO 95/19443), a benzenesulfonamide-inducible promoter (EP 0 388 186), a tetracyclin-inducible promoter (Gatz et al. (1992) Plant J 2:397-404), an abscisic-acid-inducible promoter (EP 0 335 528) or an ethanol- or cyclohexanone-inducible promoter (WO 93/21334) can likewise be used.

Other promoters are those which are induced by biotic or abiotic stress such as, for example, the pathogen-inducible promoter of the PRP1 gene (Ward et al. (1993) Plant Mol Biol 22:361-366), the heat-inducible hsp70 or hsp80 promoter from tomato (U.S. Pat. No. 5,187,267), the cold-inducible alpha-amylase promoter from potato (WO 96/12814), the light-inducible PPDK promoter or the wounding-induced pinII promoter (EP375091).

Pathogen-inducible promoters comprise the promoters of genes which are induced as the result of a pathogen attack such as, for example, genes of PR proteins, SAR proteins, beta-1,3-glucanase, chitinase and the like (for example Redolfi et al. (1983) Neth J Plant Pathol 89:245-254; Uknes, et al. (1992) The Plant Cell 4:645-656; Van Loon (1985) Plant Mol Viral 4:111-116; Marineau et al. (1987) Plant Mol Biol 9:335-342; Matton et al. (1987) Molecular Plant-Microbe Interactions 2:325-342; Somssich et al. (1986) Proc Natl Acad Sci USA 83:2427-2430; Somssich et al. (1988) Mol Gen Genetics 2:93-98; Chen et al. (1996) Plant J 10:955-966; Zhang and Sing (1994) Proc Natl Acad Sci USA 91:2507-2511; Warner, et al. (1993) Plant J 3:191-201; Siebertz et al. (1989) Plant Cell 1:961-968(1989).

Also comprised are wounding-inducible promoters such as that of the promoter of the pinII gene (Ryan (1990) Ann Rev Phytopath 28:425-449; Duan et al. (1996) Nat Biotech 14:494-498), the promoters of the wun1 and wun2 genes (U.S. Pat. No. 5,428,148), the promoters of the win1 and win2 genes (Stanford et al. (1989) Mol Gen Genet 215:200-208), of the systemin gene (McGurl et al. (1992) Science 225:1570-1573), the WIP1 gene (Rohmeier et al. (1993) Plant Mol Biol 22:783-792; Ekelkamp et al. (1993) FEBS Letters 323:73-76), or the MPI gene (Corderok et al. (1994) The Plant J 6(2):141-150) and the like.

Further suitable promoters are, for example, fruit-maturation-specific promoters such as the fruit-maturation-specific promoter from tomato (WO 94/21794, EP 409 625). Some of the development specific promoters are additionally tissue-specific since the individual tissues are formed as a function of the development.

Furthermore suitable are those promoters which ensure the expression in tissues or plant parts in which, for example, the biosynthesis of ketocarotenoids or their precursors takes place. Examples of preferred promoters are promoters with specificities for the anthers, ovaries, petals, sepals, flowers, leaves, stems and roots and combinations hereof.

Tuber-specific, storage-root-specific or root-specific promoters are, for example, the patatin promoter class I (B33) or the promoter of the cathepsin D inhibitor from potato.

Examples of leaf-specific promoters are, for example, the promoter of the cytosolic FBPase from potato (WO 97/05900), the SSU promoter (small subunit) of Rubisco (ribulose-1,5-bisphosphate carboxylase) or the ST-LSI promoter from potato (Stockhaus et al. (1989) EMBO J 8:2445-2451).

Examples of anther-specific promoters are the 5126 promoter (U.S. Pat. No. 5,689,049, U.S. Pat. No. 5,689,051) or the glob-1 promoter or the g-zein promoter.

Promoters suitable for expression in plastids and/or chromoplasts are for example plastid derived promoters. A number of plastid functional promoters are available in the art. Such promoters include, but are not limited to the promoter of the D1 thylakoid membrane protein, psbA (Staub et al. (1993) EMBO Journal, 12(2):601-606) the 16s rRNA promoter region, Pm (Staub et al. (1992) Plant Ce11 4:39-45) or the rbcL promoter from spinach.

Further promoters which are suitable for expression in plants are described in Rogers et al. (1987) Methods in Enzymol 153:253-277; Schardl et al. (1987) Gene 61:1-11 and Berger et al. (1989) Proc Natl Acad Sci USA 86:8402-8406.
Non-limiting flower specific promoters are disclosed in WO 04/27070, WO 05/019460, WO 06/117381 und EP06115339.1 as filed on June 13, 2006.

Examples of flower-specific promoters are the phytoene synthase promoter (WO 92/16635), the promoter of the P-rr gene (WO 98/22593), the EPSPS promoter (database entry M37029), the DFR-A promoter (database entry X79723), the B gene promoter (WO 00/08920) and the CHRC promoter (WO 98/24300; Vishnevetsky et al. (1996) Plant J. 10, 1111-1118), and the promoters of the Arabidopsis gene loci At5g33370, At5g22430 and At1g26630.

Specific mention is made for the following promoters disclosed in WO05/019460:

| Name | Source |
|---|---|
| EPSPS Promoter | Petunia hybrida |
| B-Gene Promoter | Lycopersicon esculentum |
| PDS Promoter | Lycopersicon esculentum |
| CHRC Promoter | Cucumis sativus |

in WO 04/27070: the Arabidopsis promoters: P76, P60, P84
and in WO 06/117381: the Arabidopsis promoters: M1s, M2s, M3s, M1L, M2L

### e) Transit peptides

Transit peptides are examples of targeting sequence. Targeting sequences ensure the subcellular localization in the apoplast, in the vacuole, in plastids, in the mitochondrium, in the endoplasmic reticulum (ER), in the nucleus, in oil bodies or other compartments.
The translocation in plastids, and a discussion of suitable transit peptides is described in:
Woolhead et al., Biochemical Society Transactions. 28 (Part4):491-494, 2000
Reumann et al., Molecular Membrane Biology. 22(1-2):73-N)L_20, 2005
Lubeck et al., Physiologia Plantarum. 100(1):53-64, 1997 and
Robinson et al., Plant Molecular Biology. 38(1-2):209-221, 1998

### f) Expression in plastids

The expression improved ketolase sequence may be expressed in plastids such as chromoplasts, preferentially in flower chromoplasts. Such transplastomic plants are a further aspect of the present invention. Methods of plant plastid transformation and functional expression of genes in the organelles are known in the art. Such methods may be found for example in EP1458875 or EP1461439.

### g) Further aspects of proteins/polypeptides/enzymes of the invention

The invention also comprises likewise "functional equivalents" of the specifically disclosed proteins/polypeptides/enzymes (subsequently simply referred to as polypeptides).

"Functional equivalents" or analogs of the specifically disclosed polypeptides are in the context of the present invention polypeptides which differ therefrom, such as, for example, those having a degree of homology of less than 100%, but which still have the desired biological activity.

"Functional equivalents" mean according to the invention in particular mutants, which have in at least one of the positions of the specific sequences described herein an amino acid which differs from that specifically mentioned, but nevertheless have one of the biological activities mentioned herein. "Functional equivalents" thus comprise the mutants obtainable by one or more amino acid additions, substitutions, deletions and/or inversions, it being possible for the changes to occur in any sequence position as long as they lead to a mutant having a property according to the invention. Functional equivalence exists in particular also when there is a qualitative agreement between the mutant and unmodified polypeptide in the reactivity pattern, i.e. for example identical biological effects are to be observed but differ greatly in the level of expression. Examples of suitable substitutions of amino acid residues are the following:

| Original residue | Examples of substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Functional equivalents" in the above sense are also precursors of the polypeptides described, and functional derivatives and salts of the polypeptides. The term "salts" means both salts of carboxyl groups and acid addition salts of amino groups of the protein molecules of the invention. Salts of carboxyl groups can be prepared in a manner known per se and comprise inorganic salts such as, for example, sodium, calcium, ammonium, iron or zinc salts, and salts with organic bases such as, for example, amines, such as triethanolamine, arginine, lysine or piperidine. Acid addition salts such as, for example, salts with mineral acids such as hydrochloric acid or sulfuric acid and salts with organic acids, such as acetic acid or oxalic acid are likewise an aspect of the invention.

"Functional derivatives" of polypeptides of the invention can likewise be prepared on functional amino acid side groups or on their N- or C-terminal end with the aid of known techniques. Derivatives of these types comprise for example aliphatic esters of carboxylic acid groups, amides of carboxylic acid groups, obtainable by reaction with ammonia or with a primary or secondary amine; N-acryl derivatives of free amino groups prepared by reaction with acyl groups; or O-acyl derivatives of free hydroxy groups prepared by reaction with acyl groups.

"Functional equivalents" of course also comprise polypeptides obtainable from other organisms, and naturally occurring variants. For example, areas of homologous sequence regions can be found by sequence comparison, and equivalent enzymes/polypeptides can be established on the basis of the specific requirements of the invention.

"Functional equivalents" are moreover fusion proteins having one of the abovementioned polypeptide sequences or functional equivalents derived therefrom, and at least one further heterologous sequence functionally different therefrom in functional N- or C-terminal linkage (i.e. with negligible mutual functional impairment of the portions of the fusion proteins). No limiting examples of such heterologous sequences are other enzymes.

"Functional equivalents" also comprised by the invention are homologues of the specifically disclosed proteins. These have at least 60%, preferably at least 75%, in particular at least 85%, such as, for example, 90%, 95, 96, 97, 98 or 99%, homology to one of the specifically disclosed sequences, calculated by the algorithm of Pearson and Lipman, Proc. Natl. Acad, Sci. (USA) 85(8), 1988, 2444-2448. A percentage homology of a homologous polypeptide of the invention means in particular the percentage identity of the amino acid residue based on the complete length of one of the amino acid sequences specifically described herein.

A "derived" amino acid sequence means according to the invention, unless indicated otherwise, a sequence which has an identity of at least 80% or at least 90%, in particular 91%, 92%, 93%, 94%, 95%, 96%, 97%, 98% and 99%, with the initial sequence.

"Identity" or "homology" between two sequences means identity of the amino acid residues over the complete length of the sequence in each case, such as, for example, the identity calculated by comparison with the aid of the Vector NTI Suite 7.1 Software from Informax (USA) using the Clustal method (Higgins DG, Sharp PM. Fast and sensitive multiple sequence alignments on a microcomputer. Comput Appl. Biosci. 1989 Apr;5(2):151-1), setting the following parameters:

**Multiple alignment parameter:**

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

**Pairwise alignment parameter:**

| | |
|---|---|
| FAST algorithm | on |
| K-tuple size | 1 |
| Gap penalty | 3 |
| Window size | 5 |
| Number of best diagonals | 5 |

In the case where protein glycosylation is possible, equivalents of the invention comprise proteins of the type designated above in deglycosylated or glycosylated form and modified forms obtainable by altering the glycosylation pattern.

Homologues of the peptides of the invention can be identified by screening combinatorial libraries of mutants such as, truncation mutants. For example, it is possible to generate a variegated library of peptide variants by combinatorial mutagenesis at the nucleic acid level, for example, by enzymatic ligation of a mixture of synthetic oligonucleotides. There are a large number of methods which can be used to produce libraries of potential homologues from a degenerate oligonucleotide sequence. Chemical synthesis of a degenerate gene sequence can be carried out in an automatic DNA synthesizer, and the synthetic gene can then be ligated into a suitable expression vector. The use of a degenerate set of genes makes it possible to provide all sequences which encode the desired set of potential protein sequences in one mixture. Methods for synthesizing degenerate oligonucleotides are known to the skilled worker (e.g. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

### h) Further aspects on nucleic acids

All nucleic acid sequences of the invention (single- and double-stranded DNA and RNA sequences, such as cDNA or mRNA) can be prepared in a manner known per se by chemical synthesis from the nucleotide units, for example, by fragment condensation of individual overlapping, complementary nucleic acid units of the double helix. Chemical synthesis of oligonucleotides can take place for example in a known manner, for example by the phosphoamidite method (Voet, Voet, 2nd edition, Wiley Press New York, pages 896-897). Addition of synthetic oligonucleotides and filling in of gaps using the Klenow fragment of DNA polymerase and ligation reactions, and general cloning methods are for instance described in Sambrook et al. (1989), Molecular Cloning: A laboratory manual, Cold Spring Harbor Laboratory Press.

A "derived" nucleic acid sequence means according to the invention, unless indicated otherwise, a sequence which has an identity of at least 80% or at least 90%, in particular 91%, 92%, 93%, 94%, 95%, 96%, 97%, 98% and 99%, with the initial sequence.

"Identity" or "homology" between two nucleic acids means the identity of the nucleotides over the complete length of the nucleic acid in each case, in particular the identity by comparison with the aid of the Vector NTI Suite 7.1 Software from Informax (USA) using the Clustal method (see above).

The invention also relates to nucleic acid sequences coding for one of the above peptides and their functional equivalents, which can be obtained for example by use of artificial nucleotide analogs.

The invention relates both to isolated nucleic acid molecules which code for peptides of the invention or biologically active segments thereof, and nucleic acid fragments which can be used for example as hybridization probes or primers for identifying or amplifying coding nucleic acids of the invention.

The nucleic acid molecules of the invention may additionally comprise untranslated sequences from the 3' and/or 5' end of the coding region of the gene.

"Isolated" nucleic acid molecules are separated from other nucleic acid molecules which are present in the natural source of the isolated nucleic acid and may moreover be substantially free of other cellular material or culture medium if it is prepared by recombinant techniques, or free of chemical precursors or other chemicals if it is synthesized chemically.

A nucleic acid molecule of the invention can be isolated by means of standard techniques of molecular biology and the sequence information provided by the invention. For example, cDNA can be isolated from a suitable cDNA library by using one of the specifically disclosed complete sequences or a segment thereof as hybridization probe and standard hybridization techniques (as described for example in Sambrook, J., Fritsch, E.F. and Maniatis, T. Molecular Cloning: A Laboratory Manual. 2nd edition, Cold Spring Harbor Laboratory, Cold Spring Harbor Laboratory Press, Cold Spring Harbor, NY, 1989). It is moreover possible to isolate a nucleic acid molecule comprising one of the sequences of the invention or a segment thereof by polymerase chain reaction using the oligonucleotide primers constructed on the basis of this sequence. The nucleic acid amplified in this way can be cloned into a suitable vector and characterized by DNA sequence analysis. The oligonucleotides of the invention can also be prepared by standard synthesis methods, e.g. using an automatic DNA synthesizer.

The invention further comprises the nucleic acid molecules complementary to the specifically described nucleotide sequences, or a segment thereof.

The nucleotide sequences of the invention make it possible to produce probes and primers which can be used for identifying and/or cloning homologous sequences in other cell types and organisms. Such probes and primers usually comprise a nucleotide sequence region which hybridizes under stringent conditions to at least about 12, preferably at least about 25, such as, for example, about 40, 50 or 75, consecutive nucleotides of a sense strand of a nucleic acid sequence of the invention or of a corresponding antisense strand.

Further nucleic acid sequences of the invention are derived from the sequences as specifically mentioned herein and differ therefrom by addition, substitution, insertion or deletion of one or more nucleotides, but still code for peptides having the desired profile of properties.

The invention also comprises nucleic acid sequences, which comprise so-called silent mutations, as well as naturally occurring variants such as, for example, splice variants or allelic variants, thereof. Sequences obtainable by conservative nucleotide substitutions (i.e. the relevant amino acid is replaced by an amino acid of the same charge, size, polarity and/or solubility) are likewise an aspect.

The invention also relates to the molecules derived from the specifically disclosed nucleic acids through sequence polymorphisms. These genetic polymorphisms may exist because of the natural variation between individuals within a population. These natural variations normally result in a variance of from 1% to 5% in the nucleotide sequence of a gene.

The invention further also comprises nucleic acid sequences which hybridize with the abovementioned coding sequences or are complementary thereto. These polynucleotides can be found by screening genomic or cDNA libraries and if appropriate be amplified therefrom by means of PCR with suitable primers, and subsequently isolated for example with suitable probes. A further possibility is the transformation of suitable microorganisms with polynucleotides or vectors of the invention, to multiply the microorganisms and thus the polynucleotides and subsequently to isolate them. An additional possibility is to synthesize polynucleotides of the invention also by a chemical route.

The property of being able to "hybridize" onto polynucleotides means the ability of a polynucleotide or oligonucleotide to bind under stringent conditions to an almost complementary sequence, while there are nonspecific bindings between non-complementary partners under these conditions. For this purpose, the sequences should be from 70% to 100%, preferably from 90% to 100%, complementary. The property of complementary sequences being able to bind specifically to one another is made use of, for example, in the Northern or Southern blotting technique or in the primer binding in PCR or RT-PCR. Oligonucleotides with a length of 30 base pairs or more are normally employed for this purpose. Stringent conditions mean, for example, in the Northern blotting technique the use of a washing solution, for example 0.1x SSC buffer with 0.1 % SDS (20x SSC: 3M NaCl, 0.3M Na citrate, pH 7.0), from 50°C to 70°C, preferably from 60°C to 65°C, for eluting nonspecifically hybridized cDNA probes or oligonucleotides. In this case, as mentioned above, only nucleic acids with a high degree of complementarity remain bound to one another. The setting up of stringent conditions is known to the skilled worker and is described for example in Ausubel et al., Current Protocols in Molecular Biology, John Wiley & Sons, N.Y. (1989), 6.3.1-6.3.6.

For example, the conditions during the washing step can be selected from the range of conditions delimited by those with less stringency (with 2 x SSC at 50 °C) and those with high stringency (with 0.2 x SSC at 50 °C, preferably at 65 °C) (20 x SSC: 0.3 M sodium citrate, 3 M sodium chloride, pH 7.0).

Moreover, the temperature during the washing step can be increased from moderate conditions at room temperature, 22 °C, to stringent conditions at 65 °C

Both parameters, salt concentration and temperature can be varied simultaneously, or else one of the two parameters can be kept constant, while only the other one is varied. Also, denaturing agents such as, for example, formamide or SDS can be employed during the hybridization step. In the presence of 50% formamide, the hybridization is preferably carried out at 42 °C

Some examples of conditions for hybridization and washing step are shown herein below:
(1) hybridization conditions with, for example,
   (i) 4 x SSC at 65 °C, or [0074] (ii) 6 x SSC at 45 °C, or
   (iii) 6 x SSC at 68 °C, 100 mg/ml denatured fish sperm DNA, or
   (iv) 6 x SSC, 0.5% SDS, 100 mg/ml denatured, fragmented salmon sperm DNA at 68 °C, or
   (v) 6 x SSC, 0.5% SDS, 100 mg/ml denatured, fragmented salmon sperm DNA, 50% formamide at 42 °C, or
   (vi) 50% formamide, 4 x SSC at 42 °C, or
   (vii) 50% (vol/vol) formamide, 0.1% bovine serum albumin, 0.1% Ficoll, 0.1% polyvinylpyrrolidone, 50 mM sodium phosphate buffer pH 6.5, 750 mM NaCl, 75 mM sodium citrate at 42 °C, or
   (viii) 2.times.or 4 x SSC at 50 °C (moderate conditions), or
   (ix) 30 to 40% formamide, 2.times.or 4 x SSC at 42 °C (moderate conditions).
(2) washing steps for in each case 2x 20 minutes, with, for example,
   (i) 0.015 M NaCl/0.0015 M sodium citrate/0.1% SDS at 50 °C, or
   (ii) 0.1 x SSC at 65 °C, or
   (iii) 0.1 x SSC, 0.5% SDS at 68 °C, or
   (iv) 0.1 x SSC, 0.5% SDS, 50% formamide at 42 °C, or
   (v) 0.2 x SSC, 0.1 % SDS at 42 °C, or
   (vi) 2 x SSC at 65 °C (moderate conditions).

### i) Expression constructs and vectors

The invention additionally relates to expression constructs comprising, under the genetic control of regulatory nucleic acid sequences, a nucleic acid sequence coding at least one polypeptide of the invention and to vectors comprising at least one of these expression constructs.

Such constructs of the invention preferably comprise a promoter 5'-upstream from the particular coding sequence, and a terminator sequence 3'-downstream, and, if appropriate, other usual regulatory elements, in particular each operatively linked to the coding sequence.

A" promoter" is understood according to the invention as meaning a nucleic acid having expression activity, that is a nucleic acid which, in functional linkage with a nucleic acid to be expressed, also described as a gene, regulates the expression, that is the transcription of this nucleic acid or this gene.

A "constitutive" promoter means those promoters which ensure expression in a large number of, preferably all, tissues over a substantial period of the plant's development, preferably at all points in time of the plant's development.

"Transcription" is understood according to the invention as meaning the process by which, starting from a DNA matrix, a complementary RNA molecule is prepared. Proteins such as RNA polymerase, "sigma factors" and transcriptional regulator proteins are involved in this process. The RNA synthesized is then used as a matrix in the translation process, which then leads to the biosynthetically active protein.

"Operative linkage" or "functional linkage" means the sequential arrangement of promoter, coding sequence, terminator and, if appropriate, other regulatory elements in such a way that each of the regulatory elements is able to comply with its function as intended for expression of the coding sequence. To this end, a direct linkage in the chemical sense is not imperative. Genetic control sequences, such as enhancer sequences, can also exert their function on the target sequence from positions which are further removed or even from other DNA molecules. Arrangements are preferred in which the nucleic acid sequence to be expressed or the gene to be expressed is positioned behind (i.e. at the 3'-end) the promoter sequence according to the invention, such that both sequences are bonded covalently to one another. Preferably, the distance between the promoter sequence and the nucleic acid sequence to be expressed is in this case lower than 200 base pairs, particularly preferably less than 100 base pairs, very particularly preferably less than 50 base pairs. Examples of sequences which can be operatively linked are targeting sequences and enhancers, polyadenylation signals. Other regulatory elements comprise amplification signals, origins of replication and translation enhancers such as the tobacco mosaic virus 5'-leader sequence (Gallie et al., Nucl. Acids Res. 15 (1987), 8693-8711). Suitable regulatory sequences are described, for example, in Goeddel, Gene Expression Technology: Methods in Enzymology 185, Academic Press, San Diego, CA (1990).

In addition to the artificial regulatory sequences it is possible for the natural regulatory sequence still to be present in front (i.e. at the 5'-end) of the actual structural gene. This natural regulation can, if appropriate, be switched off by genetic modification, and expression of the genes can be increased or decreased. The gene construct can, however, also have a simpler structure, that is to say no additional regulatory signals are inserted in front of the structural gene, and the natural promoter with its regulation is not deleted. Instead, the natural regulatory sequence is mutated so that regulation no longer takes place, and gene expression is enhanced. The nucleic acid sequences may be present in one or more copies in the gene construct.

The regulatory sequences are intended to make specific expression of the nucleic acid sequences and protein expression possible. This may mean, for example, depending on the host organism, that the gene is expressed or over expressed only after induction or that it is immediately expressed and/or over expressed.

The regulatory sequences or factors may moreover preferably influence positively, and thus increase expression. Thus, enhancement of the regulatory elements can take place advantageously at the level of transcription by using strong transcription signals such as promoters and/or enhancers. However, it is also possible to enhance translation by, for example, improving the stability of the mRNA.

An expression cassette is produced by fusing a suitable promoter to a suitable coding nucleotide sequence and to a terminator signal or polyadenylation signal. Conventional techniques of recombination and cloning are used for this purpose, as described, for example, in T. Maniatis, E.F. Fritsch and J. Sambrook, Molecular Cloning: A Laboratory Manual, Cold Spring Harbor Laboratory, Cold Spring Harbor, NY (1989) and in T.J. Silhavy, M.L. Berman and L.W. Enquist, Experiments with Gene Fusions, Cold Spring Harbor Laboratory, Cold Spring Harbor, NY (1984) and in Ausubel, F.M. et al., Current Protocols in Molecular Biology, Greene Publishing Assoc. and Wiley Interscience (1987).

An expression cassette or a vector comprising a suitable promoter, suitable coding nucleotide sequence and a terminator signal or polyadenylation signal can also be produced by synthesis of the entire sequence as described above.

### Examples of suitable expression vectors which may be mentioned are:

Suitable plant expression vectors are for example described in detail in: Becker, D., Kemper, E., Schell, J. and Masterson, R. (1992) "New plant binary vectors with selectable markers located proximal to the left border", Plant Mol. Biol. 20:1195-1197; and Bevan, M.W. (1984) "Binary Agrobacterium vectors for plant transformation", Nucl. Acids Res. 12:8711-8721.

### k) Transfer of foreign genes into a plant

The transfer of foreign genes in the genome of plants is referred to as transformation.

To this end, it is possible to exploit methods which are known per se for the transformation and regeneration of plants from plant tissues or plant cells in order to carry out a transient or stable transformation.

Suitable methods for the transformation of plants are the transformation of protoplasts by means of polyethylene-glycol-induced DNA uptake, the biolistic method using the gene gun, known as "particle bombardment method", electroporation, incubation of dry embryos in DNA-comprising solution, microinjection, and Agrobacterium-mediated gene transfer. The above methods are described, for example, in B. Jenes et al., Techniques for Gene Transfer, in: Transgenic Plants, Vol. 1, Engineering and Utilization, edited by S. D. Kung and R. Wu, Academic Press (1993), 128-143 and in Potrykus, Annu. Rev. Plant Physiol. Plant Molec. Biol. 42 (1991), 205-225).

By preference, the construct to be expressed is cloned into a vector which is suitable for the transformation of Agrobacterium tumefaciens, for example pBin19 (Bevan et al., Nucl. Acids Res. 12 (1984), 8711) or particularly preferably pSUN2, pSUN3, pSUN4 or pSUN5 (WO 02/00900).

Agrobacteria which have been transformed with an expression plasmid can be used in the known manner for the transformation of plants, for example by bathing scarified leaves or leaf segments in an agrobacterial solution and subsequently growing them in suitable media.

For the preferred generation of genetically modified plants, herein below also referred to as transgenic plants, the fused expression cassette which expresses a ketolase is cloned into a vector, for example pBin19 or pSUN2, which is suitable for being transformed into Agrobacterium tumefaciens. Agrobacteria which have been transformed with such a vector can then be used in the known manner for the transformation of plants, in particular crop plants, for example by bathing scarified leaves or leaf segments in an agrobacterial solution and subsequently growing them in suitable media.

Suitable Agrobacteria are for example Agrobacterium tumefaciens or Agrobacterium rhizogenes. Other Agrobacteria useful for plant transformation are known in the art and can be used in the process of the present invention.

The transformation of plants by Agrobacteria is known, inter alia, from F. F. White, Vectors for Gene Transfer in Higher Plants; in Transgenic Plants, Vol. 1, Engineering and Utilization, edited by S. D. Kung and R. Wu, Academic Press, 1993, pp. 15-38. Transgenic plants can be regenerated in the known manner from the transformed cells of the scarified leaves or leaf segments or hypocotyls, and such plants comprise a gene for the expression of a nucleic acid encoding a ketolase integrated into the expression cassette.

To transform host plants with a nucleic acid which encodes a ketolase, an expression cassette is incorporated, as insertion, into a recombinant vector whose vector DNA comprises additional functional regulatory signals, for example sequences for replication or integration. Suitable vectors are described, inter alia, in "Methods in Plant Molecular Biology and Biotechnology" (CRC Press), chapter 6/7, pp. 71-119 (1993).

### m) Preparation of transgenic ketocarotenoid producing Tagetes plants

Recombinant Tagetes plants expressing expression-optimized ketolase activity may be prepared starting from per se known ketolase-encoding sequences (see Annex 1) and improving the sequence of the ketolase by for example adapting the sequence to the codon usage of the Tagetes plants to be used. Suitable Tagetes plants may be transformed in a manner known per se with the modified ketolase coding sequence. Specific examples are given in the attached experimental part. Based on the specific information a skilled reader will be in a position, to prepare transformants of different Tagetes plants modified by the same or different ketolase coding sequences.

Suitable methods of preparing transgenic Tagetes plants are disclosed in EP-A-1 240 342.

### n) Carotenoid extraction methods

Carotenoids and their esters, such as astaxanthin and its mono- and diesters, can be extracted from the carotenoid-containing plants or plant parts, which have previously been dried and/or comminuted where appropriate, by organic solvents such as, for example, by acetone, hexane, methylene chloride, tert-butyl methyl ether or by solvent mixtures such as ethanol/hexane or acetone/hexane. The extractive effect can be varied on the basis of differences in polarity through different solvent mixing ratios. Enrichment of carotenoids and their esters to high concentration is possible by such an extraction.

Extracts prepared in this way are particularly suitable as reactant for carrying out the chemical hydrolysis reaction of the invention.

### o) Workup of the ester hydrolysis products

The carotenoids, especially ketocarotenoids as obtained by hydrolysis, can advantageously be isolated from the aqueous reaction solution by extraction. The extraction can be repeated more than once to increase the yield. Examples of suitable extractants are organic solvents such as toluene, methylene chloride, butyl acetate, diisopropyl ether, benzene, MTBE (Methyl-tert-butylether), petroleum ether or ethyl acetate. After concentration of the organic phase obtained in this way, the products can ordinarily be isolated in good chemical purities.

The identity and purity of the isolated compound(s) can be determined by known techniques. These include high performance liquid chromatography (HPLC), spectroscopic methods, staining methods, thin-layer chromatography, enzyme assay or microbiological assays. These analytical methods are summarized in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Vol. A27, VCH: Weinheim, pages 89-90, 521-540, 540-547, 559-566, 575-581 and 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Vol. 17.

### p) Applications of the products prepared according to the invention:

The carotenoids obtained according to the invention are particularly suitable as additives for human and animal foods. As additive for human food, they may be used for coloring of any food product for example beverages, sweets or convenient food. In case they are used as animal food additive they promote in particular pigmentation after, preferably oral, administration.

"Pigmentation" means according to the invention preferably the intensification or causation of a color of at least part of an animal or animal product of the pigmented animal compared with the non-pigmented animal. Thus, in particular, astaxanthin-containing pigmenting agents generate or intensify a pink to pinkish red hue.

Preferred animals, which can be pigmented by the oral administration of the invention are animals selected from fish, crustaceans or birds, especially galliformes and anatidae. Preferred fish are salmonids, especially salmon or trout. Preferred crustaceans are shrimps or crayfish. Preferred galliformes are chickens, ducks and geese. Preferred anatidae are flamingo.

Depending on the pigmented animal, the preferred pigmented animal products mean, in particular, flesh for salmon or trout, skin for chickens, ducks or geese, feathers for chickens, ducks, geese or flamingo and egg or yolk for chickens, ducks or geese.

Oral administration of the carotenoids to animals can take place directly or, preferably, by oral administration of animal food preparations previously admixed with the carotenoid. The carotenoids may in this case be in liquid or solid form.

The carotenoids may, as long as the solvents still present are physiologically harmless for the appropriate animals, be added directly to the animal food preparation or be employed in the form of carotenoid-containing powders or oils after evaporation of the solvents still present. Previous purification of the resulting hydrolysis product is not absolutely necessary.

The resulting carotenoid-containing powders or oils can for example be incorporated in fish oil, be applied to powdered carrier materials such as, for example, wheat flour, or be enclosed in alginates, gelatin or lipids.

The invention also relates to animal food preparations comprising at least one carotenoid hydrolysate of the invention in addition to conventional animal food ingredients.

Thus, for example, a fish food preparation may comprise further conventional fish food components such as, for example, fish meal and/or other proteins, oils such as, for example, fish oils, cereals, vitamins, minerals, preservatives and, where appropriate, medicaments in conventional amounts.

A typical fish food formula for trout is composed for example of the following components:

| | | Weight for 500 kg |
|---|---|---|
| Components | % by weight | kg |
| Fish meal | 30.00 | 150.00 |
| Full-fat soybeans | 20.00 | 100.00 |
| Pregelatinized wheat flour | 18.00 | 90.00 |
| Vitamin premix | 0.80 | 4.00 |
| Choline chloride (50%) | 0.20 | 1.00 |
| Wheat gluten | 20.00 | 100.00 |
| Sipernat 50S | 3.00 | 15.00 |
| Fish oil | 8.00 | 40.00 |

A typical fish food formula for salmon is composed for example of the following components:

| | |
|---|---|
| | |

| Components | % by weight |
|---|---|
| Fish meal | 75.00 |
| Vegetable protein | 5.00 |
| Cereals | 7.80 |
| Vitamins/minerals | 1.00 |
| Antioxidants/preservatives | 0.20 |
| Fish oil | 11.00 |

The carotenoids of the invention can be admixed in the form of powder or liquid for example solved or suspended in oil to the animal food preparations. The animal food preparations obtained in this way can be pelleted or, particularly advantageously, extruded in a manner known per se.

In a preferred embodiment, the carotenoid-containing products are admixed preferably in the liquid form to the animal food preparations. This is particularly advantageous for producing extruded feed preparations. The extrusion process may lead to extrusion stress on the sensitive substances such as, for example, astaxanthin, which may lead to loss of astaxanthin. Extrusion stress takes the form primarily of the action of mechanical forces (kneading, shearing, pressure, etc.) but also of hydrothermal stress caused by addition of water and water vapor; oxidative stress is also to be observed.

In order to avoid the losses of substance occurring in the extrusion process described above, it is possible to apply liquid carotenoid-containing extracts by the so-called PPA (post pelleting application) technique after the extrusion and drying process under vacuum.

The carotenoids may also be administered orally to animals directly as long as the solvents still present are physiologically harmless to the corresponding animals.

However, the carotenoids can also be administered in the form of powders or oils only after evaporation of the solvents still present.

The resulting carotenoid-containing powders or oils can for example be incorporated in fish oil, be applied to powdered carrier materials such as, for example, wheat flour, or be enclosed in alginates, gelatin or lipids.

The invention therefore also relates to pigmenting agents comprising carotenoids produced by the invention.

### The invention is illustrated by the following examples but is not restricted to these:

### EXPERIMENTAL PART

### A. General Examples

### EXAMPLE 1: Development of transformable Tagetes germplasm

### Generation of Lutein-depleted Tagetes

Naturally occurring Tagetes plants which accumulate relatively high concentrations of beta-carotenoids while most of the alpha-carotenoids do not accumulate are not known. Therefore, it was the task to develop Tagetes plants which fulfill the requirements of i) being largely devoid of lutein and associated alpha-carotenoids in their flower petals and ii) being characterized with relatively high levels of total carotenoids. The process for creating Tagetes plants with lutein depleted flowers is described in the US patent No. 6,784,351. This patent describes in detail i) the EMS mutagenesis of Tagetes erecta "Scarletade" and "13819", and ii) the HPLC screening procedure to identify certain abnormal carotenoid profiles in flowers of "Scarletade" and "13819". The especially interesting mutant of Scarletade, 124-257, is described by its changed carotenoid profile in petals and leaves.

### Breeding of 31360-2-08 and 31360-2-09

Tagetes erecta selection 124-257, described in U.S. Patent No. 6,784,351, was found to have a low transformation rate using the identified tissue culture regeneration medium and Agrobacterium transformation technique. Using a standardized method (see review of S.B. Gelvin; Agrobacterium-mediated plant transformation: the biology behind the "Gene-jockeying" tool; Microbiol. Mol. Biol. Rev. 2003 67(1), 16-37), different plant selections can be transformed at different rates; therefore, to recover a target number of transformed plants, it can be expected that a selection having a low transformation rate would require use of a higher number of explants than a selection having a high transformation rate. A selection having a low transformation rate would require at least about 200 explants to recover about 1 transformed plant.

Instead of further optimizing the transformation protocol, a plant breeding backcross technique well known to those skilled in the art was used to transfer the mutation resulting in the increased zeaxanthin to lutein ratio of selection 124-257 to a selection having a higher transformation rate. Several Tagetes erecta marigold plants were identified as having acceptable transformation rates, and from these Tagetes erecta marigold plant named 13819 was selected. Tagetes erecta 13819 is a proprietary breeding selection of PanAmerican Seed located at 622 Town Road, West Chicago, Illinois 60185.

In the backcross program, selection 124-257 was used as the female parent in a cross with a selection of 13819 as the male parent. The resulting population was identified as 11754, and from this population a plant identified as 11754-2F was selected based on its hybrid characteristics. Plant 11754-2F was selfed and from this population plant identified as 11754-2F-1 was selected based on carotenoid profile and plant habit. Plant 11754-2F-1 was used as male parent in a cross with 13819 as the female parent. The resulting population was identified as 31360, and from this population a plant identified as 31360-2 was selected based on carotenoid profile and plant habit. Plant 31360-2 was selfed and from the resulting population, plants 31360-2-08 and 31360-2-09 were selected based on carotenoid profile, total carotenoid concentration, and plant habit. Both selections were selfed and seed from the cross was used to test transformation rates, and the seedlings from both selections were found to have acceptable transformation rates. In addition, the resulting plants from the selfed 31360-2-08 plant were found to be uniform for carotenoid profile, carotenoid concentration, and plant habit. The resulting plants from the selfed 31360-2-09 plant were found to segregate for total carotenoid concentration and plant habit characteristics. From the selfed 31360-2-09 population, a plant identified as 31360-2-09-08 was selected based on carotenoid profile, total carotenoid concentration, and plant habit. The selfed population from the 31360-2-09-08 plant was found to be uniform for carotenoid profile, carotenoid concentration, and to have an acceptable transformation rate.

### EXAMPLE 2: Tagetes transformation protocol for 31360-2-08 and 31360-2-09

Seeds of Tagetes erecta line 31360-2-08 were disinfected with 2% NaOCl solution for 10 minutes followed by three washes with autoclaved distilled water. Afterwards seeds were dried and can be stored under aseptic conditions at room temperature for a period of up to two weeks before in vitro germination. Germination occurred on solidified MS medium (Murashige, T., and Skoog, F., A revised medium for rapid growth and bioassays with tobacco tissue cultures. Physiol. Plant. 15, 473-497,1962) in a 16/8 h light/darkness photoperiod for 1-3 weeks. Cotyledonary segments were prepared and used as primary target material for transformation. These segments were inoculated for 20 minutes in liquid MS medium containing Agrobacterium tumefaciens strain EHA105 cells at an OD₆₀₀ of 0.1. The binary vector contained the gene pat (plus additional gene cassettes encoding effect genes) and allows therefore phosphinothricin (PPT) and/or BASTA selection. Explants are co-cultured for a period of 6 days on MS medium (pH 5.8) solidified with 0.8% agar and supplemented with 1 mg/l 3-indole-3-acetic acid (IAA), 3 mg/l indole-3-butyric acid (IBA), 500 mg/l 2-(N-morpholino) ethanesulfonic acid (MES) and 2% sucrose. Cultivation occurred under controlled conditions at 21°C, 35-40 µmol m⁻² s⁻¹ white light intensity and 16/8 light/darkness rhythm. Shoots were induced on cotyledon explants on fresh MS medium, adjusted to pH 5.5, as described before supplemented with 500 mg/l Timentin, 1 mg/l PPT, 5 mg/l Silver Nitrate (AgNO₃). Second to fourth subcultures were done onto fresh MS medium following the formulation described above at pH 5.8 and 15 days subculture period. The newly formed shoot buds were transferred to a new medium to promote shoot regeneration. The shoot regeneration medium follows the formulation of MS supplemented with 0,7% agar, 250 mg/l Timentin, 1 mg/l PPT, 5 mg/l AgNO₃, 1 mg/l IAA, 3 mg/l 6-Benzylaminopurine (BAP), 500 mg/l MES and 2% sucrose and was adjusted to pH 5.8. Three subcultures were promoted in a 15 days subculture period. Regenerated shoots were then transferred onto elongation medium (MS) supplemented with 0.7% agar, 250 mg/l Timentin, 1 mg/l PPT, 5 mg/l AgNO₃, 0.5 mg/l IAA, 0.5 mg/l gibberellic acid (GAs), 500 mg/l MES, 2% sucrose, pH 5.8. Three subcultures were performed, each for 15 days. Well elongated shoots (1.5 - 3.5 cm in length) with well expanded leaves were transferred onto rooting MS medium solidified with 0.7% agar and supplemented with 250 mg/l Timentin, 1 mg/l PPT, 0,5 mg/l IBA, 500 mg/l MES, 2% sucrose and adjusted to pH 5.8. Leaf material from rooted plants was analyzed by qPCR for the selection marker gene in order to confirm transgenicity and to determine the copy number of the construct integrated into the genome. After four weeks the well rooted transgenic shoots were transferred to ex vitro-conditions at the greenhouse. Hardening of plants in soil could be achieved with inverted funnels. They prevented dehydration of the plantlets. Afterwards plants were transferred into bigger pots with soil to promote growth and development until flowering under greenhouse conditions.

### EXAMPLE 3: Biochemical analytics protocol

About 5 mg freeze-dried material of Tagetes petals were homogenized (e.g. via milling) (manufacturer: Retsch, Germany) at twice for 1 minute at room temperature using stainless steal beads). The homogenous material was extracted with acetone, usually 5times with 500µl acetone till the supernatant is colorless. Supernatants were combined and evaporated to dryness using a SpeedVac concentrator.

### Xanthophyll analysis

The concentrated carotenoids of 5 mg dry petal material were dissolved in 180 µl of acetone and eventually briefly sonicated. For saponification, 20 µl of 10% KOH (in methanol) was added and incubated for 30 minutes under constant shaking (1000 rpm) in the dark at room temperature. The reaction was stopped by the addition of 25-35 µl 1 M HCl (till neutral pH value was reached). Samples were centrifuged for 10 min at 13,000 rpm to pellet debris and analyzed by HPLC.

### Ketocarotenoid analysis

The concentrated carotenoids of 5 mg dry petal material were transferred to an anaerobic gloves box (e.g. manufacturer COY Laboratory Products Inc., USA) which allow chemical reactions at very low oxygen levels (around 1-10 ppm). Inside the glove box, the pellet was dissolved in 200 µl toluene. 200 µl of fresh 0,5 M sodium methoxide was added, the solutions were thoroughly mixed, and reaction proceeded for 10 min at 9°C (or lower) at constant shaking at 1000 rpm. The reaction was stopped by adding 200 µl 0,5 M sulfuric acid to neutralize the reaction. Perhaps, more sulfuric acid needs to be added for neutralization. Reaction vials were taken out of the anaerobic chamber, briefly centrifuged, and carotenoids were extracted with toluene (about 5 times with 200 µl). The combined toluene extracts were combined and evaporated to dryness. Carotenoids were re-suspended in small volume for HPLC analysis.

### HPLC separation of carotenoids

The analysis of samples prepared according to the procedure described above was done under the following HPLC conditions:

| | |
|---|---|
| HPLC column: | Prontosil C30, 250 x 4.6 mm, (Bischoff, Leonberg, Germany) |
| Flow rate: | 1.0 ml/min |
| Eluents: | Solvent A - 100% methanol |
| | Solvent B - 80% methanol, 0.2% ammonium acetate |
| | Solvent C - 100% t-butyl-methyl ether |
| Detection: | 300-530 nm |

**Gradient profile:**

| Time (min) | Flow rate | % Solvent A | % Solvent B | % Solvent C |
|---|---|---|---|---|
| 1.00 | 1.0 | 95.0 | 5.0 | 0 |
| 12.00 | 1.0 | 95.0 | 5.0 | 0 |
| 12.10 | 1.0 | 80.0 | 5.0 | 15.0 |
| 22.00 | 1.0 | 76.0 | 5.0 | 19.0 |
| 22.10 | 1.0 | 66.5 | 5.0 | 28.5 |
| 38.00 | 1.0 | 15.0 | 5.0 | 80.0 |
| 45.00 | 1.0 | 95.0 | 5.0 | 0 |
| 46.0 | 1.0 | 95.0 | 5.0 | 0 |

Some typical retention times for carotenoids were: violaxanthin at about 11.7 min, astaxanthin at about 17.7 min, adonixanthin at about 19 min, adonirubin at about 19.9 min, zeaxanthin at about 21 min and beta-carotene at 32 min.

### Materials and General Methods

Unless indicated otherwise, chemicals and reagents in the Examples were obtained from Sigma Chemical Company (St. Louis, MO), restriction endonucleases were from New England Biolabs (Beverly, MA) or Roche (Indianapolis, IN), oligonucleotides were synthesized by MWG Biotech Inc. (High Point, NC), and other modifying enzymes or kits regarding biochemicals and molecular biological assays were from Clontech (Palo Alto, CA), Pharmacia Biotech (Piscataway, NJ), Promega Corporation (Madison, WI), or Stratagene (La Jolla, CA). Materials for cell culture media were obtained from Gibco/BRL (Gaithersburg, MD) or DIFCO (Detroit, MI). The cloning steps carried out for the purposes of the present invention, such as, for example, restriction cleavages, agarose gel electrophoresis, purification of DNA fragments, transfer of nucleic acids to nitrocellulose and nylon membranes, linking DNA fragments, transformation of E. coli cells, growing bacteria, multiplying phages and sequence analysis of recombinant DNA, are carried out as described by Sambrook (1989). The sequencing of recombinant DNA molecules is carried out using ABI laser fluorescence DNA sequencer following the method of Sanger (Sanger 1977).

### EXAMPLE 4: Expression of the ketolase gene from Haematococcus pluvialis optimized for expression in Tagetes erecta

### Determination of Tagetes codon usage

A cDNA library was created from Tagetes petals (cv. Scarletade) using state-of-the art technology (e.g. Chenchik et al., Clontechniques X(1):5-8, 1995; A Laboratory guide to RNA: Isolation, Analysis and Synthesis, edited by P.A:Krieg (Wiley-Liss. Inc, 1996; or as described in the instruction manuals of companies like Clontech, Invitrogen). A collection of several thousand ESTs (11118 clones) was sequenced and contig sequences generated. These sequences were the basis for generation of a codon-usage table which included 778346 good codons and 4693 good ORFs.

**Codon usage table for Tagetes erecta:**

| A (Alanine): | G (Glycine): | M (Methionine): | S (Serine): |
|---|---|---|---|
| GCA: 32.934 % | GGA: 29.938 % | AUG: 100.0 % | AGU: 18.542 % |
| GCU: 41.017 % | GGU: 35.802 % | | AGC: 11.764 % |
| GCG: 10.628 % | GGG: 19.598 % | | UCA: 25.831 % |
| GCC: 15.419% | GGC: 14.66 % | | UCU: 24.296 % |
| | | | UCG: 9.335 % |
| | | | UCC: 10.23% |

| C (Cysteine): | H (Histidine): | N (Asparagine): | T (Threonine): |
|---|---|---|---|
| UGU: 61.935 % | CAU: 66.666 % | AAU: 57.021 % | ACA: 34.496 % |
| UGC: 38.064 % | CAC: 33.333% | AAC: 42.978 % | ACU: 31.976 % |
| | | | ACG: 11.821 % |
| | | | ACC: 21.705 % |

| D (Aspartate): | I (Isoleucine): | P (Proline): | V (Valine): |
|---|---|---|---|
| GAU: 71.329 % | AUA: 24.475 % | CCA: 36.323 % | GUA: 15.244 % |
| GAC: 28.67 % | AUU: 46.503 % | CCU: 34.792 % | GUU: 44.055 % |
| | AUC: 29.02 % | CCG: 15.536 % | GUG: 26.153 % |
| | | CCC: 13.347 % | GUC: 14.545 % |

| E (Glutamate): | K (Lysine): | Q (Glutamine): | W (Tryptophan): |
|---|---|---|---|
| GAA: 60.0 % | AAA: 54.281 % | CAA: 62.078 % | UGG: 100.0 % |
| GAG: 40.0 % | AAG: 45.718 % | CAG: 37.921 % | |

| F (Phenylalanine): | L (Leucine): | R (Arginine): | Y (Tyrosine): |
|---|---|---|---|
| UUU: 64.235 % | UUA: 17.23 % | AGA: 29.918 % | UAU: 59.87 % |
| UUC: 35.764 % | UUG: 24.754 % | AGG: 19.877 % | UAC: 40.129 % |
| | CUA: 10.25 % | CGA: 13.934 % | |
| | CUU: 27.044 % | CGU: 17.622 % | |
| | CUG: 10.468 % | CGG: 10.45 % | |
| | CUC: 10.25 % | CGC: 8.196 % | |

! (Stop):
UAA: 33.333 %
UAG: 33.333 %
UGA: 33.333 %

### Expression experiments

The protein sequence of the Haematococcus pluvialis beta carotene ketolase (SEQ ID NO:1) was used to obtain its reverse translation considering the specific codon usage of Tagetes as outlined above. In addition, the amino acid sequence of the Pisum sativum Rubisco small subunit (RbcS) transit peptide (SEQ ID NO:2) was reverse translated considering the Tagetes codon usage. All genetic elements were optimized using the software LETO1.0 from Entelechon.

The synthetic DNA fragment (SEQ ID NO:3) corresponding to the optimized RbcS transit peptide coding sequence fused in frame to the optimized Haematococcus pluvialis beta carotene ketolase coding sequence was used in subsequent cloning steps to generate binary plant transformation vector VC-SIW122-13 (SEQ ID NO:4) which is based on the vector VC-LLL544-1qcz backbone (SEQ ID NO:5). All cloning steps were carried out following standard molecular biology protocols.

The T-DNA of VC-SIW122-13 (SEQ ID NO:4) contains a cassette for regeneration of plants under phosphinothricin selection pressure comprising the nos (nopaline synthase) promoter, the coding region of a synthetic phosphinothricin acetyltransferase gene, and the nos terminator. The second expression cassette comprises the Brassica napus plastid-associated protein X (PAPX) promoter, the Pisum sativum RbcS transit peptide coding sequence optimized for expression in Tagetes, the Haematococcus pluvialis beta carotene ketolase gene coding sequence optimized for Tagetes, and the Solanum tuberosum cathepsin D Inhibitor (CAT) terminator.

The following sequences are used:
SEQ ID NO:1: HP BKT (amino acid sequence of Haematococcus pluvialis beta carotene ketolase coding sequence (329 aa))
SEQ ID NO:2: tp-RbcS (Amino acid sequence of Pisum sativum Rubisco small subunit (RbcS) transit peptide)
SEQ ID NO:3: synthetic DNA fragment

| | |
|---|---|
| Position 1 to 168 | Pisum sativum RbcS transit peptide coding sequence optimized for expression in Tagetes (168 bp), underlined |
| Position 169 to 1158 | Haematococcus pluvialis beta carotene ketolase gene, coding sequence optimized for expression in Tagetes (990 bp) |

SEQ ID NO:4: VC-SIW122-13 T-DNA (T-DNA region of binary vector)

| | |
|---|---|
| Position 1 to 215 | Left border |
| Position 218 to 505 | Nos (nopaline synthase) gene promoter |
| Position 518 to 1069 | Phosphinothricin Acetyltransferase synthetic gene/CDS |
| Position 1139 to 1391 | Nos (nopaline synthase) gene terminator |
| Position 1734 to 2764 | Brassica napus plastid-associated protein X (PAPX) promoter |
| Position 1769 to 2963 | Pisum sativum Rubisco small subunit (RbcS) transit peptide coding sequence optimized for expression in Tagetes |
| Position 2967 to 3956 | Haematococcus pluvialis beta-carotene ketolase gene coding sequence optimized for expression in Tagetes |
| Position 3981 to 4209 | Solanum tuberosum Cathepsin D Inhibitor (CAT) terminator |
| Position 4325 to 4470 | Right border |

SEQ ID NO:5: VC-LLL544-1 qcz (binary vector backbone)

| | |
|---|---|
| Position 1 to 146 | Right border |
| Position 320 to 1111 | Adenyltransferase [aadA] gene coding region |
| Position 1560 to 2241 | ColE1 E. coli origin of replication |
| Position 2615 to 2809 | pVS1 origin (complementary) |
| Position 2413 to 5682 | pVS1 replicon (complementary) |
| Position 5691 to 5905 | Left border |
| Position 5606 to 5916 | Placeholder (to be replaced by respective T-DNA) |

### EXAMPLE 5: Analysis of carotenoids of Tagetes plants transformed with Haematococcus pluvialis beta carotene ketolase and of Tagetes plants transformed with Haematococcus pluvialis beta carotene ketolase optimized for expression in Tagetes erecta

After transformation of Tagetes explants of 31360-2-08 with the binary vector VC-SIW122-13 (comprising Haematococcus pluvialis beta carotene ketolase optimized for expression in Tagetes erecta), several transgenic plants were obtained which were named SIW122. These plants were analyzed for individual yellow carotenoids, the natural occurring and endogenous xanthophylls, and the newly formed ketocarotenoids, especially for astaxanthin, canthaxanthin, echinenone, 3'-hydroxyechinenone, 3-hydroxy-echinenone, phoenicoxanthin (= adonirubin) and adonixanthin.

**Table 1: Individual carotenoids in petals of transgenic Tagetes SIW122**

| | ng Carotenoid/mg | | | | | % |
|---|---|---|---|---|---|---|
| Plant | Total Ketos | Astaxanthin | Phoenicoxanthin | beta-Caroten e | Epoxide s & Zea | Asta/ ketos |
| M1-SIW122-1-4106-2-057 | 1835 | 778 | 544 | 6110 | 5333 | 42% |
| M1-SIW122-1-4106-2-081 | 1805 | 604 | 555 | 4120 | 4484 | 33% |
| M1-SIW122-1-4106-2-087 | 1753 | 843 | 497 | 6400 | 5081 | 48% |
| M1-SIW122-1-4106-2-100 | 2291 | 1056 | 605 | 4901 | 4398 | 46% |
| M1-SIW122-1-4106-2-110 | 2231 | 1075 | 651 | 4838 | 6129 | 48% |
| M1-SIW122-1-4106-2-116 | 2617 | 1554 | 615 | 10255 | 5349 | 59% |

Listed values represent individual carotenoids in percent of total carotenoids, extracted and analyzed as described. The carotenoid extract was prepared from fully opened Tagetes flowers. Values refer to dry weight.

**Table 2: Individual ketocarotenoids in petals of transgenic Tagetes SIW122**

| | ng Carotenoid/mg | | | | | |
|---|---|---|---|---|---|---|
| Plant | Astaxanthin | Adonixanthin | Canthaxanthin | 3' Hy- nicodroxy | Phoexanthin | Total ketos |
| M1-SIW122-1-4106-2-057 | 778 | 62 | 409 | 42 | 544 | 1835 |
| M1-SIW122-1-4106-2-081 | 604 | 40 | 560 | 46 | 555 | 1805 |
| M1-SIW122-1-4106-2-087 | 843 | 64 | 307 | 41 | 497 | 1753 |
| M1-SIW122-1-4106-2-100 | 1056 | 64 | 523 | 43 | 605 | 2291 |
| M1-SIW122-1-4106-2-110 | 1075 | 54 | 409 | 42 | 651 | 2231 |
| M1-SIW122-1-4106-2-116 | 1554 | 80 | 342 | 25 | 615 | 2617 |

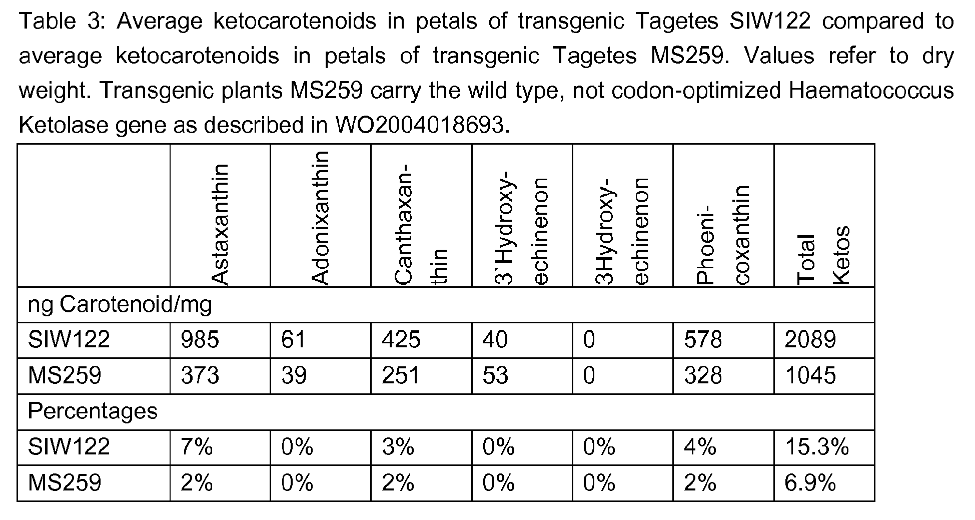

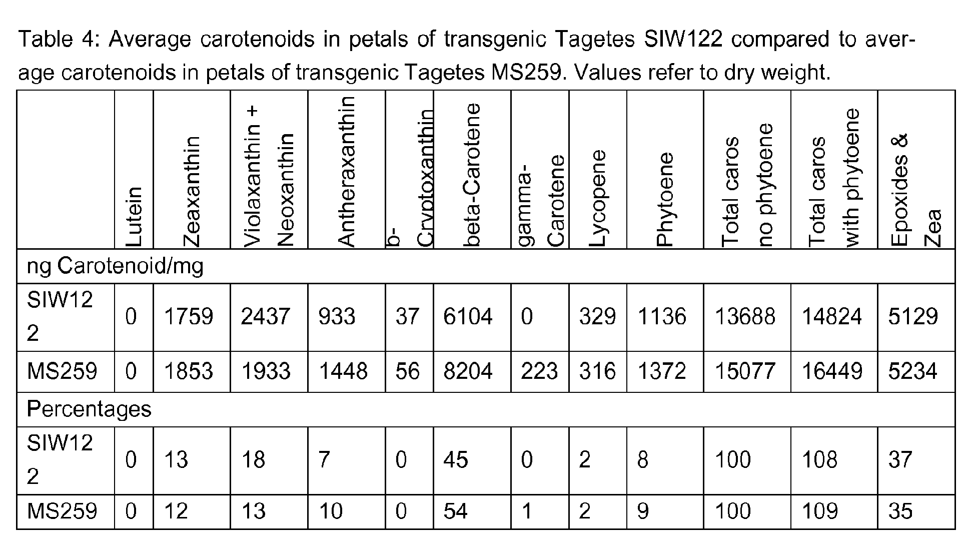

Legend for table 1 to table 4:
- "Total caros": total amounts of all carotenoids extracted from Tagetes petals
- "Total ketos": sum of all ketocarotenoids extracted from Tagetes petals (canthaxanthin, phoenicoxanthin, astaxanthin, adonixanthin, echinenone, 3'- and 3-hydroxy-echinenone).
- "Zea": designation for zeaxanthin
- "3'-Hydroxy" : designation for 3'-hydroxyechinenone
- "Epoxides": designation for the combined concentration of the carotenoid epoxides violaxanthin, antheraxanthin and neoxanthin

### EXAMPLE 6: Expression of the ketolase gene from Scenedesmus vacuolatus optimized for expression in Tagetes erecta

The protein sequence of the Scenedesmus vacuolatus beta carotene ketolase (SEQ ID NO:6) was used to obtain its reverse translation considering the specific codon usage of Tagetes as outlined in Example 4. In addition, the amino acid sequence of the Pisum sativum Rubisco small subunit (RbcS) transit peptide (SEQ ID NO:2) was reverse translated considering the Tagetes codon usage.

The synthetic DNA fragment (SEQ ID NO:7) corresponding to the optimized RbcS transit peptide coding sequence fused in frame to the optimized Scenedesmus vacuolatus beta carotene ketolase coding sequence was used in subsequent cloning steps to generate binary plant transformation vector VC-SIW182-6 (SEQ ID NO:8) which is based on the vector VC-LLL544-1qcz backbone (SEQ ID NO:5). All cloning steps were carried out following standard molecular biology protocols.

The T-DNA of VC-SIW182-6 (SEQ ID NO:8) contains a cassette for regeneration of plants under phosphinothricin selection pressure comprising the nos (nopaline synthase) promoter, the coding region of a synthetic phosphinothricin acetyltransferase gene, and the nos terminator.The second expression cassette comprises the Brassica napus plastid-associated protein X (PAPX) promoter, the Pisum sativum RbcS transit peptide coding sequence optimized for expression in Tagetes, the Scenedesmus vacuolatus beta carotene ketolase gene coding sequence optimized for Tagetes, and the Solanum tuberosum cathepsin D Inhibitor (CAT) terminator.

The following sequences are used: SEQ ID NO:2 and 5 from Example 4.
SEQ ID NO:6: SV211 BKT (amino acid sequence of Scenedesmus vacuolatus beta carotene ketolase coding sequence (331 aa)
SEQ ID NO:7: synthetic DNA fragment

| | |
|---|---|
| Position 1 to 171 | Pisum sativum RbcS transit peptide coding sequence optimized for expression in Tagetes (171 bp), underlined |
| Position 172 to 1158 | Scenedesmus vacuolatus beta carotene ketolase gene, coding sequence optimized for expression in Tagetes (996 bp) |
| SEQ ID NO:8: | VC-SIW182-6 (T-DNA region of binary vector) |
| Position 1 to 215 | Left border |
| Position 218 to 505 | Nos (nopaline synthase) gene promoter |
| Position 518 to 1069 | Phosphinothricin Acetyltransferase synthetic gene/CDS |
| Position 1139 to 1391 | Nos (nopaline synthase) gene terminator |
| Position 1582 to 1813 | Solanum tuberosum Cathepsin D Inhibitor (CAT) terminator (complementary) |
| Position 1835 to 2830 | Scenedesmus vacuolatus beta-carotene ketolase gene coding sequence optimized for expression in Tagetes (complementary) |
| Position 2831 to 3001 | Pisum sativum Rubisco small subunit (RbcS) transit peptide coding sequence optimized for expression in Tagetes (complementary) |
| Position 3033 to 4063 | Brassica napus plastid-associated protein X (PAPX) promoter (complementary) |
| Position 4331 to 4476 | Right border |

### EXAMPLE 7: Analysis of carotenoids of Tagetes plants transformed with Scenedesmus vacuolatus beta carotene ketolase and of Tagetes plants transformed with Scenedesmus vacuolatus beta carotene ketolase optimized for expression in Tagetes erecta

After transformation of Tagetes explants of 31360-2-08 with the binary vector VC-SIW182-6 (comprising Scenedesmus vacuolatus beta carotene ketolase optimized for expression in Tagetes erecta), several transgenic plants were obtained which were named SIW182. These plants were analyzed for individual yellow carotenoids, the natural occurring and endogenous xanthophylls, and the newly formed ketocarotenoids, especially for astaxanthin, canthaxanthin, echinenone, 3'-hydroxechinenone, 3-hydroxy-echinenone, phoenicoxanthin (= adonirubin) and adonixanthin.

**Table 5: Individual carotenoids in petals of transgenic Tagetes SIW182**

| | ng Carotenoid/mg | | | | | | % |
|---|---|---|---|---|---|---|---|
| Plant | Total Ketos | Astaxanthin | Phoenicoxanthin | beta-Carotene | Epoxi des | Zeaxa nthin | Asta/ ketos |
| M1-SIW182-6-3 | 7823 | 5530 | 1570 | 841 | 1099 | 96 | 75 |
| M1-SIW182-6-4 | 8076 | 5254 | 1968 | 602 | 812 | 112 | 79 |
| M1-SIW182-6-6 | 7778 | 4340 | 2370 | 462 | 385 | 130 | 84 |
| M1-SIW182-6-7 | 6755 | 4578 | 1425 | 805 | 877 | 99 | 76 |
| M1-SIW182-6-8 | 8696 | 5925 | 1933 | 577 | 962 | 118 | 80 |
| M1-SIW182-6-10 | 7985 | 5721 | 1742 | 157 | 564 | 67 | 87 |
| M1-SIW182-6-18 | 11518 | 8404 | 2350 | 318 | 1198 | 86 | 85 |
| M1-SIW182-6-27 | 8815 | 6650 | 1615 | 321 | 1201 | 80 | 82 |
| M1-SIW182-6-32 | 6908 | 4870 | 1442 | 302 | 753 | 76 | 81 |
| M1-SIW182-6-39 | 9089 | 7537 | 1238 | 143 | 1337 | 65 | 83 |
| M1-SIW182-6-45 | 8237 | 6930 | 1008 | 236 | 1711 | 85 | 78 |

Listed values represent individual carotenoids in percent of total carotenoids, extracted and analyzed as described. The carotenoid extract was prepared from fully opened Tagetes flowers. Values refer to dry weight.

**Table 6: Individual ketocarotenoids in petals of transgenic Tagetes SIW182**

| | ng Carotenoid/mg | | | |
|---|---|---|---|---|
| Plant | Astaxanthin | Canthaxanthin | Phoenicoxanthin | Total ketos |
| M1-SIW182-6-3 | 5530 | 723 | 1570 | 7823 |
| M1-SIW182-6-4 | 5254 | 854 | 1968 | 8076 |
| M1-SIW182-6-6 | 4340 | 1068 | 2370 | 7778 |
| M1-SIW182-6-7 | 4578 | 752 | 1425 | 6755 |
| M1-SIW182-6-8 | 5925 | 838 | 1933 | 8696 |
| M1-SIW182-6-10 | 5721 | 522 | 1742 | 7985 |
| M1-SIW182-6-18 | 8404 | 764 | 2350 | 11518 |
| M1-SIW182-6-27 | 6650 | 550 | 1615 | 8815 |
| M1-SIW182-6-32 | 4870 | 596 | 1442 | 6908 |
| M1-SIW182-6-39 | 7537 | 314 | 1238 | 9089 |
| M1-SIW182-6-45 | 6930 | 299 | 1008 | 8237 |

### EXAMPLE 8: Expression of the ketolase gene from Chlorella zoofingiensis optimized for expression in Tagetes erecta

The protein sequence of the Chlorella zoofingiensis beta carotene ketolase (SEQ ID NO:9) was used to obtain its reverse translation considering the specific codon usage of Tagetes as outlined in Example 4. In addition, the amino acid sequence of the Pisum sativum Rubisco small subunit (RbcS) transit peptide (SEQ ID NO:2) was reverse translated considering the Tagetes codon usage.

The synthetic DNA fragment (SEQ ID NO:10) corresponding to the optimized RbcS transit peptide coding sequence fused in frame to the optimized Chlorella zoofingiensis beta carotene ketolase coding sequence was used in subsequent cloning steps to generate binary plant transformation vector VC-SIW198-1 (SEQ ID NO:11) which is based on the vector VC-LLL544-1qcz backbone (SEQ ID NO:5). All cloning steps were carried out following standard molecular biology protocols.

The T-DNA of VC-SIW198-1 (SEQ ID NO:11) contains a cassette for regeneration of plants under phosphinothricin selection pressure comprising the nos (nopaline synthase) promoter, the coding region of a synthetic phosphinothricin acetyltransferase gene, and the nos terminator.The second expression cassette comprises the Brassica napus plastid-associated protein X (PAPX) promoter, the Pisum sativum RbcS transit peptide coding sequence optimized for expression in Tagetes, the Chlorella zoofingiensis beta carotene ketolase gene coding sequence optimized for Tagetes, and the Solanum tuberosum cathepsin D Inhibitor (CAT) terminator.

The following sequences were used in addition to SEQ ID NO:2 and 5 of Example 4
SEQ ID NO:9: CZ BKT (amino acid sequence of Chlorella zoofingiensis beta carotene ketolase coding sequence (312 aa)
SEQ ID NO:10: synthetic DNA fragment

| | |
|---|---|
| Position 1 to 171 | Pisum sativum RbcS transit peptide coding sequence optimized for expression in Tagetes (171 bp), underlined |
| Position 172 to 1110 | Chlorella zoofingiensis beta carotene ketolase gene, coding sequence optimized for expression in Tagetes (939 bp) |
| SEQ ID NO:11: | VC-SIW198-1 (T-DNA region of binary vector) |
| Position 1 to 215 | Left border |
| Position 218 to 505 | Nos (nopaline synthase) gene promoter |
| Position 518 to 1069 | Phosphinothricin Acetyltransferase synthetic gene/CDS |
| Position 1139 to 1391 | Nos (nopaline synthase) gene terminator |
| Position 1582 to 1813 | Solanum tuberosum Cathepsin D Inhibitor (CAT) terminator (complementary) |
| Position 1823 to 2761 | Chlorella zoofingiensis beta-carotene ketolase gene coding sequence optimized for expression in Tagetes (complementary) |
| Position 2762 to 2932 | Pisum sativum Rubisco small subunit (RbcS) transit peptide coding sequence optimized for expression in Tagetes (complementary) |
| Position 2968 to 3998 | Brassica napus plastid-associated protein X (PAPX) promoter (complementary) |
| Position 4266 to 4411 | Right border |

### EXAMPLE 9: Analysis of carotenoids of Tagetes plants transformed with Chlorella zoofingiensis beta carotene ketolase and of Tagetes plants transformed with Chlorella zoofingiensis beta carotene ketolase optimized for expression in Tagetes erecta

After transformation of Tagetes explants of 31360-2-08 with the binary vector VC-SIW198-1 (comprising Chlorella zoofingiensis beta carotene ketolase optimized for expression in Tagetes erecta), several transgenic plants were obtained which were named SIW198. These plants were analyzed for individual yellow carotenoids, the natural occurring and endogenous xanthophylls, and the newly formed ketocarotenoids, especially for astaxanthin, canthaxanthin, echinenone, 3'-hydroxechinenone, 3-hydroxy-echinenone, phoenicoxanthin (= adonirubin) and adonixanthin.

Tagetes plants SIW122 exhibit a clearly orange flower phenotype due to the accumulation of ketocarotenoids produced in the petals of the flowers. This phenotype is even more pronounced in Tagetes plants SIW182. Several flowers of SIW182 are clearly intensely red due to the high concentrations of ketocarotenoids in those petals. In contrast, flowers of SIW 198 showed only minor to moderate phenotype. Many flowers showed a yellow/orangish phenotype which is not much distinguishable from the control plants. A few flowers showed a slight orange phenotype due to low amounts of ketocarotenoids accumulating in the flowers.

### EXAMPLE 10: Expression of the ketolase gene from Chlamydomonas reinhardtii optimized for expression in Tagetes erecta

The protein sequence of the Chlamydomonas reinhardtii beta carotene ketolase (SEQ ID NO:12) was used to obtain its reverse translation considering the specific codon usage of Tagetes as outlined in Example 4. In addition, the amino acid sequence of the Pisum sativum Rubisco small subunit (RbcS) transit peptide (SEQ ID NO:2) was reverse translated considering the Tagetes codon usage.

The synthetic DNA fragment (SEQ ID NO:13) corresponding to the optimized RbcS transit peptide coding sequence fused in frame to the optimized Chlamydomonas reinhardtii beta carotene ketolase coding sequence was used in subsequent cloning steps to generate binary plant transformation vector VC-SIW195-1 (SEQ ID NO:14) which is based on the vector VC-LLL544-1qcz backbone (SEQ ID NO:5). All cloning steps were carried out following standard molecular biology protocols.

The T-DNA of VC-SIW195-1 (SEQ ID NO:14) contains a cassette for regeneration of plants under phosphinothricin selection pressure comprising the nos (nopaline synthase) promoter, the coding region of a synthetic phosphinothricin acetyltransferase gene, and the nos terminator.The second expression cassette comprises the Brassica napus plastid-associated protein X (PAPX) promoter, the Pisum sativum RbcS transit peptide coding sequence optimized for expression in Tagetes, the Chlamydomonas reinhardtii beta carotene ketolase gene coding sequence optimized for Tagetes, and the Solanum tuberosum cathepsin D Inhibitor (CAT) terminator.

The following sequences were used in addition to SEQ ID NO:2 and 5 of Example 4
SEQ ID NO:12: CR BKT (amino acid sequence of Chlamydomonas reinhardtii beta carotene ketolase coding sequence (328 aa)

The ketolase version used has been shortened at the C-terminus of the protein. When aligned with known ketolases of public databases, the Chlamydomonas ketolase showed an extended C-terminus not found in other ketolases of proven function. In addition, the extension showed longer stretches of repetitive alanine, serine and glycine stretches.
SEQ ID NO:13: synthetic DNA fragment

| | |
|---|---|
| Position 1 to 171 | Pisum sativum RbcS transit peptide coding sequence optimized for expression in Tagetes (171 bp), underlined |
| Position 172 to 1158 | Chlamydomonas reinhardtii beta carotene ketolase gene, 3'-shortened coding sequence optimized for expression in Tagetes (987 bp) |

SEQ ID NO:14: VC-SIW195-1 (T-DNA region of binary vector)

| | |
|---|---|
| Position 1 to 215 | Left border |
| Position 218 to 505 | Nos (nopaline synthase) gene promoter |
| Position 518 to 1069 | Phosphinothricin Acetyltransferase synthetic gene/CDS |
| Position 1139 to 1391 | Nos (nopaline synthase) gene terminator |
| Position 1582 to 1813 | Solanum tuberosum Cathepsin D Inhibitor (CAT) terminator (complementary) |
| Position 1835 to 2821 | Chlamydomonas reinhardtii beta-carotene ketolase gene coding sequence optimized for expression in Tagetes (complementary) |
| Position 2822 to 2992 | Pisum sativum Rubisco small subunit (RbcS) transit peptide coding sequence optimized for expression in Tagetes (complementary) |
| Position 3028 to 4058 | Brassica napus plastid-associated protein X (PAPX) promoter (complementary) |
| Position 4326 to 4471 | Right border |

### EXAMPLE 11: Analysis of carotenoids of Tagetes plants transformed with Chlamydomonas reinhardtii beta carotene ketolase and of Tagetes plants transformed with Chlamydomonas reinhardtii beta carotene ketolase optimized for expression in Tagetes erecta

After transformation of Tagetes explants of 31360-2-09 with the binary vector VC-SIW195-1 (comprising Chlamydomonas reinhardtii beta carotene ketolase optimized for expression in Tagetes erecta), several transgenic plants were obtained which were named SIW195. These plants were analyzed for individual yellow carotenoids, the natural occurring and endogenous xanthophylls, and the newly formed ketocarotenoids, especially for astaxanthin, canthaxanthin, echinenone, 3'-hydroxechinenone, 3-hydroxy-echinenone, phoenicoxanthin (= adonirubin) and adonixanthin.

**Table 7: Individual carotenoids in petals of transgenic Tagetes SIW195**

| | ng Carotenoid/mg | | | | | | % |
|---|---|---|---|---|---|---|---|
| Plant | Total Ketos | Astaxanthin | Phoenicoxanthin | beta-Carote ne | Epoxides | Zeaxanthin | Asta/ ketos |
| M4-SIW195-5 | 4166 | 2934 | 550 | 2009 | 2614 | 633 | 70 |
| M4-SIW195-30 | 4469 | 3230 | 601 | 1719 | 2380 | 754 | 72 |
| M4-SIW195-36 | 3179 | 2268 | 446 | 1375 | 2545 | 704 | 71 |
| M4-SIW195-45 | 3561 | 2301 | 546 | 1854 | 2233 | 687 | 65 |
| M4-SIW195-46 | 4274 | 3198 | 525 | 1429 | 2342 | 554 | 75 |
| M4-SIW195-53 | 4645 | 3119 | 712 | 2083 | 2669 | 620 | 67 |
| M4-SIW195-57 | 4127 | 2899 | 612 | 1798 | 2779 | 671 | 70 |
| M4-SIW195-61 | 4044 | 2727 | 605 | 2111 | 2428 | 911 | 67 |
| M4-SIW195-62 | 1201 | 613 | 283 | 1811 | 2953 | 3039 | 51 |
| M4-SIW195-77 | 4409 | 3047 | 626 | 1757 | 3195 | 1217 | 69 |
| M4-SIW195-78 | 4570 | 3038 | 702 | 2077 | 2546 | 827 | 66 |
| M4-SIW195-79 | 3337 | 2420 | 434 | 1305 | 2739 | 798 | 73 |
| M4-SIW195-82 | 2510 | 1656 | 364 | 1744 | 3413 | 1747 | 66 |
| M4-SIW195-89 | 3555 | 2540 | 513 | 1503 | 3533 | 716 | 71 |
| M4-SIW195-94 | 3749 | 2741 | 442 | 1642 | 2310 | 1014 | 73 |
| M4-SIW195-99 | 3415 | 2263 | 538 | 1801 | 2204 | 877 | 66 |
| M4-SIW195-109 | 4159 | 2523 | 731 | 2075 | 2347 | 811 | 61 |
| M4-SIW195-118 | 3844 | 2373 | 614 | 2448 | 2431 | 897 | 62 |
| M4-SIW195-118 | 3353 | 2227 | 497 | 1572 | 2049 | 838 | 66 |
| M4-SIW195-126 | 4744 | 2875 | 793 | 2091 | 1919 | 597 | 61 |
| M4-SIW195-127 | 3280 | 2069 | 511 | 1755 | 1567 | 644 | 63 |
| M4-SIW195-136 | 3093 | 2079 | 444 | 1817 | 2088 | 987 | 67 |
| M4-SIW195-154 | 3908 | 2517 | 633 | 2008 | 2416 | 954 | 64 |

Listed values represent individual carotenoids, extracted and analyzed as described. The carotenoid extract was prepared from fully opened Tagetes flowers. Values refer to dry weight.

**Table 8: Individual ketocarotenoids in petals of transgenic Tagetes SIW195**

| | ng carotenoid / mg | | | |
|---|---|---|---|---|
| Plant | Astaxanthin | Canthaxanthin | Phoenicoxanthin | Total ketos |
| M4-SIW195-5 | 2934 | 682 | 550 | 4166 |
| M4-SIW195-30 | 3230 | 638 | 601 | 4469 |
| M4-SIW195-36 | 2268 | 466 | 446 | 3179 |
| M4-SIW195-45 | 2301 | 713 | 546 | 3561 |
| M4-SIW195-46 | 3198 | 551 | 525 | 4274 |
| M4-SIW195-53 | 3119 | 814 | 712 | 4645 |
| M4-SIW195-57 | 2899 | 616 | 612 | 4127 |
| M4-SIW195-61 | 2727 | 712 | 605 | 4044 |
| M4-SIW195-62 | 613 | 305 | 283 | 1201 |
| M4-SIW195-77 | 3047 | 736 | 626 | 4409 |
| M4-SIW195-78 | 3038 | 831 | 702 | 4570 |
| M4-SIW195-79 | 2420 | 483 | 434 | 3337 |
| M4-SIW195-82 | 1656 | 490 | 364 | 2510 |
| M4-SIW195-89 | 2540 | 502 | 513 | 3555 |
| M4-SIW195-94 | 2741 | 566 | 442 | 3749 |
| M4-SIW195-99 | 2263 | 614 | 538 | 3415 |
| M4-SIW195-109 | 2523 | 905 | 731 | 4159 |
| M4-SIW195-118 | 2373 | 857 | 614 | 3844 |
| M4-SIW195-118 | 2227 | 629 | 497 | 3353 |
| M4-SIW195-126 | 2875 | 1077 | 793 | 4744 |
| M4-SIW195-127 | 2069 | 701 | 511 | 3280 |
| M4-SIW195-136 | 2079 | 570 | 444 | 3093 |
| M4-SIW195-154 | 2517 | 757 | 633 | 3908 |

**Annex 1**

| Molecule type | Sequence name in database | Original DB Accession number | Description in database |
|---|---|---|---|
| dna | 41_BKT_O23973 | GENESEQ_DNA\|AAV34437 | H. pluvialis beta-carotene C-4-oxygenase enzyme (crtO) encoding cDNA. |
| protein | 41_BKT_023973 | SPTREMBL\|O23973_HAEPL | Beta-carotene C-4 oxygenase (Ketolase). |
| protein | GENESEQ_PROT\|AAB62157 | GENESEQ_PROT\|AAB62157 | Transit peptide and beta-carotene C-4-oxygenase fusion protein. |
| dna | GENESEQ_PROT\|AAO16024 | GENESEQ_DNA\|ABT14221 | Brevundimonas aurantiaca Beta-carotene C4 oxygenase gene. |
| protein | GENESEQ_PROT\|AAO16024 | GENESEQ_PROT\|AAO16024 | Brevundimonas aurantiaca Beta-carotene C4 oxygenase. |
| protein | GENESEQ_PROT\|AAR79058 | GENESEQ_PROT\|AAR79058 | 3 hydroxy-beta-ionone ring methylene to keto group converting peptide. |
| dna | GENESEQ_PROT\|AAR92098 | unknown id | Beta-ionone 4-methylene gp. to keto gp. converting enzyme cDNA. |
| protein | GENESEQ_PROT\|AAR92098 | GENESEQ_PROT\|AAR92098 | Beta-ionone 4-methylene gp. to keto gp. converting enzyme cDNA. |
| dna | GENESEQ_PROT\|AAR92099 | unknown id | Beta-ionone 4-methylene gp. to keto gp. converting enzyme cDNA. |
| protein | GENESEQ_PROT\|AAR92099 | GENESEQ_PROT\|AAR92099 | Beta-ionone 4-methylene gp. to keto gp. converting enzyme cDNA. |
| dna | GENESEQ_PROT\|AAW98198 | GENESEQ_DNA\|AAX25068 | SSU/crtW (beta-carotene ketolase) gene fusion. |
| protein | GENESEQ_PROT\|AAW98198 | GENESEQ_PROT\|AAW98198 | SSU/(beta-carotene ketolase fusion. |
| dna | GENESEQ_PROT\|ABU97244 | GENESEQ_DNA\|ACA99471 | DNA encoding enzyme polypeptide #10. |
| protein | GENESEQ_PROT\|ABU97244 | GENESEQ_PROT\|ABU97244 | Enzyme polypeptide #10. |
| dna | GENESEQ_PROT\|ADP74106 | unknown id | Nostoc punctiforme ketolase DNA SEQ ID NO 3 variant #1. |
| protein | GENESEQ_PROT\|ADP74106 | GENESEQ_PROT\|ADP74106 | Nostoc punctiforme ketolase DNA SEQ ID NO 3 variant #1. |
| dna | GENESEQ_PROT\|ADP74108 | unknown id | Nostoc punctiforme ketolase DNA SEQ ID NO 3 variant #2. |
| protein | GENESEQ_PROT\|ADP74108 | GENESEQ_PROT\|ADP74108 | Nostoc punctiforme ketolase DNA SEQ ID NO 3 variant #2. |
| dna | GENESEQ_PROT\|ADP74110 | unknown id | Nostoc punctiforme ketolase DNA SEQ ID NO 5 variant #1. |
| protein | GENESEQ_PROT\|ADP74110 | GENESEQ_PROT\|ADP74110 | Nostoc punctiforme ketolase DNA SEQ ID NO 5 variant #1. |
| dna | GENESEQ_PROT\|ADP74112 | unknown id | Nostoc punctiforme ketolase DNA SEQ ID NO 5 variant #2. |
| protein | GENESEQ_PROT\|ADP74112 | GENESEQ_PROT\|ADP74112 | Nostoc punctiforme ketolase DNA SEQ ID NO 5 variant #2. |
| dna | GENESEQ_PROT\|ADP74147 | unknown id | Synechococcus sp. WH 8102 ketolase variant DNA#1. |
| protein | GENESEQ_PROT\|ADP74147 | GENESEQ_PROT\|ADP74147 | Synechococcus sp. WH 8102 ketolase variant DNA#1. |
| dna | GENESEQ_PROT\|ADP74149 | unknown id | Synechococcus sp. WH 8102 ketolase variant DNA #2. |
| protein | GENESEQ_PROT\|ADP74149 | GENESEQ_PROT\|ADP74149 | Synechococcus sp. WH 8102 ketolase variant DNA #2. |
| dna | GENESEQ_PROT\|ADQ38260 | unknown id | H. pluvialis ketolase DNA. |
| protein | GENESEQ_PROT\|ADQ38260 | GENESEQ_PROT\|ADQ38260 | H. pluvialis ketolase DNA. |
| dna | GENESEQ_PROT\|ADQ38262 | unknown id | H. pluvialis ketolase DNA. |
| protein | GENESEQ_PROT\|ADQ38262 | GENESEQ_PROT\|ADQ38262 | H. pluvialis ketolase DNA. |
| dna | GENESEQ_PROT\|ADQ38264 | unknown id | H. pluvialis ketolase DNA. |
| protein | GENESEQ_PROT\|ADQ38264 | GENESEQ_PROT\|ADQ38264 | H. pluvialis ketolase DNA. |
| dna | GENESEQ_PROT\|ADQ38323 | unknown id | Synechocystis sp. WH 8102 ketolase DNA. |
| protein | GENESEQ_PROT\|ADQ38323 | GENESEQ_PROT\|ADQ38323 | Synechocystis sp. WH 8102 ketolase DNA. |
| dna | GENESEQ_PROT\|ADQ96834 | unknown id | CrtWcrtY nucleotide sequence. |
| protein | GENESEQ_PROT\|ADQ96834 | GENESEQ_PROT\|ADQ96834 | CrtWcrtY nucleotide sequence. |
| dna | GENESEQ_PROT\|ADR03950 | unknown id | Nostoc punctiforme ketolase coding sequence #1. |
| protein | GENESEQ_PROT\|ADR03950 | GENESEQ_PROT\|ADR03950 | "no start codon" |
| dna | GENESEQ_PROT\|ADR03952 | unknown id | Nostoc punctiforme ketolase coding sequence #2. |
| protein | GENESEQ_PROT\|ADR03952 | GENESEQ_PROT\|ADR03952 | "no start codon" |
| dna | GENESEQ_PROT\|ADY51414 | unknown id | Nodularia spumigena NODK ketolase DNA. |
| protein | GENESEQ_PROT\|ADY51414 | GENESEQ_PROT\|ADY51414 | Nodularia spumigena NODK ketolase DNA. |
| dna | GENESEQ_PROT\|ADY52469 | GENESEQ_DNA\|ADY51401 | Nostoc punctiforme ketolase NP196 DNA. |
| protein | GENESEQ_PROT\|ADY52469 | GENESEQ_PROT\|ADY52469 | Novel ketocarotenoid preparation method-related protein SeqID103. |
| dna | GENESEQ_PROT\|ADY52906 | GENESEQ_DNA\|ADY52905 | N. spumigena strain NSOR10 ketocarotenoid-related putative ketolase DNA. |
| protein | GENESEQ_PROT\|ADY52906 | GENESEQ_PROT\|ADY52906 | N. spumigena NSOR10 ketocarotenoid-related putative ketolase protein. |
| dna | GENESEQ_PROT\|ADY52908 | GENESEQ_DNA\|ADY52907 | Nodularia spumigena ketocarotenoid-related ketolase DNA - SEQ ID 3. |
| protein | GENESEQ_PROT\|ADY52908 | GENESEQ_PROT\|ADY52908 | Nodularia spumigena ketocarotenoid-related ketolase protein - SEQ ID 4. |
| dna | GENESEQ_PROT\|ADY52910 | GENESEQ_DNA\|ADY52911 | Nodularia spumigena ketocarotenoid-related ketolase DNA - SEQ ID 7. |
| protein | GENESEQ_PROT\|ADY52910 | GENESEQ_PROT\|ADY52910 | Nodularia spumigena ketocarotenoid-related ketolase protein - SEQ ID 6. |
| dna | GENESEQ_PROT\|ADY52914 | GENESEQ_DNA\|ADY52913 | Nostoc punctiforme ketocarotenoid-related ketolase DNA - SEQ ID 9. |
| protein | GENESEQ_PROT\|ADY52914 | GENESEQ_PROT\|ADY52914 | Nostoc punctiforme ketocarotenoid-related ketolase protein - SEQ ID 10. |
| dna | GENESEQ_PROT\|ADY52916 | GENESEQ_DNA\|ADY52915 | Nostoc punctiforme ketocarotenoid-related ketolase DNA - SEQ ID 11. |
| protein | GENESEQ_PROT\|ADY52916 | GENESEQ_PROT\|ADY52916 | Nostoc punctiforme ketocarotenoid-related ketolase protein - SEQ ID 12. |
| dna | GENESEQ_PROT\|ADY52966 | GENESEQ_DNA\|ADY52965 | Nostoc punctiforme ketocarotenoid-related ketolase DNA - SEQ ID 61. |
| protein | GENESEQ_PROT\|ADY52966 | GENESEQ_PROT\|ADY52966 | Nostoc punctiforme ketocarotenoid-related ketolase protein - SEQ ID 62. |
| dna | GENESEQ_PROT\|ADY52971 | GENESEQ_DNA\|ADY52970 | Nostoc punctiforme ketocarotenoid-related ketolase DNA - SEQ ID 66. |
| protein | GENESEQ_PROT\|ADY52971 | GENESEQ_PROT\|ADY52971 | Nostoc punctiforme ketocarotenoid-related ketolase protein - SEQ ID 67. |
| dna | GENESEQ_PROT\|ADY52974 | GENESEQ_DNA\|ADY52973 | Nodularia spumigena ketocarotenoid-related ketolase DNA - SEQ ID 69. |
| protein | GENESEQ_PROT\|ADY52974 | GENESEQ_PROT\|ADY52974 | Nodularia spumigena ketocarotenoid-related ketolase protein - SEQ ID 70. |
| dna | GENESEQ_PROT\|ADY52976 | GENESEQ_DNA\|ADY52975 | Nodularia spumigena ketocarotenoid-related ketolase DNA - SEQ ID 71. |
| protein | GENESEQ_PROT\|ADY52976 | GENESEQ_PROT\|ADY52976 | Nodularia spumigena ketocarotenoid-related ketolase protein - SEQ ID 72. |
| dna | GENESEQ_PROT\|ADY52980 | GENESEQ_DNA\|ADY52917 | Gloeobacter violaceus ketocarotenoid-related ketolase DNA - SEQ ID 13. |
| protein | GENESEQ_PROT\|ADY52980 | GENESEQ_PROT\|ADY52980 | Gloeobacter violaceus ketocarotenoid-related ketolase protein - SEQ 76. |
| dna | GENESEQ_PROT\|AEB92197 | unknown id | S. melonis beta-carotene C4 oxygenase DNA. |
| protein | GENESEQ_PROT\|AEB92197 | GENESEQ_PROT\|AEB92197 | S. melonis beta-carotene C4 oxygenase DNA. |
| dna | GENESEQ_PROT\|AEB92199 | unknown id | B. vesicularis beta-carotene C4 oxygenase DNA. |
| protein | GENESEQ_PROT\|AEB92199 | GENESEQ_PROT\|AEB92199 | B. vesicularis beta-carotene C4 oxygenase DNA. |
| dna | GENESEQ_PROT\|AEK41647 | GENESEQ_DNA\|AEK41646 | Carotenoid biosynthetic pathway associated DNA, SEQ ID NO:17. |
| protein | GENESEQ_PROT\|AEK41647 | GENESEQ_PROT\|AEK41647 | Carotenoid biosynthetic pathway associated enzyme, SEQ ID NO:18. |
| dna | GENESEQ_PROT\|AEK41703 | GENESEQ_DNA\|AEK41702 | Carotenoid biosynthetic pathway associated DNA, SEQ ID NO:73. |
| protein | GENESEQ_PROT\|AEK41703 | GENESEQ_PROT\|AEK41703 | Carotenoid biosynthetic pathway associated enzyme, SEQ ID NO:74. |
| dna | GENESEQ_PROT\|AEK41725 | GENESEQ_DNA\|AEK41724 | Carotenoid biosynthetic pathway associated DNA, SEQ ID NO:95. |
| protein | GENESEQ_PROT\|AEK41725 | GENESEQ_PROT\|AEK41725 | Carotenoid biosynthetic pathway associated enzyme, SEQ ID NO:96. |
| dna | GENESEQ_PROT\|AEK41826 | GENESEQ_DNA\|AEK41686 | Carotenoid biosynthetic pathway associated DNA, SEQ ID NO:57. |
| protein | GENESEQ_PROT\|AEK41826 | GENESEQ_PROT\|AEK41826 | Astaxanthin biosynthetic pathway vector related protein, SEQ ID NO:197. |
| dna | GENESEQ_PROT\|AEL17450 | unknown id | Carotene ketolase gene. |
| protein | GENESEQ_PROT\|AEL17450 | GENESEQ_PROT\|AEL17450 | Carotene ketolase gene. |
| dna | REFSEQ_PROTEIN\|NP_108024 | REFSEQ_NUCLEOTIDE\|NC_002678 | Mesorhizobium loti MAFF303099, complete genome. |
| protein | REFSEQ_PROTEIN\|NP_08024 | REFSEQ_PROTEIN\|NP_108024 | similar to Rhizopine catabolism protein mocD [Mesorhizobium loti MAFF303099]. |
| dna | REFSEQ_PROTEIN\|NP_487229 | REFSEQ_NUCLEOTIDE\|NC_003272 | Nostoc sp. PCC 7120, complete genome. |
| protein | REFSEQ_PROTEIN\|NP_487229 | REFSEQ_PROTEIN\|NP_487229 | beta-carotene ketolase [Nostoc sp. PCC 7120]. |
| dna | REFSEQ_PROTEIN\|NP_897461 | REFSEQ_NUCLEOTIDE\|NC_005070 | Synechococcus sp. WH 8102, complete genome. |
| protein | REFSEQ_PROTEIN\|NP_897461 | REFSEQ_PROTEIN\|NP_897461 | possible beta-carotene ketolase [Synechococcus sp. WH 8102]. |
| dna | REFSEQ_PROTEIN\|NP_924674 | REFSEQ_NUCLEOTIDE\|NC_005125 | Gloeobacter violaceus PCC 7421, complete genome. |
| protein | REFSEQ_PROTEIN\|NP_924674 | REFSEQ_PROTEIN\|NP_924674 | beta-carotene ketolase [Gloeobacter violaceus PCC 7421]. |
| dna | REFSEQ_PROTEIN\|YP_322565 | REFSEQ_NUCLEOTIDE\|NC_007413 | Anabaena variabilis ATCC 29413, complete genome. |
| protein | REFSEQ_PROTEIN\|YP_322565 | REFSEQ_PROTEIN\|YP_322565 | Fatty acid desaturase [Anabaena variabilis ATCC 29413]. |
| dna | REFSEQ_PROTEIN\|YP_324388 | REFSEQ_NUCLEOTIDE\|NC_007413 | Anabaena variabilis ATCC 29413, complete genome. |
| protein | REFSEQ_PROTEIN\|YP_324388 | REFSEQ_PROTEIN\|YP_324388 | Fatty acid desaturase [Anabaena variabilis ATCC 29413]. |
| dna | REFSEQ_PROTEIN\|YP_376982 | REFSEQ_NUCLEOTIDE\|NC_007513 | Synechococcus sp. CC9902, complete genome. |
| protein | REFSEQ_PROTEIN\|YP_376982 | REFSEQ_PROTEIN\|YP_376982 | possible beta-carotene ketolase [Synechococcus sp. CC9902]. |
| dna | REFSEQ_PROTEIN\|YP_457553 | REFSEQ_NUCLEOTIDE\|NC_007722 | Erythrobacter litoralis HTCC2594, complete genome. |
| protein | REFSEQ_PROTEIN\|YP_457553 | REFSEQ_PROTEIN\|YP_457553 | beta-carotene ketolase [Erythrobacter litoralis HTCC2594]. |
| dna | REFSEQ_PROTEIN\|YP_475340 | REFSEQ_NUCLEOTIDE\|NC_007775 | Synechococcus sp. JA-3-3Ab, complete genome. |
| protein | REFSEQ_PROTEIN\|YP_475340 | REFSEQ_PROTEIN\|YP_475340 | fatty acid desaturase [Synechococcus sp. JA-3- 3Ab]. |
| dna | REFSEQ_PROTEIN\|YP_476366 | REFSEQ_NUCLEOTIDE\|NC_007776 | Synechococcus sp. JA-2-3B'a(2-13), complete genome. |
| protein | REFSEQ_PROTEIN\|YP_476366 | REFSEQ_PROTEIN\|YP_476366 | beta-carotene ketolase , putative [Synechococcus sp. JA-2-3B'a(2-13)]. |
| dna | REFSEQ_PROTEIN\|YP_634097 | REFSEQ_NUCLEOTIDE\|NC_008095 | Myxococcus xanthus DK 1622, complete genome. |
| protein | REFSEQ_PROTEIN\|YP_634097 | REFSEQ_PROTEIN\|YP_634097 | fatty acid desaturase family protein [Myxococcus xanthus DK 1622]. |
| dna | REFSEQ_PROTEIN\|YP_634184 | REFSEQ_NUCLEOTIDE\|NC_008095 | Myxococcus xanthus DK 1622, complete genome. |
| protein | REFSEQ_PROTEIN\|YP_634184 | REFSEQ_PROTEIN\|YP_634184 | fatty acid desaturase family protein [Myxococcus xanthus DK 1622]. |
| dna | REFSEQ_PROTEIN\|YP_731008 | REFSEQ_NUCLEOTIDE\|NC_008319 | Synechococcus sp. CC9311, complete genome. |
| protein | REFSEQ_PROTEIN\|YP_731008 | REFSEQ_PROTEIN\|YP_731008 | possible beta-carotene ketolase [Synechococcus sp. CC9311]. |
| dna | REFSEQ_PROTEIN\|ZP_00111258 | REFSEQ_NUCLEOTIDE\|NZ_AMY02000007 | Nostoc punctiforme PCC 73102, unfinished sequence, whole genome shotgun sequence. |
| protein | REFSEQ_PROTEIN\|ZP_00111258 | REFSEQ_PROTEIN\|ZP_00111258 | COG3239: Fatty acid desaturase [Nostoc punctiforme PCC 73102]. |
| dna | REFSEQ_PROTEIN\|ZP_00345866 | GB_patent\|DD399711 | Method for producing carotenoids or their precursors using genetically modified organisms of the Blakeslea genus, carotenoids or their precursors produced by said method and use thereof. |
| protein | REFSEQ_PROTEIN\|ZP_00345866 | REFSEQ_PROTEIN\|ZP_00345866 | hypothetical protein Npun02000865 [Nostoc punctiforme PCC 73102]. |
| dna | REFSEO_PROTEIN\|ZP_00514501 | REFSEO_NUCLEOTIDE\|NZ_AADV02000002 | Crocosphaera watsonii WH 8501, unfinished sequence, whole genome shotgun sequence. |
| protein | REFSEQ_PROTEIN\|ZP_00514501 | REFSEQ_PROTEIN\|ZP_00514501 | Fatty acid desaturase [Crocosphaera watsonii WH 8501]. |
| dna | REFSEQ_PROTEIN\|ZP_01018056 | REFSEQ_NUCLEOTIDE\|NZ_AAMU01000003 | Parvularcula bermudensis HTCC2503, unfinished sequence, whole genome shotgun sequence. |
| protein | REFSEQ_PROTEIN\|ZP_01018056 | REFSEQ_PROTEIN\|ZP_01018056 | Fatty acid desaturase [Parvularcula bermudensis HTCC2503]. |
| dna | REFSEQ_PROTEIN\|ZP_01041617 | REFSEQ_NUCLEOTIDE\|NZ_AAMW01000003 | Erythrobacter sp. NAP1, unfinished sequence, whole genome shotgun sequence. |
| protein | REFSEQ_PROTEIN\|ZP_01041617 | REFSEQ_PROTEIN\|ZP_01041617 | Fatty acid desaturase [Erythrobacter sp. NAP1]. |
| dna | REFSEQ_PROTEIN\|ZP_01080541 | REFSEO_NUCLEOTIDE\|NZ_AANP01000004 | Synechococcus sp. RS9917, unfinished sequence, whole genome shotgun sequence. |
| protein | REFSEQ_PROTEIN\|ZP_01080541 | REFSEQ_PROTEIN\|ZP_01080541 | possible beta-carotene ketolase [Synechococcus sp. RS9917]. |
| dna | REFSEQ_PROTEIN\|ZP_01083421 | REFSEQ_NUCLEOTIDE\|NZ_AAN001000001 | Synechococcus sp. WH 5701, unfinished sequence, whole genome shotgun sequence. |
| protein | REFSEQ_PROTEIN\|ZP_01083421 | REFSEQ_PROTEIN\|ZP_01083421 | possible beta-carotene ketolase [Synechococcus sp. WH 5701]. |
| dna | REFSEQ_PROTEIN\|ZP_01123773 | REFSEQ_NUCLEOTIDE\|NZ_AAOK01000003 | Synechococcus sp. WH 7805, unfinished sequence, whole genome shotgun sequence. |
| protein | REFSEQ_PROTEIN\|ZP_01123773 | REFSEQ_PROTEIN\|ZP_01123773 | possible beta-carotene ketolase [Synechococcus sp. WH 7805]. |
| dna | REFSEQ_PROTEIN\|ZP_01226542 | REFSEQ_NUCLEOTIDE\|NZ_AAPJ01000002 | Aurantimonas sp. SI85-9A1, unfinished sequence, whole genome shotgun sequence. |
| protein | REFSEQ_PROTEIN\|ZP_01226542 | REFSEQ_PROTEIN\|ZP_01226542 | beta-carotene ketolase/oxygenase [Aurantimonas sp. SI85-9A1]. |
| dna | REFSEQ_PROTEIN\|ZP_01439496 | REFSEQ_NUCLEOTIDE\|NZ_AATP01000004 | Fulvimarina pelagi HTCC2506, unfinished sequence, whole genome shotgun sequence. |
| protein | REFSEQ_PROTEIN\|ZP_01439496 | REFSEQ_PROTEIN\|ZP_01439496 | beta-carotene ketolase [Fulvimarina pelagi HTCC2506]. |
| dna | REFSEQ_PROTEIN\|ZP_01468055 | REFSEQ_NUCLEOTIDE\|NZ_AATZ01000001 | Synechococcus sp. BL107, unfinished sequence, whole genome shotgun sequence. |
| protein | REFSEQ_PROTEIN\|ZP_01468055 | REFSEQ_PROTEIN\|ZP_01468055 | possible beta-carotene ketolase [Synechococcus sp. BL107]. |
| dna | REFSEQ_PROTEIN\|ZP_01632305 | REFSEQ_NUCLEOTIDE\|NZ_AAVW01000111 | Nodularia spumigena CCY9414, unfinished sequence, whole genome shotgun sequence. |
| protein | REFSEQ_PROTEIN\|ZP_01632305 | REFSEQ_PROTEIN\|ZP_01632305 | Fatty acid desaturase [Nodularia spumigena CCY9414]. |
| dna | REFSEQ_PROTEIN\|ZP_01632726 | REFSEQ_NUCLEOTIDE\|NZ_AAVW01000162 | Nodularia spumigena CCY9414, unfinished sequence, whole genome shotgun sequence. |
| protein | REFSEQ_PROTEIN\|ZP_01632726 | REFSEQ_PROTEIN\|ZP_01632726 | Fatty acid desaturase [Nodularia spumigena CCY9414]. |
| dna | SPTREMBL\|O23973_HAEPL | GB_plant\|X86782 | H.pluvialis mRNA for beta-carotene C-4 oxygenase. |
| protein | SPTREMBL\|O23973_HAEPL | SPTREMBL\|O23973_HAEPL | Beta-carotene C-4 oxygenase (Ketolase). |
| dna | SPTREMBL\|Q0H2C9_BREVE | GB_bacterial\|DQ309446 | Brevundimonas vesicularis carotenoid synthesis gene cluster, complete sequence. |
| protein | SPTREMBL\|Q0H2C9_BREVE | SPTREMBL\|Q0H2C9_BREVE | Beta-carotene ketolase. |
| protein | SPTREMBL\|Q15190_9SPHI | SPTREMBL\|Q15190_9SPHI | CrtW. |
| dna | SPTREMBL\|Q24K64_9RHOB | GB_bacterial\|AY957386 | Paracoccus haeundaensis astaxanthin biosynthesis gene cluster, complete sequence. |
| protein | SPTREMBL\|Q24K64_9RHOB | SPTREMBL\|Q24K64_9RHOB | Beta-carotene ketolase. |
| dna | SPTREMBL\|Q2XST9_HAEPL | GB_plant\|DQ257290 | Haematococcus pluvialis 34-1n betacarotene ketolase (crtO) mRNA, partial cds. |
| protein | SPTREMBL\|Q2XST9_HAEPL | SPTREMBL\|Q2XST9_HAEPL | Beta-carotene ketolase (Fragment). |
| protein | SPTREMBL\|Q4FE71_HAEPL | SPTREMBL\|Q4FE71_HAEPL | Beta-carotene C-4 oxygenase. |
| dna | SPTREMBL\|Q4VKB4_CHLRE | GB_plant\|AY860820 | Chlamydomonas reinhardtii putative chloroplast carotene beta ketolase precursor (BKT) mRNA, complete cds; nuclear gene for chloroplast product. |
| protein | SPTREMBL\|Q4VKB4_CHLRE | SPTREMBL\|Q4VKB4_CHLRE | Putative chloroplast carotene beta ketolase. |
| protein | SPTREMBL\|Q4W8B8_9CAUL | SPTREMBL\|Q4W8B8_9CAUL | Beta-carotene ketolase. |
| protein | SPTREMBL\|Q5U931_9CHLO | SPTREMBL\|Q5U931_9CHLO | Beta-carotene ketolase/oxygenase. |
| dna | SPTREMBL\|Q6J3N5_HAEPL | GB_plant\|AY603347 | Haematococcus pluvialis strain NIES-144 beta-carotene ketolase (bkt3) mRNA, complete cds. |
| protein | SPTREMBL\|Q6J3N5_HAEPL | SPTREMBL\|Q6J3N5_HAEPL | Beta-carotene ketolase. |
| dna | SPTREMBL\|Q847D1_NODSP | GB_patent\|CS050451 | Sequence 37 from Patent WO2005019460. |
| protein | SPTREMBL\|Q847D1_NODSP | SPTREMBL\|Q847D1_NODSP | Putative beta-carotene ketolase. |
| dna | SPTREMBL\|Q8GCT5_9CAUL | GB_bacterial\|AY166610 | Brevundimonas aurantiaca beta-carotene C4 oxygenase (crtW) gene, complete cds. |
| protein | SPTREMBL\|Q8GCT5_9CAUL | SPTREMBL\|Q8GCT5_9CAUL | Beta-carotene C4 oxygenase. |
| dna | SPTREMBL\|Q8LJQ2_HAEPL | GB_patent\|CS050445 | Sequence 31 from Patent WO2005019460. |
| protein | SPTREMBL\|Q8LJQ2_HAEPL | SPTREMBL\|Q8LJQ2_HAEPL | BKT. |
| dna | SPTREMBL\|Q9KIX0_9BRAD | GB_patent\|DD399668 | Method for producing carotenoids or their precursors using genetically modified organisms of the Blakeslea genus, carotenoids or their precursors produced by said method and use thereof. |
| protein | SPTREMBL\|Q9KIX0_9BRAD | SPTREMBL\|Q9KIX0_9BRAD | Beta-carotene ketolase. |
| dna | SPTREMBL\|Q9RLH7_9RHOB | GB_patent\|DD399666 | Method for producing carotenoids or their precursors using genetically modified organisms of the Blakeslea genus, carotenoids or their precursors produced by said method and use thereof. |
| protein | SPTREMBL\|Q9RLH7_9RHOB | SPTREMBL\|Q9RLH7_9RHOB | Beta-carotene C-4-oxygenase (Ketolase). |
| dna | SWISSPROT\|CRTW_HAEPL | GB_patent\|DD367504 | Peptides capable of transiting to chromoplast and a method for producing a plant having yellowish petals using the peptides. |
| protein | SWISSPROT\|CRTW_HAEPL | SWISSPROT\|CRTW_HAEPL | Beta-carotene ketolase (EC 1.13.-.-) (Beta-carotene oxygenase). |
| protein | SWISSPROT\|CRTW_PARS1 | SWISSPROT\|CRTW_PARS1 | Beta-carotene ketolase (EC 1.13.-.-) (Beta-carotene oxygenase). |
| protein | SWISSPROT\|CRTW_PARSN | SWISSPROT\|CRTW_PARSN | Beta-carotene ketolase (EC 1.13.-.-) Beta-carotene oxygenase). |
| | | | |
| protein | 42_MBKT_NP442491 | | b-carotene ketolase [Synechocystis sp. PCC 6803]. |
| protein | FastAlert_P\|JP2005185101.35237 | FastAlert_P\|JP2005185101.35237 | |
| protein | FastAlert_P\|WO2005083127.1018 | FastAlert_P\|WO2005083127.1018 | |
| dna | GENESEQ_PROT\|ABP71892 | unknown id | R. erythropolis carotenoid ketolase crtO gene. |
| protein | GENESEQ_PROT\|ABP71892 | GENESEQ_PROT\|ABP71892 | R. erythropolis carotenoid ketolase crtO gene. |
| protein | GENESEQ_PROT\|ADN22148 | GENESEQ_PROT\|ADN22148 | Bacterial polypeptide #4801. |
| protein | GENESEQ_PROT\|ADN22285 | GENESEQ_PROT\|ADN22285 | Bacterial polypeptide #4938. |
| protein | GENESEQ_PROT\|ADS21776 | GENESEQ_PROT\|ADS21776 | Bacterial polypeptide #10809. |
| protein | GENESEQ_PROT\|ADS29845 | GENESEQ_PROT\|ADS29845 | Bacterial polypeptide #18878. |
| protein | GENESEQ_PROT\|ADS30953 | GENESEQ_PROT\|ADS30953 | Bacterial polypeptide #19986. |
| protein | GENESEQ_PROT\|ADS41983 | GENESEQ_PROT\|ADS41983 | Bacterial polypeptide #20413. |
| dna | GENESEQ_PROT\|AEE85921 | unknown id | Codon optimized carotenoid ketolase crtO303 coding sequence. |
| protein | GENESEQ_PROT\|AEE85921 | GENESEQ_PROT\|AEE85921 | Codon optimized carotenoid ketolase crtO303 coding sequence. |
| protein | GENESEQ_PROT\|AEE85930 | GENESEQ_PROT\|AEE85930 | Carotenoid ketolase mutant 319M3022. |
| protein | GENESEQ_PROT\|AEE85930 | GENESEQ_PROT\|AEE85930 | Carotenoid ketolase mutant 319M3022. |
| dna | GENESEQ_PROT\|AEE85932 | unknown id | Carotenoid ketolase mutant 302M3044 coding sequence. |
| protein | GENESEQ_PROT\|AEE85932 | GENESEQ_PROT\|AEE85932 | Carotenoid ketolase mutant 302M3044 coding sequence. |
| protein | GENESEQ_PROT\|AEE85934 | GENESEQ_PROT\|AEE85934 | Carotenoid ketolase mutant 320M4006. |
| protein | GENESEQ_PROT\|AEE85936 | GENESEQ_PROT\|AEE85936 | Carotenoid ketolase mutant 320M4007. |
| protein | GENESEQ_PROT\|AEE85938 | GENESEQ_PROT\|AEE85938 | Carotenoid ketolase mutant 320M4009. |
| protein | GENESEQ_PROT\|AEE85938 | GENESEQ_PROT\|AEE85938 | Carotenoid ketolase mutant 320M4009. |
| protein | GENESEQ_PROT\|AEE85940 | GENESEQ_PROT\|AEE85940 | Carotenoid ketolase mutant 320M4018. |
| protein | GENESEQ_PROT\|AEE85942 | GENESEQ_PROT\|AEE85942 | Carotenoid ketolase mutant 320M4019. |
| protein | GENESEQ_PROT\|AEE85946 | GENESEQ_PROT\|AEE85946 | Carotenoid ketolase mutant 320M4023. |
| protein | GENESEQ_PROT\|AEE85950 | GENESEQ_PROT\|AEE85950 | Carotenoid ketolase mutant 320M4032. |
| protein | GENESEQ_PROT\|AEE85950 | GENESEQ_PROT\|AEE85950 | Carotenoid ketolase mutant 320M4032. |
| protein | GENESEQ_PROT\|AEE85952 | GENESEQ_PROT\|AEE85952 | Carotenoid ketolase mutant 320M4036. |
| protein | GENESEQ_PROT\|AEE85956 | GENESEQ_PROT\|AEE85956 | Carotenoid ketolase mutant 320SHU004. |
| protein | GENESEQ_PROT\|AEE85958 | GENESEQ_PROT\|AEE85958 | Carotenoid ketolase mutant 320SHU008. |
| protein | GENESEQ_PROT\|AEE85960 | GENESEQ_PROT\|AEE85960 | Carotenoid ketolase mutant 320SHU015. |
| protein | GENESEQ_PROT\|AEE85962 | GENESEQ_PROT\|AEE85962 | Carotenoid ketolase mutant 320SHU016. |
| protein | GENESEQ_PROT\|AEE85964 | GENESEQ_PROT\|AEE85964 | Carotenoid ketolase mutant 320SHU017. |
| protein | GENESEQ_PROT\|AEE85966 | GENESEQ_PROT\|AEE85966 | Carotenoid ketolase mutant 320SHU019. |
| protein | GENESEQ_PROT\|AEE85968 | GENESEQ_PROT\|AEE85968 | Carotenoid ketolase mutant 320SHU022. |
| protein | GENESEQ_PROT\|AEE85969 | GENESEQ_PROT\|AEE85969 | Carotenoid ketolase mutant M142L/A164V. |
| protein | GENESEQ_PROT\|AEE85969 | GENESEQ_PROT\|AEE85969 | Carotenoid ketolase mutant M142L/A164V. |
| dna | GENESEQ_PROT\|AEJ02072 | unknown id | Mutant carotenoid ketolase crtO DNA SEQ ID NO 16. |
| protein | GENESEQ_PROT\|AEJ02072 | GENESEQ_PROT\|AEJ02072 | Mutant carotenoid ketolase crtO DNA SEQ ID NO 16. |
| dna | GENESEQ_PROT\|AEJ02077 | unknown id | Mutant carotenoid ketolase crtO DNA SEQ ID NO 21. |
| protein | GENESEQ_PROT\|AEJ02077 | GENESEQ_PROT\|AEJ02077 | Mutant carotenoid ketolase crtO DNA SEQ ID NO 21. |
| dna | REFSEQ_PROTEIN\|NP_001067343 | REFSEQ_NUCLEOTIDE\|NM_001073875 | Oryza sativa (japonica cultivar-group) Os12g0631800 (Os12g0631800) mRNA, complete cds. |
| protein | REFSEQ_PROTEIN\|NP_001067343 | REFSEQ_PROTEIN\|NP_001067343 | Os12g0631800 [Oryza sativa (japonica cultivar- group)]. |
| dna | REFSEQ_PROTEIN\|NP_104734 | REFSEQ_NUCLEOTIDE\|NC_002678 | Mesorhizobium loti MAFF303099, complete genome. |
| protein | REFSEQ_PROTEIN\|NP_104734 | REFSEQ_PROTEIN\|NP_104734 | phytoene dehydrogenase [Mesorhizobium loti MAFF303099]. |
| dna | REFSEQ_PROTEIN\|NP_293819 | REFSEQ_NUCLEOTIDE\|NC_001263 | Deinococcus radiodurans R1 chromosome 1, complete sequence. |
| protein | REFSEQ_PROTEIN\|NP_293819 | REFSEQ_PROTEIN\|NP_293819 | phytoene dehydrogenase, putative [Deinococcus radiodurans R1]. |
| dna | REFSEQ_PROTEIN\|NP_343776 | REFSEQ_NUCLEOTIDE\|NC_002754 | Sulfolobus solfataricus P2, complete genome. |
| protein | REFSEQ_PROTEIN\|NP_343776 | REFSEQ_PROTEIN\|NP_343776 | Phytoene dehydrogenase related protein [Sulfolobus solfataricus P2]. |
| dna | REFSEQ_PROTEIN\|NP_376437 | REFSEQ_NUCLEOTIDE\|NC_003106 | Sulfolobus tokodaii str. 7, complete genome. |
| protein | REFSEQ_PROTEIN\|NP_376437 | REFSEQ_PROTEIN\|NP_376437 | hypothetical protein ST0549 [Sulfolobus tokodaii str. 7]. |
| dna | REFSEQ_PROTEIN\|NP_421915 | REFSEQ_NUCLEOTIDE\|NC_002696 | Caulobacter crescentus CB15, complete genome. |
| protein | REFSEQ_PROTEIN\|NP_421915 | REFSEQ_PROTEIN\|NP_421915 | phytoene dehydrogenase-related protein [Caulobacter crescentus CB15]. |
| dna | REFSEQ_PROTEIN\|NP_442491 | REFSEQ_NUCLEOTIDE\|NC_000911 | Synechocystis sp. PCC 6803, complete genome. |
| protein | REFSEQ_PROTEIN\|NP_442491 | REFSEQ_PROTEIN\|NP_442491 | b-carotene ketolase [Synechocystis sp. PCC 6803]. |
| dna | REFSEQ_PROTEIN\|NP_487784 | REFSEQ_NUCLEOTIDE\|NC_003272 | Nostoc sp. PCC 7120, complete genome. |
| protein | REFSEQ_PROTEIN\|NP_487784 | REFSEQ_PROTEIN\|NP_487784 | hypothetical protein all3744 [Nostoc sp. PCC 7120]. |
| dna | REFSEQ_PROTEIN\|NP_568712 | REFSEQ_NUCLEOTIDE\|NM_124333 | Arabidopsis thaliana FAD binding / oxidoreductase, acting on paired donors, with incorporation or reduction of molecular oxygen (AT5G49555) mRNA, complete cds. |
| protein | REFSEQ_PROTEIN\|NP_568712 | REFSEQ_PROTEIN\|NP_568712 | FAD binding / oxidoreductase, acting on paired donors, with incorporation or reduction of molecular oxygen [Arabidopsis thaliana]. |
| dna | REFSEQ_PROTEIN\|NP_631440 | REFSEQ_NUCLEOTIDE\|NC_003888 | Streptomyces coelicolor A3(2), complete genome. |
| protein | REFSEQ_PROTEIN\|NP_631440 | REFSEQ_PROTEIN\|NP_631440 | dehydrogenase [Streptomyces coelicolor A3(2)]. |
| dna | REFSEQ_PROTEIN\|NP_643053 | REFSEQ_NUCLEOTIDE\|NC_003919 | Xanthomonas axonopodis pv. citri str. 306, complete genome. |
| protein | REFSEQ_PROTEIN\|NP_643053 | REFSEQ_PROTEIN\|NP_643053 | phytoene dehydrogenase [Xanthomonas axonopodis pv. citri str. 306]. |
| dna | REFSEQ_PROTEIN\|NP_773226 | REFSEQ_NUCLEOTIDE\|NC_004463 | Bradyrhizobium japonicum USDA 110, complete genome. |
| protein | REFSEQ_PROTEIN\|NP_773226 | REFSEQ_PROTEIN\|NP_773226 | probable phytoene dehydrogenase [Bradyrhizobium japonicum USDA 110]. |
| dna | REFSEQ_PROTEIN\|NP_822176 | REFSEQ_NUCLEOTIDE\|NC_003155 | Streptomyces avermitilis MA-4680, complete genome. |
| protein | REFSEQ_PROTEIN\|NP_822176 | REFSEQ_PROTEIN\|NP_822176 | dehydrogenase [Streptomyces avermitilis MA-4680]. |
| dna | REFSEQ_PROTEIN\|NP_923340 | REFSEQ_NUCLEOTIDE\|NC_005125 | Gloeobacter violaceus PCC 7421, complete genome. |
| protein | REFSEQ_PROTEIN\|NP_923340 | REFSEQ_PROTEIN\|NP_923340 | beta-carotene ketolase [Gloeobacter violaceus PCC 7421]. |
| dna | REFSEQ_PROTEIN\|NP_948851 | REFSEQ_NUCLEOTIDE\|NC_005296 | Rhodopseudomonas palustris CGA009, complete genome. |
| protein | REFSEQ_PROTEIN\|NP_948851 | REFSEQ_PROTEIN\|NP_948851 | phytoene dehydrogenase-related protein [Rhodopseudomonas palustris CGA009]. |
| dna | REFSEQ_PROTEIN\|YP_001045046 | REFSEQ_NUCLEOTIDE\|NC_009050 | Rhodobacter sphaeroides ATCC 17029 chromosome 2, complete sequence. |
| protein | REFSEQ_PROTEIN\|YP_001045046 | REFSEQ_PROTEIN\|YP_001045046 | FAD dependent oxidoreductase [Rhodobacter sphaeroides ATCC 17029]. |
| dna | REFSEQ_PROTEIN\|YP_117963 | REFSEQ_NUCLEOTIDE\|NC_006361 | Nocardia farcinica IFM 10152, complete genome. |
| protein | REFSEQ_PROTEIN\|YP_117963 | REFSEQ_PROTEIN\|YP_117963 | putative dehydrogenase [Nocardia farcinica IFM 10152]. |
| dna | REFSEQ_PROTEIN\|YP_318971 | REFSEQ_NUCLEOTIDE\|NC_007406 | Nitrobacterwinogradskyi Nb-255, complete genome. |
| protein | REFSEQ_PROTEIN\|YP_318971 | REFSEQ_PROTEIN\|YP_318971 | FAD dependent oxidoreductase [Nitrobacter winogradskyi Nb-255]. |
| dna | REFSEQ_PROTEIN\|YP_322099 | REFSEQ_NUCLEOTIDE\|NC_007413 | Anabaena variabilis ATCC 29413, complete genome. |
| protein | REFSEQ_PROTEIN\|YP_322099 | REFSEQ_PROTEIN\|YP_322099 | FAD dependent oxidoreductase [Anabaena variabilis ATCC 29413]. |
| dna | REFSEQ_PROTEIN\|YP_355033 | REFSEQ_NUCLEOTIDE\|NC_007494 | Rhodobacter sphaeroides 2.4.1 chromosome 2, complete sequence. |
| protein | REFSEQ_PROTEIN\|YP_355033 | REFSEQ_PROTEIN\|YP_355033 | Oxidoreductase [Rhodobacter sphaeroides 2.4.1]. |
| dna | REFSEQ_PROTEIN\|YP_364628 | REFSEQ_NUCLEOTIDE\|NC_007508 | Xanthomonas campestris pv. vesicatoria str. 85-10, complete genome. |
| protein | REFSEQ_PROTEIN\|YP_364628 | REFSEQ_PROTEIN\|YP_364628 | Phytoene dehydrogenase [Xanthomonas campestris pv. vesicatoria str. 85-10]. |
| dna | REFSEQ_PROTEIN\|YP_445625 | REFSEQ_NUCLEOTIDE\|NC_007677 | Salinibacter ruber DSM 13855, complete genome. |
| protein | REFSEQ_PROTEIN\|YP_445625 | REFSEQ_PROTEIN\|YP_445625 | beta-carotene ketolase [Salinibacter ruber DSM 13855]. |
| dna | REFSEQ_PROTEIN\|YP_466506 | REFSEQ_NUCLEOTIDE\|NC_007760 | Anaeromyxobacter dehalogenans 2CPC, complete genome. |
| protein | REFSEQ_PROTEIN\|YP_466506 | REFSEQ_PROTEIN\|YP_466506 | FAD dependent oxidoreductase [Anaeromyxobacter dehalogenans 2CP-C]. |
| dna | REFSEQ_PROTEIN\|YP_476281 | REFSEQ_NUCLEOTIDE\|NC_007776 | Synechococcus sp. JA-2-3B'a(2-13), complete genome. |
| protein | REFSEQ_PROTEIN\|YP_476281 | REFSEQ_PROTEIN\|YP_476281 | phytoene desaturase family protein [Synechococcus sp. JA-2-3B'a(2-13)]. |
| dna | REFSEQ_PROTEIN\|YP_485633 | REFSEQ_NUCLEOTIDE\|NC_007778 | Rhodopseudomonas palustris HaA2, complete genome. |
| protein | REFSEQ_PROTEIN\|YP_485633 | REFSEQ_PROTEIN\|YP_485633 | FAD dependent oxidoreductase [Rhodopseudomonas palustris HaA2]. |
| dna | REFSEQ_PROTEIN\|YP_496947 | REFSEQ_NUCLEOTIDE\|NC_007794 | Novosphingobium aromaticivorans DSM 12444, complete genome. |
| protein | REFSEQ_PROTEIN\|YP_496947 | REFSEQ_PROTEIN\|YP_496947 | FAD dependent oxidoreductase [Novosphingobium aromaticivorans DSM 12444]. |
| dna | REFSEQ_PROTEIN\|YP_533149 | REFSEQ_NUCLEOTIDE\|NC_007925 | Rhodopseudomonas palustris BisB18, complete genome. |
| protein | REFSEQ_PROTEIN\|YP_533149 | REFSEQ_PROTEIN\|YP_533149 | FAD dependent oxidoreductase [Rhodopseudomonas palustris BisB18]. |
| dna | REFSEQ_PROTEIN\|YP_552801 | REFSEQ_NUCLEOTIDE\|NC_007952 | Burkholderia xenovorans LB400 chromosome 2, complete sequence. |
| protein | REFSEQ_PROTEIN\|YP_552801 | REFSEQ_PROTEIN\|YP_552801 | Putative phytoene dehydrogenase [Burkholderia xenovorans LB400]. |
| dna | REFSEQ_PROTEIN\|YP_555885 | REFSEQ_NUCLEOTIDE\|NC_007953 | Burkholderia xenovorans LB400 chromosome 3, complete sequence. |
| protein | REFSEQ_PROTEIN\|YP_555885 | REFSEQ_PROTEIN\|YP_555885 | Putative dehydrogenase [Burkholderia xenovorans LB400]. |
| dna | REFSEQ_PROTEIN\|YP_570500 | REFSEQ_NUCLEOTIDE\|NC_007958 | Rhodopseudomonas palustris BisB5, complete genome. |
| protein | REFSEQ_PROTEIN\|YP_570500 | REFSEQ_PROTEIN\|YP_570500 | FAD dependent oxidoreductase [Rhodopseudomonas palustris BisB5]. |
| dna | REFSEQ_PROTEIN\|YP_577983 | REFSEQ_NUCLEOTIDE\|NC_007964 | Nitrobacter hamburgensis X14, complete genome. |
| protein | REFSEQ_PROTEIN\|YP_577983 | REFSEQ_PROTEIN\|YP_577983 | FAD dependent oxidoreductase [Nitrobacter hamburgensis X14]. |
| dna | REFSEQ_PROTEIN\|YP_590346 | REFSEQ_NUCLEOTIDE\|NC_008009 | Acidobacteria bacterium Ellin345, complete genome. |
| protein | REFSEQ_PROTEIN\|YP_590346 | REFSEQ_PROTEIN\|YP_590346 | FAD dependent oxidoreductase [Acidobacteria bacterium Ellin345]. |
| dna | REFSEQ_PROTEIN\|YP_590347 | REFSEQ_NUCLEOTIDE\|NC_008009 | Acidobacteria bacterium Ellin345, complete genome. |
| protein | REFSEQ_PROTEIN\|YP_590347 | REFSEQ_PROTEIN\|YP_590347 | amine oxidase [Acidobacteria bacterium Ellin345]. |
| dna | REFSEQ_PROTEIN\|YP_605771 | REFSEQ_NUCLEOTIDE\|NC_008025 | Deinococcus geothermalis DSM 11300, complete genome. |
| protein | REFSEQ_PROTEIN\|YP_605771 | REFSEQ_PROTEIN\|YP_605771 | FAD dependent oxidoreductase [Deinococcus geothermalis DSM 11300]. |
| dna | REFSEQ_PROTEIN\|YP_618202 | REFSEQ_NUCLEOTIDE\|NC_008048 | Sphingopyxis alaskensis RB2256, complete genome. |
| protein | REFSEQ_PROTEIN\|YP_618202 | REFSEQ_PROTEIN\|YP_618202 | FAD dependent oxidoreductase [Sphingopyxis alaskensis RB2256]. |
| dna | REFSEQ_PROTEIN\|YP_701086 | REFSEQ_NUCLEOTIDE\|NC_008268 | Rhodococcus sp. RHA1, complete genome. |
| protein | REFSEQ_PROTEIN\|YP_701086 | REFSEQ_PROTEIN\|YP_701086 | probable beta-carotene ketolase [Rhodococcus sp. RHA1]. |
| dna | REFSEQ_PROTEIN\|YP_713282 | REFSEQ_NUCLEOTIDE\|NC_008278 | Frankia alni ACN14a, complete genome. |
| protein | REFSEQ_PROTEIN\|YP_713282 | REFSEQ_PROTEIN\|YP_713282 | hypothetical protein FRAAL3071 [Frankia alni ACN14a]. |
| dna | REFSEQ_PROTEIN\|YP_714449 | REFSEQ_NUCLEOTIDE\|NC_008278 | Frankia alni ACN14a, complete genome. |
| protein | REFSEQ_PROTEIN\|YP_714449 | REFSEQ_PROTEIN\|YP_714449 | Putative phytoene dehydrogenase [Frankia alni ACN14a]. |
| dna | REFSEQ_PROTEIN\|YP_755539 | REFSEQ_NUCLEOTIDE\|NC_008347 | Maricaulis maris MCS10, complete genome. |
| protein | REFSEQ_PROTEIN\|YP_755539 | REFSEQ_PROTEIN\|YP_755539 | FAD dependent oxidoreductase [Maricaulis maris MCS10]. |
| dna | REFSEQ_PROTEIN\|YP_781051 | REFSEQ_NUCLEOTIDE\|NC_008435 | Rhodopseudomonas palustris BisA53, complete genome. |
| protein | REFSEQ_PROTEIN\|YP_781051 | REFSEQ_PROTEIN\|YP_781051 | FAD dependent oxidoreductase [Rhodopseudomonas palustris BisA53]. |
| dna | REFSEQ_PROTEIN\|YP_828532 | REFSEQ_NUCLEOTIDE\|NC_008536 | Solibacter usitatus Ellin6076, complete genome. |
| protein | REFSEQ_PROTEIN\|YP_828532 | REFSEQ_PROTEIN\|YP_828532 | FAD dependent oxidoreductase [Solibacter usitatus Ellin6076]. |
| dna | REFSEQ_PROTEIN\|YP_885073 | REFSEQ_NUCLEOTIDE\|NC_008596 | Mycobacterium smegmatis str. MC2 155, complete genome. |
| protein | REFSEQ_PROTEIN\|YP_885073 | REFSEQ_PROTEIN\|YP_885073 | FAD dependent oxidoreductase [Mycobacterium smegmatis str. MC2 155]. |
| dna | REFSEQ_PROTEIN\|YP_925628 | REFSEQ_NUCLEOTIDE\|NC_008699 | Nocardioides sp. JS614, complete genome. |
| protein | REFSEQ_PROTEIN\|YP_925628 | REFSEQ_PROTEIN\|YP_925628 | FAD dependent oxidoreductase [Nocardioides sp. JS614]. |
| dna | REFSEQ_PROTEIN\|YP_996730 | REFSEQ_NUCLEOTIDE\|NC_008786 | Verminephrobacter eiseniae EF01-2, complete genome. |
| protein | REFSEQ_PROTEIN\|YP_996730 | REFSEQ_PROTEIN\|YP_996730 | FAD dependent oxidoreductase [Verminephrobacter eiseniae EF01-2]. |
| dna | REFSEQ_PROTEIN\|ZP_00111616 | REFSEQ_NUCLEOTIDE\|NZ_AAAY02000005 | Nostoc punctiforme PCC 73102, unfinished sequence, whole genome shotgun sequence. |
| protein | REFSEQ_PROTEIN\|ZP_00111616 | REFSEQ_PROTEIN\|ZP_00111616 | COG1233: Phytoene dehydrogenase and related proteins [Nostoc punctiforme PCC 73102]. |
| dna | REFSEQ_PROTEIN\|ZP_00112486 | REFSEQ_NUCLEOTIDE\|NZ_AAAY02000001 | Nostoc punctiforme PCC 73102, unfinished sequence, whole genome shotgun sequence. |
| protein | REFSEQ_PROTEIN\|ZP_00112486 | REFSEQ_PROTEIN\|ZP_00112486 | COG1233: Phytoene dehydrogenase and related proteins [Nostoc punctiforme PCC 73102]. |
| dna | REFSEQ_PROTEIN\|ZP_00567490 | REFSEQ_NUCLEOTIDE\|NZ_AAII01000004 | Frankia sp. EAN1pec, unfinished sequence, whole genome shotgun sequence. |
| protein | REFSEQ_PROTEIN\|ZP_00567490 | REFSEQ_PROTEIN\|ZP_00567490 | beta-carotene ketolase [Frankia sp. EAN1pec]. |
| dna | REFSEQ_PROTEIN\|ZP_00768112 | REFSEQ_NUCLEOTIDE\|NZ_AAAH02000023 | Chloroflexus aurantiacus J-10-fl, unfinished sequence, whole genome shotgun sequence. |
| protein | REFSEQ_PROTEIN\|ZP_00768112 | REFSEQ_PROTEIN\|ZP_00768112 | Amine oxidase:FAD dependent oxidoreductase [Chloroflexus aurantiacus J-10-fl]. |
| dna | REFSEQ_PROTEIN\|ZP_00860549 | REFSEQ_NUCLEOTIDE\|NZ_AALJ01000005 | Bradyrhizobium sp. BTAi1, unfinished sequence, whole genome shotgun sequence. |
| protein | REFSEQ_PROTEIN\|ZP_00860549 | REFSEQ_PROTEIN\|ZP_00860549 | probable phytoene dehydrogenase [Bradyrhizobium sp. BTAi1]. |
| dna | REFSEQ_PROTEIN\|ZP_00914726 | REFSEQ_NUCLEOTIDE\|NZ_AAME01000021 | Rhodobacter sphaeroides ATCC 17025, unfinished sequence, whole genome shotgun sequence. |
| protein | REFSEQ_PROTEIN\|ZP_00914726 | REFSEQ_PROTEIN\|ZP_00914726 | phytoene dehydrogenase [Rhodobacter sphaeroides ATCC 17025]. |
| dna | REFSEQ_PROTEIN\|ZP_01034755 | REFSEQ_NUCLEOTIDE\|NZ_AAMV01000001 | Roseovarius sp. 217, unfinished sequence, whole genome shotgun sequence. |
| protein | REFSEQ_PROTEIN\|ZP_01034755 | REFSEQ_PROTEIN\|ZP_01034755 | phytoene dehydrogenase [Roseovarius sp. 217]. |
| dna | REFSEQ_PROTEIN\|ZP_01045675 | REFSEQ_NUCLEOTIDE\|NZ_AAMY01000004 | Nitrobacter sp. Nb-311A, unfinished sequence, whole genome shotgun sequence. |
| protein | REFSEQ_PROTEIN\|ZP_01045675 | REFSEQ_PROTEIN\|ZP_01045675 | FAD dependent oxidoreductase [Nitrobacter sp. Nb- 311A]. |
| dna | REFSEQ_PROTEIN\|ZP_01088978 | REFSEQ_NUCLEOTIDE\|NZ_AANZ01000002 | Blastopirellula marina DSM 3645, unfinished sequence, whole genome shotgun sequence. |
| protein | REFSEQ_PROTEIN\|ZP_01088978 | REFSEQ_PROTEIN\|ZP_01088978 | phytoene dehydrogenase-related protein [Blastopirellula marina DSM 3645]. |
| dna | REFSEQ_PROTEIN\|ZP_01104558 | REFSEQ_NUCLEOTIDE\|NZ_AAOA01000017 | gamma proteobacterium KT 71, unfinished sequence, whole genome shotgun sequence. |
| protein | REFSEQ_PROTEIN\|ZP_01104558 | REFSEQ_PROTEIN\|ZP_01104558 | phytoene dehydrogenase-related protein [gamma proteobacterium KT 71]. |
| dna | REFSEQ_PROTEIN\|ZP_01304444 | REFSEQ_NUCLEOTIDE\|NZ_AAQG01000017 | Sphingomonas sp. SKA58, unfinished sequence, whole genome shotgun sequence. |
| protein | REFSEQ_PROTEIN\|ZP_01304444 | REFSEQ_PROTEIN\|ZP_01304444 | FAD dependent oxidoreductase [Sphingomonas sp. SKA58]. |
| dna | REFSEQ_PROTEIN\|ZP_01356922 | REFSEQ_NUCLEOTIDE\|NZ_AAQU01000003 | Roseiflexus sp. RS-1, unfinished sequence, whole genome shotgun sequence. |
| protein | REFSEQ_PROTEIN\|ZP_01356922 | REFSEQ_PROTEIN\|ZP_01356922 | Amine oxidase:FAD dependent oxidoreductase [Roseiflexus sp. RS-1]. |
| dna | REFSEQ_PROTEIN\|ZP_01420973 | REFSEQ_NUCLEOTIDE\|NZ_AATH01000004 | Caulobacter sp. K31, unfinished sequence, whole genome shotgun sequence. |
| protein | REFSEQ_PROTEIN\|ZP_01420973 | REFSEQ_PROTEIN\|ZP_01420973 | FAD dependent oxidoreductase [Caulobacter sp. K31 ]. |
| dna | REFSEQ_PROTEIN\|ZP_01424586 | REFSEQ_NUCLEOTIDE\|NZ_AATI01000012 | Herpetosiphon aurantiacus ATCC 23779, unfinished sequence, whole genome shotgun sequence. |
| protein | REFSEQ_PROTEIN\|ZP_01424586 | REFSEQ_PROTEIN\|ZP_01424586 | FAD dependent oxidoreductase [Herpetosiphon aurantiacus ATCC 23779]. |
| dna | REFSEQ_PROTEIN\|ZP_01513930 | REFSEQ_NUCLEOTIDE\|NZ_AAUI01000001 | Chloroflexus aggregans DSM 9485, unfinished sequence, whole genome shotgun sequence. |
| protein | REFSEQ_PROTEIN\|ZP_01513930 | REFSEQ_PROTEIN\|ZP_01513930 | FAD dependent oxidoreductase [Chloroflexus aggregans DSM 9485]. |
| dna | REFSEQ_PROTEIN\|ZP_01529376 | REFSEQ_NUCLEOTIDE\|NZ_AAUM01000001 | Roseiflexus castenholzii DSM 13941, unfinished sequence, whole genome shotgun sequence. |
| protein | REFSEQ_PROTEIN\|ZP_01529376 | REFSEQ_PROTEIN\|ZP_01529376 | FAD dependent oxidoreductase [Roseiflexus castenholzii DSM 13941]. |
| dna | REFSEQ_PROTEIN\|ZP_01601027 | REFSEQ_NUCLEOTIDE\|NZ_AAVI01000006 | Metallosphaera sedula DSM 5348, unfinished sequence, whole genome shotgun sequence. |
| protein | REFSEQ_PROTEIN\|ZP_01601027 | REFSEQ_PROTEIN\|ZP_01601027 | FAD dependent oxidoreductase [Meta-Ilosphaera sedula DSM 5348]. |
| dna | REFSEQ_PROTEIN\|ZP_01608402 | REFSEQ_NUCLEOTIDE\|NZ_AAVK01000004 | Sphingomonas wittichii RW1, unfinished sequence, whole genome shotgun sequence. |
| protein | REFSEQ_PROTEIN\|ZP_01608402 | REFSEQ_PROTEIN\|ZP_01608402 | FAD dependent oxidoreductase [Sphingomonas wittichii RW1]. |
| dna | REFSEQ_PROTEIN\|ZP_01623956 | REFSEQ_NUCLEOTIDE\|NZ_AAVU01000053 | Lyngbya sp. PCC 8106, unfinished sequence, whole genome shotgun sequence. |
| protein | REFSEQ_PROTEIN\|ZP_01623956 | REFSEQ_PROTEIN\|ZP_01623956 | FAD dependent oxidoreductase [Lyngbya sp. PCC 8106]. |
| dna | REFSEQ_PROTEIN\|ZP_01626983 | REFSEQ_NUCLEOTIDE\|NZ_AAVV01000009 | marine gamma proteobacterium HTCC2080, unfinished sequence, whole genome shotgun sequence. |
| protein | REFSEQ_PROTEIN\|ZP_01626983 | REFSEQ_PROTEIN\|ZP_01626983 | FAD dependent oxidoreductase [marine gamma proteobacterium HTCC2080]. |
| dna | REFSEQ_PROTEIN\|ZP_01627857 | REFSEQ_NUCLEOTIDE\|NZ_AAVV01000019 | marine gamma proteobacterium HTCC2080, unfinished sequence, whole genome shotgun sequence. |
| protein | REFSEQ_PROTEIN\|ZP_01627857 | REFSEQ_PROTEIN\|ZP_01627857 | phytoene dehydrogenase [marine gamma proteobacterium HTCC2080]. |
| dna | REFSEQ_PROTEIN\|ZP_01628140 | REFSEQ_NUCLEOTIDE\|NZ_AAVW01000001 | Nodularia spumigena CCY9414, unfinished sequence, whole genome shotgun sequence. |
| protein | REFSEQ_PROTEIN\|ZP_01628140 | REFSEQ_PROTEIN\|ZP_01628140 | hypothetical protein N9414_21450 [Nodularia spumigena CCY9414]. |
| dna | REFSEQ_PROTEIN\|ZP_01644524 | REFSEQ_NUCLEOTIDE\|NZ_AAVZ01000033 | Stenotrophomonas maltophilia R551-3, unfinished sequence, whole genome shotgun sequence. |
| protein | REFSEQ_PROTEIN\|ZP_01644524 | REFSEQ_PROTEIN\|ZP_01644524 | amine oxidase [Stenotrophomonas maltophilia R551- 3]. |
| dna | REFSEQ_PROTEIN\|ZP_01670982 | REFSEQ_NUCLEOTIDE\|NZ_AAWM01000006 | Anaeromyxobacter sp. Fw109-5, unfinished sequence, whole genome shotgun sequence. |
| protein | REFSEQ_PROTEIN\|ZP_01670982 | REFSEQ_PROTEIN\|ZP_01670982 | FAD dependent oxidoreductase [Anaeromyxobacter sp. Fw109-5]. |
| protein | SPTREMBL\|Q5IKE4_RHOER | SPTREMBL\|Q5IKE4_RHOER | Beta-carotene ketolase. |
| protein | SPTREMBL\|Q8VYK3_ARATH | SPTREMBL\|Q8VYK3_ARATH | AT5g49550/K6M13_10. |
| protein | SPTREMBL\|Q9FGZ1_ARATH | SPTREMBL\|Q9FGZ1_ARATH | Phytoene dehydrogenase-like. |

## Claims

1. A process for the preparation of at least one carotenoid in genetically modified plants, which method comprises expressing in said plant at least one carotene ketolase enzyme encoded by an expression improved heterologous carotene ketolase coding sequence (improved ketolase sequence).

2. The process of claim 1, wherein at least on of the carotenoids is a ketocarotenoid

3. The process of claim 1, wherein the plant is of any of the following families:
Ranunculaceae, Berberidaceae, Begoniaceae, Papaveraceae, Cannabaceae, Chenopodiaceae, Cruciferae, Rosaceae, Fabaceae, Linaceae, Vitaceae, Brassiceae, Cucurbitaceae, Primulaceae, Caryophyllaceae, Amaranthaceae, Apocynaceae, Balsaminaceae, Gentianaceae, Geraniaceae, Graminae, Euphorbiaceae, Labiatae, Leguminosae, Caprifoliaceae, Oleaceae, Tropaeolaceae, Solanaceae, Lobeliaceae, Scrophulariaceae, Compositae, Asteraceae, Plumbaginaceae, Liliaceae, Amaryllidaceae, Rubiaceae, Poaceae, Polemoniaceae, Orchidaceae, Umbelliferae, Verbenaceae, Violaceae, Malvaceae, Illiaceae or Lamiaceae.

4. The process of any of the preceding claims, wherein said plant is selected from the genus Tagetes.

5. The process of any of the preceding claims, wherein a ketolase enzyme derived from the improved ketolase sequence is expressed in flowers of said plant.

6. The process of claim 5, wherein the ketolase enzyme derived from the improved ketolase sequence is expressed in petals of said plant.

7. The process of claim 6, wherein the ketolase enzyme derived from the improved ketolase sequence is targeted to plastids of said plant.

8. The process of claim 6, wherein the ketolase enzyme derived from the improved ketolase sequence is expressed in plastids of said plant.

9. The process of any of the preceding claims, wherein the improved ketolase sequence was modified by adapting the corresponding non-improved ketolase coding sequence to the codon usage of said plant or compartment of the plant such as tissue or plastid.

10. The process of claim 9, wherein at least one codon of the non-improved ketolase coding sequence was adapted to the most abundant codon for the same amino acid of the plant or compartment of the plant such as tissue or plastid.

11. The process of any of the preceding claims, wherein the non-improved ketolase sequence was modified by avoiding and/or removing signals and/or structures negatively interfering with expression efficiency in plants or plant plastids.

12. The process of any of the preceding claims, wherein the improved ketolase sequence is derived from the corresponding coding sequence from bacteria, yeast or algae.

13. The process of claim 12, wherein the improved ketolase sequence is derived from the corresponding coding sequences of algae of the genus Haematococcus, Chlamydomonas, Scenedesmus or Chlorella.

14. The process of claim 13, wherein the improved ketolase sequence is derived from the corresponding coding sequences of the species Haematococcus pluvialis, Chlamydomonas reinhardtii, Scenedesmus vacuolatus or Chlorella zoofingiensis.

15. The process of any of the preceding claims, wherein a ketolase enzyme is expressed having an amino acid sequence encoded by the corresponding coding portions comprised by a nucleotide sequence selected from SEQ ID NO: 3, 4, 7, 8, 10, 11, 13 and 14; or coding sequences derived there from by nucleic acid substitution, addition, deletion or insertion, and encoding a ketolase enzyme having a sequence identity of at least 50% with respect to the parent sequence and retaining ketolase activity.

16. The process of claim 15, wherein the improved ketolase sequence is selected from the corresponding coding portions comprised by a nucleotide sequence selected from SEQ ID NO: 3, 4, 7, 8, 10, 11, 13 and 14; or coding sequences derived there from by nucleic acid substitution, addition, deletion or insertion, and encoding a ketolase enzyme having a sequence identity of at least 50% with respect to the parent sequence and retaining ketolase activity.

17. The process of any of the preceding claims, wherein a genetically modified plant is employed which carries an expression construct encompassing at least one improved ketolase sequence under the control of suitable regulatory elements.

18. The process of claim 17, wherein the improved ketolase sequence is under the control of a plant specific promoter.

19. The process of claim 18, wherein the plant specific promoter is a tissue specific promoter.

20. The process of claim 19, wherein the tissue specific promoter directs petal specific expression.

21. The process of claim 18, wherein the plant specific promoter is a plastid specific promoter.

22. The process of any of the claims 17 to 20, wherein the expression construct further comprises the coding sequence for a transit peptide operably linked to the coding sequence of the improved ketolase sequence.

23. The process of claim 22, wherein the coding sequence for the transit peptide is also expression improved.

24. An expression construct as defined in any of the claims 17 to 23.

25. A recombinant vector comprising at least one expression construct as defined in claim 24.

26. A recombinant microorganism comprising at least one expression construct as defined in claim 24.

27. A genetically modified plant carrying at least one improved ketolase sequence as defined in any of the claims 1 to 17, or at least one expression construct as defined in claim 24, or at least one vector as defined in claim 25.

28. A genetically modified plant according to claim 27 expressing carotene ketolase activity in at least one plant tissue or at least one plastid.

29. A genetically modified plant according to claim 27 expressing carotene ketolase activity in its petals.

30. A genetically modified plant according to any of claim 27 to 29 having an altered carotenoid profile.

31. A genetically modified plant according to claim 30 having an altered carotenoid profile in petals and/or plastids thereof.

32. A genetically modified plant according to claim 30 or 31, containing a detectable amount of at least one ketocarotenoid in at least one plant tissue.

33. A genetically modified plant according to claim 30 or 31, containing a detectable amount of at least one ketocarotenoid in petals and/or at least one plastid thereof.

34. A genetically modified plant according to any of the claims 27 to 33, selected from a plant of the families as defined in claim 2.

35. Parts of a genetically modified plant as defined in any of the claims 27 to 34.

36. Seeds of a genetically modified plant as defined in any of the claims 27 to 34

37. A process for the preparation of a genetically modified plant as defined in any of the claims 27 to 34, which process comprises introducing at least one expression construct as defined in claim 24 into a starting plant.

38. A process for the preparation of parts of a genetically modified plant as defined in any of the claims 27 to 34, which process comprises introducing at least one expression construct as defined in claim 24 into a starting plant, growing the so obtained genetically modified plant and obtaining parts thereof.

39. A process for the preparation of seeds of a genetically modified plant as defined in any of the claims 27 to 34, which process comprises introducing at least one expression construct as defined in claim 24 into a starting plant, growing the so obtained plant and obtaining seeds thereof.

40. A process for the preparation of at least one carotenoid, which process comprises cultivating a genetically modified plant as defined in any of the claims 26 to 33 under conditions which allow the expression of improved ketolase activity for a sufficient time to produce a detectable amount of at least one carotenoid within the plant, and isolating said carotenoid thereof.

41. A process according to claim 40, wherein the carotenoid is a ketocarotenoid.

42. The use of a genetically modified plant as defined in any of the claims 27 to 34 for preparing carotenoids.

43. The use of a genetically modified plant as defined in claim 42 wherein at least one carotenoid is a ketocarotenoid.

44. An improved ketolase sequence as defined in any of the claims 9 to 16.


SEQUENCE LISTING

<110>  BASF Plant Science GmbH

<120>  Production of ketocarotenoids in plants

<130>  PF0000061565

<160>  14

<170>  PatentIn version 3.3

<210>  1
<211>  329
<212>  PRT
<213>  Haematococcus pluvialis

<400>  1

Met Gln Leu Ala Ala Thr Val Met Leu Glu Gln Leu Thr Gly Ser Ala
1               5                   10                  15

Glu Ala Leu Lys Glu Lys Glu Lys Glu Val Ala Gly Ser Ser Asp Val
            20                  25                  30

Leu Arg Thr Trp Ala Thr Gln Tyr Ser Leu Pro Ser Glu Glu Ser Asp
        35                  40                  45

Ala Ala Arg Pro Gly Leu Lys Asn Ala Tyr Lys Pro Pro Pro Ser Asp
    50                  55                  60

Thr Lys Gly Ile Thr Met Ala Leu Ala Val Ile Gly Ser Trp Ala Ala
65                  70                  75                  80

Val Phe Leu His Ala Ile Phe Gln Ile Lys Leu Pro Thr Ser Leu Asp
                85                  90                  95

Gln Leu His Trp Leu Pro Val Ser Asp Ala Thr Ala Gln Leu Val Ser
            100                 105                 110

Gly Ser Ser Ser Leu Leu His Ile Val Val Val Phe Phe Val Leu Glu
        115                 120                 125

Phe Leu Tyr Thr Gly Leu Phe Ile Thr Thr His Asp Ala Met His Gly
    130                 135                 140

Thr Ile Ala Met Arg Asn Arg Gln Leu Asn Asp Phe Leu Gly Arg Val
145                 150                 155                 160

Cys Ile Ser Leu Tyr Ala Trp Phe Asp Tyr Asn Met Leu His Arg Lys
                165                 170                 175

His Trp Glu His His Asn His Thr Gly Glu Val Gly Lys Asp Pro Asp
            180                 185                 190

Phe His Arg Gly Asn Pro Gly Ile Val Pro Trp Phe Ala Ser Phe Met
        195                 200                 205

Ser Ser Tyr Met Ser Met Trp Gln Phe Ala Arg Leu Ala Trp Trp Thr
    210                 215                 220

Val Val Met Gln Leu Leu Gly Ala Pro Met Ala Asn Leu Leu Val Phe
225                 230                 235                 240

Met Ala Ala Ala Pro Ile Leu Ser Ala Phe Arg Leu Phe Tyr Phe Gly
                245                 250                 255

Thr Tyr Met Pro His Lys Pro Glu Pro Gly Ala Ala Ser Gly Ser Ser
            260                 265                 270

Pro Ala Val Met Asn Trp Trp Lys Ser Arg Thr Ser Gln Ala Ser Asp
        275                 280                 285

Leu Val Ser Phe Leu Thr Cys Tyr His Phe Asp Leu His Trp Glu His
    290                 295                 300

His Arg Trp Pro Phe Ala Pro Trp Trp Glu Leu Pro Asn Cys Arg Arg
305                 310                 315                 320

Leu Ser Gly Arg Gly Leu Val Pro Ala
                325

<210>  2
<211>  57
<212>  PRT
<213>  Pisum sativum

<400>  2

Met Ala Ser Met Ile Ser Ser Ser Ala Val Thr Thr Val Ser Arg Ala
1               5                   10                  15

Ser Thr Val Gln Ser Ala Ala Val Ala Pro Phe Gly Gly Leu Lys Ser
            20                  25                  30

Met Thr Gly Phe Pro Val Lys Lys Val Asn Thr Asp Ile Thr Ser Ile
        35                  40                  45

Thr Ser Asn Gly Gly Arg Val Lys Cys
    50                  55

<210>  3
<211>  1158
<212>  DNA
<213>  Artificial

<220>
<223>  synthetic fragment of TP-RbcS and HP BKT

<400>  3
atggcatcta tgattagttc tagcgctgtg actacagtta gtcgggcatc tacagttcaa      60

agtgcggctg tagcaccttt tggtggactc aagtcaatga ctgggtttcc cgtgaagaaa     120

gtcaacactg acatcacttc gataactagc aatggtggtc gcgtgaaaat gcagttggca     180

gccacagtta tgttagaaca actcaccggt tccgctgagg cattgaaaga aaaagaaaag     240

gaagtagcag ggagttcaga tgtacttagg acatgggcta ctcagtattc actgccatca     300

gaagaatcag atgcggctag accaggtctg aagaatgcgt acaagccacc tccgtcggac     360

acaaagggca ttacaatggc attagcagtg attggtagct gggctgctgt ctttctccat     420

gccatttttc aaatcaaact gcctacgtct ttagatcagc tacattggct tcccgttagt     480

gatgcgacgg cacaacttgt ttcaggatca tcctctttgc ttcatattgt tgtcgttttc     540

tttgtgcttg agtttctcta tacgggactg tttatcacta cccatgatgc aatgcacggc     600

actatcgcca tgcggaaccg tcaactcaat gatttccttg gtagagtttg catatctctc 660

tatgcttggt ttgactacaa catgctacac aggaaacatt gggaacatca caatcataca 720

ggggaggtgg gtaaagatcc cgatttccat agagggaacc cagggattgt tccttggttt 780

gcttcattca tgagttctta tatgagcatg tggcaatttg ccagacttgc ctggtggaca 840

gttgtcatgc agttgctcgg tgctccaatg gctaatctac tggttttcat ggctgctgca 900

cctatacttt cagctttccg acttttctac tttggaacct atatgcctca caaaccggaa 960

cctggtgcag cctcaggctc tagtcctgcg gtaatgaact ggtggaagtc ccgtacctcg 1020

caagcatccg acttagtgtc ttttctaacc tgttaccatt ttgatttgca ttgggagcac 1080

catagatggc cattcgctcc ttggtgggag cttccgaatt gcagaaggct tagcggacga 1140

ggtcttgttc cagcttag 1158

<210> 4
<211> 4470
<212> DNA
<213> Artificial

<220>
<223> VC-SIW122-13

<400> 4
   gtgattttgt gccgagctgc cggtcgggga gctgttggct ggctggtggc aggatatatt 60
   gtggtgtaaa caaattgacg cttagacaac ttaataacac attgcggacg tctttaatgt 120
   actgaattaa catccgtttg atacttgtct aaaattggct gatttcgagt gcatctatgc 180
   ataaaaacaa tctaatgaca attattacca agcagctgat catgagcgga gaattaaggg 240
   agtcacgtta tgacccccgc cgatgacgcg ggacaagccg ttttacgttt ggaactgaca 300
   gaaccgcaac gttgaaggag ccactcagcc gcgggtttct ggagtttaat gagctaagca 360
   catacgtcag aaaccattat tgcgcgttca aaagtcgcct aaggtcacta tcagctagca 420
   aatatttctt gtcaaaaatg ctccactgac gttccataaa ttcccctcgg tatccaatta 480
   gagtctcata ttcactctca atccaaataa tctcgacatg tctccggaga ggagaccagt 540
   tgagattagg ccagctacag cagccgatat ggccgcggtt tgtgacatcg ttaaccatta 600
   cattgagacg tctacagtga actttaggac agagccacaa acaccacaag agtggattga 660
   tgacctagag aggttgcaag atagataccc ttggttggtt gctgaggttg agggtgttgt 720
   ggctggtatt gcttacgctg ggccctggaa ggctaggaac gcttacgatt ggacagttga 780
   gagtactgtt tacgtgtcac ataggcatca aaggttgggc ctaggatcta cattgtacac 840
   acatttgctt aagtctatgg aggcgcaagg ttttaagtct gtggttgctg ttataggcct 900
   tccaaacgat ccatctgtta ggttgcatga ggctttggga tacacagcgc ggggtacatt 960
   gcgcgcggct ggatacaagc atggtggatg gcatgatgtt ggtttttggc aaagggattt 1020
   tgagttgcca gctcctccaa ggccagttag gccagttacc cagatctgag tcgatcgacc 1080
   gatcttgctg cgttcggata ttttcgtgga gttcccgcca cagacccgga tgatccccga 1140
   tcgttcaaac atttggcaat aaagtttctt aagattgaat cctgttgccg gtcttgcgat 1200
   gattatcata taatttctgt tgaattacgt taagcatgta ataattaaca tgtaatgcat 1260
   gacgttattt atgagatggg tttttatgat tagagtcccg caattataca tttaatacgc 1320
   gatagaaaac aaaatatagc gcgcaaacta ggataaatta tcgcgcgcgg tgtcatctat 1380
   gttactagat cgggcctgag tcgttgtaaa acgacggcca gtgaattatc caactttgta 1440
   taataaagtt gccatgatta cgccaagctt gcatgccgtc gaccagatct gatatctgcg 1500
   gccgcctcga gcatatgcta gaggatcccc gggtacccca ctttgtacaa gaaagctggg 1560
   tccatgatta gccaagcttg catgccgtcg accagatctg atatctgcgg ccgcctcgag 1620
   catatgctag aggatccccg ggtaccagcc tgcttttttg tacaaacttg ggtaccgagc 1680
   tcggatccac tagtaacggc cgccagtgtg ctggaattcg cccttataag cttaggctcg 1740
   tgtcctcttt gcaaccgtcc gatcattgag actcttgaca tattttgaaa ccaatcgaat 1800
   ggctgtaaat tctccatcta acacaaagtt tttttacagt ttgttgcatt tcgtttgtta 1860
   tggaaaagtg atgtaaattt caaaagaaaa aaaatacaag agattctgtt aacaaggccc 1920
   atttttagtc aattataagc ccaataaaca aatagtttgg acgtttcctc tcaatcgcgc 1980
   tttccaaaac gctgtcaaga tatttatcgc atggtagcag atctcgaccg ttaaaggacc 2040
   aaagtagttg gcaaactgag cgataatcag aaaacggcca gacaatagta cactgccgaa 2100
   gtttctagca tcggtgtcat ttgcatggcg cctaattctc gtggagaagt agtagagata 2160
   ataaagagca actttcctaa acaatgattt ctgtctatcc ctaaatatat ttttttttgt 2220
   tttttgcaga gaaactttct tttcgtttta ttagtacttt ccatgtgcat acagaaacaa 2280
   tgtattttgt ttatttcaaa atttcgcaaa gtacaaaagc atttaaaggg cattatgtac 2340
   cattaatata taacaaaaga caacaattta atgagctcta tgatttcagt ttgaagatat 2400
   aatcatggca ttgaatcaca tcgtgattat aggttacgat tatgtgattt ttctgtgtgt 2460
   aaaattgcac tcctagtttc agaatccctg actaatcctt tatgcttttt gaaaattagc 2520
   cagcatgtgt caaattggat tttcttgcca taaacaagag ccaaaaagcc caaaacaatt 2580
   atcgcctcac accacgatgc ttaagatgtc tgaatatcgt cctttgatct taccaattta 2640
   tgtggctaac atttattacc cctcgtgatt tggtttgaga ttacgccatt tgccaaatca 2700
   gcagtaatga tggctataaa ttagtagtca gtgtatgtac atttttcttg ttcactagct 2760
   tcgagtcgac tgcaattcat acagaagtga gaaaaatggc atctatgatt agttctagcg 2820
   ctgtgactac agttagtcgg gcatctacag ttcaaagtgc ggctgtagca ccttttggtg 2880
   gactcaagtc aatgactggg tttcccgtga agaaagtcaa cactgacatc acttcgataa 2940
   ctagcaatgg tggtcgcgtg aaatgtatgc agttggcagc cacagttatg ttagaacaac 3000
   tcaccggttc cgctgaggca ttgaaagaaa aagaaaagga agtagcaggg agttcagatg 3060
   tacttaggac atgggctact cagtattcac tgccatcaga agaatcagat gcggctagac 3120
   caggtctgaa gaatgcgtac aagccacctc cgtcggacac aaagggcatt acaatggcat 3180
   tagcagtgat tggtagctgg gctgctgtct ttctccatgc catttttcaa atcaaactgc 3240
   ctacgtcttt agatcagcta cattggcttc ccgttagtga tgcgacggca caacttgttt 3300
   caggatcatc ctctttgctt catattgttg tcgttttctt tgtgcttgag tttctctata 3360
   cgggactgtt tatcactacc catgatgcaa tgcacggcac tatcgccatg cggaaccgtc 3420
   aactcaatga tttccttggt agagtttgca tatctctcta tgcttggttt gactacaaca 3480
   tgctacacag gaaacattgg gaacatcaca atcatacagg ggaggtgggt aaagatcccg 3540
   atttccatag agggaaccca gggattgttc cttggtttgc ttcattcatg agttcttata 3600
   tgagcatgtg gcaatttgcc agacttgcct ggtggacagt tgtcatgcag ttgctcggtg 3660
   ctccaatggc taatctactg gttttcatgg ctgctgcacc tatactttca gctttccgac 3720
   ttttctactt tggaacctat atgcctcaca aaccggaacc tggtgcagcc tcaggctcta 3780
   gtcctgcggt aatgaactgg tggaagtccc gtacctcgca agcatccgac ttagtgtctt 3840
   ttctaacctg ttaccatttt gatttgcatt gggagcacca tagatggcca ttcgctcctt 3900
   ggtgggagct tccgaattgc agaaggctta gcggacgagg tcttgttcca gcttagggat 3960
   ccactagtga ttgcggccgc tagactatac tatgttttag cctgcctgct ggctagctac 4020
   tatgttatgt tatgttgtaa aataaacacc tgctaaggta tatctatcta tattttagca 4080
   tggctttctc aataaattgt ctttccttat cgtttactat cttataccta ataatgaaat 4140
   aataatatca catatgagga acggggcagg tttaggcata tatatacgag tgtagggcgg 4200
   agtggtttat cagatctggt cgacggcatg caagcttggc gtaatcatgg caacttttct 4260
   atacaaagtt ggatggcatg caagcttggc gtaatcatgg tcatagctgt ttcctactag 4320
   atctgattgt cgtttcccgc cttcagttta aactatcagt gtttgacagg atatattggc 4380
   gggtaaacct aagagaaaag agcgtttatt agaataatcg gatatttaaa agggcgtgaa 4440
   aaggtttatc cgttcgtcca tttgtatgtc 4470

<210> 5
<211> 5916
<212> DNA
<213> Artificial

<220>
<223> VC-LLL544-1qcz

<220>
<221> misc_feature
<222> (5906)..(5916)
<223> n is a, c, g, or t

<400> 5
ctgcttggta ataattgtca ttagattgtt tttatgcata gatgcactcg aaatcagcca 60
attttagaca agtatcaaac ggatgttaat tcagtacatt aaagacgtcc gcaatgtgtt 120
attaagttgt ctaagcgtca atttgtttac accacaatat atcctgccac cagccagcca 180
acagctcccc gaccggcagc tcggcacaaa atcaccacgc gttaccacca cgccggccgg 240
ccgcatggtg ttgaccgtgt tcgccggcat tgccgagttc gagcgttccc taatcatcga 300
ccgcacccgg agcgggcgcg aggccgccaa ggcccgaggc gtgaagtttg gcccccgccc 360
taccctcacc ccggcacaga tcgcgcacgc ccgcgagctg atcgaccagg aaggccgcac 420
cgtgaaagag gcggctgcac tgcttggcgt gcatcgctcg accctgtacc gcgcacttga 480
gcgcagcgag gaagtgacgc ccaccgaggc caggcggcgc ggtgccttcc gtgaggacgc 540
attgaccgag gccgacgccc tggcggccgc cgagaatgaa cgccaagagg aacaagcatg 600
aaaccgcacc aggacggcca ggacgaaccg tttttcatta ccgaagagat cgaggcggag 660
atgatcgcgg ccgggtacgt gttcgagccg cccgcgcacg tctcaaccgt gcggctgcat 720
gaaatcctgg ccggtttgtc tgatgccaag ctggcggcct ggccggccag cttggccgct 780
gaagaaaccg agcgccgccg tctaaaaagg tgatgtgtat ttgagtaaaa cagcttgcgt 840
catgcggtcg ctgcgtatat gatgcgatga gtaaataaac aaatacgcaa ggggaacgca 900
tgaaggttat cgctgtactt aaccagaaag gcgggtcagg caagacgacc atcgcaaccc 960
atctagcccg cgccctgcaa ctcgccgggg ccgatgttct gttagtcgat tccgatcccc 1020

agggcagtgc ccgcgattgg gcggccgtgc gggaagatca accgctaacc gttgtcggca 1080
tcgaccgccc gacgattgac cgcgacgtga aggccatcgg ccggcgcgac ttcgtagtga 1140
tcgacggagc gccccaggcg gcggacttgg ctgtgtccgc gatcaaggca gccgacttcg 1200
tgctgattcc ggtgcagcca agcccttacg acatatgggc caccgccgac ctggtggagc 1260
tggttaagca gcgcattgag gtcacggatg gaaggctaca agcggccttt gtcgtgtcgc 1320
gggcgatcaa aggcacgcgc atcggcggtg aggttgccga ggcgctggcc gggtacgagc 1380
tgcccattct tgagtcccgt atcacgcagc gcgtgagcta cccaggcact gccgccgccg 1440
gcacaaccgt tcttgaatca gaacccgagg gcgacgctgc ccgcgaggtc caggcgctgg 1500
ccgctgaaat taaatcaaaa ctcatttgag ttaatgaggt aaagagaaaa tgagcaaaag 1560
cacaaacacg ctaagtgccg gccgtccgag cgcacgcagc agcaaggctg caacgttggc 1620
cagcctggca gacacgccag ccatgaagcg ggtcaacttt cagttgccgg cggaggatca 1680
caccaagctg aagatgtacg cggtacgcca aggcaagacc attaccgagc tgctatctga 1740
atacatcgcg cagctaccag agtaaatgag caaatgaata aatgagtaga tgaattttag 1800
cggctaaagg aggcggcatg gaaaatcaag aacaaccagg caccgacgcc gtggaatgcc 1860
ccatgtgtgg aggaacgggc ggttggccag gcgtaagcgg ctgggttgtc tgccggccct 1920
gcaatggcac tggaaccccc aagcccgagg aatcggcgtg agcggtcgca aaccatccgg 1980
cccggtacaa atcggcgcgg cgctgggtga tgacctggtg gagaagttga aggccgcgca 2040
ggccgcccag cggcaacgca tcgaggcaga agcacgcccc ggtgaatcgt ggcaagcggc 2100
cgctgatcga atccgcaaag aatcccggca accgccggca gccggtgcgc cgtcgattag 2160
gaagccgccc aagggcgacg agcaaccaga ttttttcgtt ccgatgctct atgacgtggg 2220
cacccgcgat agtcgcagca tcatggacgt ggccgttttc cgtctgtcga agcgtgaccg 2280
acgagctggc gaggtgatcc gctacgagct tccagacggg cacgtagagg tttccgcagg 2340
gccggccggc atggccagtg tgtgggatta cgacctggta ctgatggcgg tttcccatct 2400
aaccgaatcc atgaaccgat accgggaagg gaagggagac aagcccggcc gcgtgttccg 2460
tccacacgtt gcggacgtac tcaagttctg ccggcgagcc gatggcggaa agcagaaaga 2520
cgacctggta gaaacctgca ttcggttaaa caccacgcac gttgccatgc agcgtacgaa 2580
gaaggccaag aacggccgcc tggtgacggt atccgagggt gaagccttga ttagccgcta 2640
caagatcgta aagagcgaaa ccgggcggcc ggagtacatc gagatcgagc tagctgattg 2700
gatgtaccgc gagatcacag aaggcaagaa cccggacgtg ctgacggttc accccgatta 2760
ctttttgatc gatcccggca tcggccgttt tctctaccgc ctggcacgcc gcgccgcagg 2820
caaggcagaa gccagatggt tgttcaagac gatctacgaa cgcagtggca gcgccggaga 2880
gttcaagaag ttctgtttca ccgtgcgcaa gctgatcggg tcaaatgacc tgccggagta 2940
cgatttgaag gaggaggcgg ggcaggctgg cccgatccta gtcatgcgct accgcaacct 3000
gatcgagggc gaagcatccg ccggttccta atgtacggag cagatgctag ggcaaattgc 3060
cctagcaggg gaaaaaggtc gaaaaggtct ctttcctgtg gatagcacgt acattgggaa 3120
cccaaagccg tacattggga accggaaccc gtacattggg aacccaaagc cgtacattgg 3180
gaaccggtca cacatgtaag tgactgatat aaaagagaaa aaaggcgatt tttccgccta 3240
aaactcttta aaacttatta aaactcttaa aacccgcctg gcctgtgcat aactgtctgg 3300
ccagcgcaca gccgaagagc tgcaaaaagc gcctaccctt cggtcgctgc gctccctacg 3360

ccccgccgct tcgcgtcggc ctatcgcggc cgctggccgc tcaaaaatgg ctggcctacg 3420
gccaggcaat ctaccagggc gcggacaagc cgcgccgtcg ccactcgacc gccggcgccc 3480
acatcaaggc accctgcctc gcgcgtttcg gtgatgacgg tgaaaacctc tgacacatgc 3540
agctcccgga gacggtcaca gcttgtctgt aagcggatgc cgggagcaga caagcccgtc 3600
agggcgcgtc agcgggtgtt ggcgggtgtc ggggcgcagc catgacccag tcacgtagcg 3660
atagcggagt gtatactggc ttaactatgc ggcatcagag cagattgtac tgagagtgca 3720
ccatatgcgg tgtgaaatac cgcacagatg cgtaaggaga aaataccgca tcaggcgctc 3780
ttccgcttcc tcgctcactg actcgctgcg ctcggtcgtt cggctgcggc gagcggtatc 3840
agctcactca aaggcggtaa tacggttatc cacagaatca ggggataacg caggaaagaa 3900
catgtgagca aaaggccagc aaaaggccag gaaccgtaaa aaggccgcgt tgctggcgtt 3960
tttccatagg ctccgccccc ctgacgagca tcacaaaaat cgacgctcaa gtcagaggtg 4020
gcgaaacccg acaggactat aaagatacca ggcgtttccc cctggaagct ccctcgtgcg 4080
ctctcctgtt ccgaccctgc cgcttaccgg atacctgtcc gcctttctcc cttcgggaag 4140
cgtggcgctt tctcatagct cacgctgtag gtatctcagt tcggtgtagg tcgttcgctc 4200
caagctgggc tgtgtgcacg aaccccccgt tcagcccgac cgctgcgcct tatccggtaa 4260
ctatcgtctt gagtccaacc cggtaagaca cgacttatcg ccactggcag cagccactgg 4320
taacaggatt agcagagcga ggtatgtagg cggtgctaca gagttcttga agtggtggcc 4380
taactacggc tacactagaa ggacagtatt tggtatctgc gctctgctga agccagttac 4440
cttcggaaaa agagttggta gctcttgatc cggcaaacaa accaccgctg gtagcggtgg 4500
tttttttgtt tgcaagcagc agattacgcg cagaaaaaaa ggatctcaag aagatccttt 4560
gatcttttct acggggtctg acgctcagtg gaacgaaaac tcacgttaag ggattttggt 4620
catgcatgat atatctccca atttgtgtag ggcttattat gcacgcttaa aaataataaa 4680
agcagacttg acctgatagt ttggctgtga gcaattatgt gcttagtgca tctaacgctt 4740
gagttaagcc gcgccgcgaa gcggcgtcgg cttgaacgaa tttctagcta gacattattt 4800
gccgactacc ttggtgatct cgcctttcac gtagtggaca aattcttcca actgatctgc 4860
gcgcgaggcc aagcgatctt cttcttgtcc aagataagcc tgtctagctt caagtatgac 4920
gggctgatac tgggccggca ggcgctccat tgcccagtcg gcagcgacat ccttcggcgc 4980
gattttgccg gttactgcgc tgtaccaaat gcgggacaac gtaagcacta catttcgctc 5040
atcgccagcc cagtcgggcg gcgagttcca tagcgttaag gtttcattta gcgcctcaaa 5100
tagatcctgt tcaggaaccg gatcaaagag ttcctccgcc gctggaccta ccaaggcaac 5160
gctatgttct cttgcttttg tcagcaagat agccagatca atgtcgatcg tggctggctc 5220
gaagatacct gcaagaatgt cattgcgctg ccattctcca aattgcagtt cgcgcttagc 5280
tggataacgc cacggaatga tgtcgtcgtg cacaacaatg gtgacttcta cagcgcggag 5340
aatctcgctc tctccagggg aagccgaagt ttccaaaagg tcgttgatca aagctcgccg 5400
cgttgtttca tcaagcctta cggtcaccgt aaccagcaaa tcaatatcac tgtgtggctt 5460
caggccgcca tccactgcgg agccgtacaa atgtacggcc agcaacgtcg gttcgagatg 5520
gcgctcgatg acgccaacta cctctgatag ttgagtcgat acttcggcga tcaccgcttc 5580
ccccatgatg tttaactttg ttttagggcg actgccctgc tgcgtaacat cgttgctgct 5640

ccataacatc aaacatcgac ccacggcgta acgcgcttgc tgcttggatg cccgaggcat 5700

agactgtacc ccaaaaaaac agtcataaca agccatgaaa accgccactg cgttccatgg 5760

acatacaaat ggacgaacgg ataaaccttt tcacgccctt ttaaatatcc gattattcta 5820

ataaacgctc ttttctctta ggtttacccg ccaatatatc ctgtcaaaca ctgatagttt 5880

aaactgaagg cgggaaacga caatcnnnnn nnnnnn 5916

<210> 6
<211> 331
<212> PRT
<213> Scenedesmus vacuolatus

<400> 6

Met Ala Pro Arg Arg Gln Ser Thr Leu Pro Gln Gln Thr Lys Ala Gly
1               5                   10                  15

Ser Pro Thr Ser Gly Ser Asp Ala Ala Ile Pro Glu Pro Asp Val Ile
            20                  25                  30

Asp Val Trp Lys Ala Gln Tyr Pro Leu Pro Asp Glu Asn Val Ala Gly
        35                  40                  45

Ser Met Asn Glu Val Lys Gln Leu Tyr Arg Pro Pro Arg Asn Asp Val
    50                  55                  60

Lys Gly Ile Ser Ile Ala Leu Gly Leu Ile Ala Ala Trp Cys Val Leu
65                  70                  75                  80

Phe Tyr His Gly Cys Trp Gln Ile Gln Leu Ser Gly Ser Gln Arg Ser
                85                  90                  95

Trp Trp Ile Asp Ile Ala Gly Thr Phe Ile Leu Leu Glu Phe Val Asn
            100                 105                 110

Thr Gly Leu Phe Ile Thr Thr His Asp Ala Met His Gly Thr Val Cys
        115                 120                 125

Tyr Arg Asn Arg Lys Leu Asn Asp Leu Leu Gly Arg Ile Ala Ile Thr
    130                 135                 140

Leu Tyr Ala Trp Phe Asp Tyr Asp Met Leu His Arg Lys His Trp Glu
145                 150                 155                 160

His His Asn Tyr Thr Gly Gln Lys Gly Lys Asp Pro Asp Phe His Arg
                165                 170                 175

Gly Asn Pro Ala Leu Pro Val Trp Tyr Ala Arg Phe Met Trp Glu Tyr
            180                 185                 190

Ser Thr Pro Leu Gln Phe Ala Lys Ile Ile Leu Val Ser Gln Val Leu
        195                 200                 205

Gln Ala Leu Gly Val Pro Tyr Asn Asn Leu Cys Val Tyr Met Ala Ala
    210                 215                 220

Ala Pro Leu Val Ala Ala Phe Arg Leu Phe Tyr Phe Gly Thr Tyr Leu
225                 230                 235                 240

Pro His Leu Pro Pro Asn Ala Gln Glu Val Met Val Trp Gln Lys Ser
                245                 250                 255

His Ser Ser Asp Ala Pro Ser Trp Leu Ser Phe Leu Lys Cys Tyr His
            260                 265                 270

Phe Asp Tyr His Trp Glu His His Arg Trp Pro Tyr Ala Pro Trp Trp
        275                 280                 285

Glu Leu Pro Lys Ala Lys Lys Ile Thr Gln Gln Thr Gln His His Gln
    290                 295                 300

Gln Thr Lys Gln Gln Gln Pro Met Gln Gln Ala Lys Ala Gln Val Val
305                 310                 315                 320

Ser Gln Leu Ala Pro Ala Gly Ala Val Val Glu
                325                 330

<210>  7
<211>  1167
<212>  DNA
<213>  Artificial

<220>
<223>  synthetic fragment TP-RbcS/SV211 BKT

<400>  7
atggcctcaa tgatctcaag ttcagctgtt actacagtct caagagcaag taccgttcaa      60

agtgctgcag ttgctccatt tggtggactt aagagcatga ctggttttcc agtgaagaaa     120

gtgaacacag acattacgtc cataacctca aatggtggac gtgttaaatg tatggctcca     180

agaaggcaat ctacacttcc acaacagact aaagctggtt ctcctacttc aggatcagat     240

gcagctatac ctgaaccaga tgtcatagat gtatggaagg ctcaatatcc tcttcctgat     300

gagaatgtag ctggaagcat gaatgaggtc aagcagcttt atagacctcc aagaaatgac     360

gtgaaaggca tctcgattgc attaggactc attgctgcat ggtgtgtact tttctatcac     420

ggttgttggc agattcaact aagtgggagt caaagatcat ggtggataga cattgctggc     480

acttttatcc tcctggaatt tgtgaatacc ggtttgttca tcacgactca tgatgcgatg     540

catggaacag tgtgttacag gaatcgtaag ctcaatgatc tactcggaag aattgccatc     600

acactttacg cttggtttga ttacgatatg cttcatcgga aacattggga gcaccataac     660

tatacaggac agaaaggaaa agacccagat tttcacagag gtaatcctgc tcttccagta     720

tggtatgcca gatttatgtg ggagtattct actccactac aattcgcgaa gattatactc     780

gtttcgcaag ttcttcaagc tcttggtgtt ccctacaaca atctgtgcgt gtatatggca     840

gctgctccac ttgttgctgc atttcgactg ttctattttg gtacatacct accacatctt     900

cctccaaatg cacaagaagt tatggtttgg cagaagtctc attcctctga tgcaccttct     960

tggctatcct tcctcaaatg ctaccacttt gattaccatt gggaacatca cagatggcct    1020

tatgcaccat ggtgggaact tcctaaggct aaaaagatta cccagcaaac acagcatcat    1080

caacagacga aacagcaaca accaatgcag caagcaaaag ctcaagtggt ttctcaactt    1140

gcaccagcag gagctgttgt cgaatag                                        1167

<210>  8
<211>  4476

<212>  DNA
<213>  Artificial

<220>
<223>  VC-SIW182-6

<400>  8
gtgattttgt gccgagctgc cggtcgggga gctgttggct ggctggtggc aggatatatt      60

gtggtgtaaa caaattgacg cttagacaac ttaataacac attgcggacg tctttaatgt     120

actgaattaa catccgtttg atacttgtct aaaattggct gatttcgagt gcatctatgc     180

ataaaaacaa tctaatgaca attattacca agcagctgat catgagcgga gaattaaggg     240

agtcacgtta tgacccccgc cgatgacgcg ggacaagccg ttttacgttt ggaactgaca     300

gaaccgcaac gttgaaggag ccactcagcc gcgggtttct ggagtttaat gagctaagca     360

catacgtcag aaaccattat tgcgcgttca aaagtcgcct aaggtcacta tcagctagca     420

aatatttctt gtcaaaaatg ctccactgac gttccataaa ttcccctcgg tatccaatta     480

gagtctcata ttcactctca atccaaataa tctcgacatg tctccggaga ggagaccagt     540

tgagattagg ccagctacag cagccgatat ggccgcggtt tgtgacatcg ttaaccatta     600

cattgagacg tctacagtga actttaggac agagccacaa acaccacaag agtggattga     660

tgacctagag aggttgcaag atagataccc ttggttggtt gctgaggttg agggtgttgt     720

ggctggtatt gcttacgctg ggccctggaa ggctaggaac gcttacgatt ggacagttga     780

gagtactgtt tacgtgtcac ataggcatca aaggttgggc ctaggatcta cattgtacac     840

acatttgctt aagtctatgg aggcgcaagg ttttaagtct gtggttgctg ttataggcct     900

tccaaacgat ccatctgtta ggttgcatga ggctttggga tacacagcgc ggggtacatt     960

gcgcgcggct ggatacaagc atggtggatg gcatgatgtt ggtttttggc aaagggattt    1020

tgagttgcca gctcctccaa ggccagttag gccagttacc cagatctgag tcgatcgacc    1080

gatcttgctg cgttcggata ttttcgtgga gttcccgcca cagacccgga tgatccccga    1140

tcgttcaaac atttggcaat aaagtttctt aagattgaat cctgttgccg gtcttgcgat    1200

gattatcata taatttctgt tgaattacgt taagcatgta ataattaaca tgtaatgcat    1260

gacgttattt atgagatggg tttttatgat tagagtcccg caattataca tttaatacgc    1320

gatagaaaac aaaatatagc gcgcaaacta ggataaatta tcgcgcgcgg tgtcatctat    1380

gttactagat cgggcctgag tcgttgtaaa acgacggcca gtgaattatc caactttgta    1440

taataaagtt gccatgatta cgccaagctt gcatgccgtc gaccagatct gatatctgcg    1500

gccgcctcga gcatatgcta gaggatcccc gggtacccca ctttgtacaa gaaagctggg    1560

tggtaccggg ccccccctcg agtttaaacc actccgccct acactcgtat atatatgcct    1620

aaacctgccc cgttcctcat atgtgatatt attatttcat tattaggtat aagatagtaa    1680

acgataagga aagacaattt attgagaaag ccatgctaaa atatagatag atatacctta    1740

gcaggtgttt attttacaac ataacataac atagtagcta gccagcaggc aggctaaaac    1800

atagtatagt ctatctgcat gctcgagcgg ccgcctattc gacaacagct cctgctggtg    1860

caagttgaga aaccacttga gcttttgctt gctgcattgg ttgttgctgt ttcgtctgtt    1920

gatgatgctg tgtttgctgg gtaatctttt tagccttagg aagttcccac catggtgcat    1980

aaggccatct gtgatgttcc caatggtaat caaagtggta gcatttgagg aaggatagcc    2040

aagaaggtgc atcagaggaa tgagacttct gccaaaccat aacttcttgt gcatttggag    2100

gaagatgtgg taggtatgta ccaaaataga acagtcgaaa tgcagcaaca agtggagcag 2160
ctgccatata cacgcacaga ttgttgtagg gaacaccaag agcttgaaga acttgcgaaa 2220
cgagtataat cttcgcgaat tgtagtggag tagaatactc ccacataaat ctggcatacc 2280
atactggaag agcaggatta cctctgtgaa aatctgggtc ttttcctttc tgtcctgtat 2340
agttatggtg ctcccaatgt ttccgatgaa gcatatcgta atcaaaccaa gcgtaaagtg 2400
tgatggcaat tcttccgagt agatcattga gcttacgatt cctgtaacac actgttccat 2460
gcatcgcatc atgagtcgtg atgaacaaac cggtattcac aaattccagg aggataaaag 2520
tgccagcaat gtctatccac catgatcttt gactcccact tagttgaatc tgccaacaac 2580
cgtgatagaa aagtacacac catgcagcaa tgagtcctaa tgcaatcgag atgcctttca 2640
cgtcatttct tggaggtcta taaagctgct tgacctcatt catgcttcca gctacattct 2700
catcaggaag aggatattga gccttccata catctatgac atctggttca ggtatagctg 2760
catctgatcc tgaagtagga gaaccagctt tagtctgttg tggaagtgta gattgccttc 2820
ttggagccat acatttaaca cgtccaccat ttgaggttat ggacgtaatg tctgtgttca 2880
ctttcttcac tggaaaacca gtcatgctct taagtccacc aaatggagca actgcagcac 2940
tttgaacggt acttgctctt gagactgtag taacagctga acttgagatc attgaggcca 3000
tttttctcac ttctgtatga attgcagata tctcgaagct agtgaacaag aaaaatgtac 3060
atacactgac tactaattta tagccatcat tactgctgat ttggcaaatg gcgtaatctc 3120
aaaccaaatc acgaggggta ataaatgtta gccacataaa ttggtaagat caaaggacga 3180
tattcagaca tcttaagcat cgtggtgtga ggcgataatt gttttgggct ttttggctct 3240
tgtttatggc aagaaaatcc aatttgacac atgctggcta attttcaaaa agcataaagg 3300
attagtcagg gattctgaaa ctaggagtgc aattttacac acagaaaaat cacataatcg 3360
taacctataa tcacgatgtg attcaatgcc atgattatat cttcaaactg aaatcataga 3420
gctcattaaa ttgttgtctt ttgttatata ttaatggtac ataatgccct ttaaatgctt 3480
ttgtactttg cgaaattttg aaataaacaa aatacattgt ttctgtatgc acatggaaag 3540
tactaataaa cgaaaaagaa agtttctctg caaaaaacaa aaaaaaatat atttagggat 3600
agacagaaat cattgtttag gaaagttgct ctttattatc tctactactt ctccacgaga 3660
attaggcgcc atgcaaatga caccgatgct agaaacttcg gcagtgtact attgtctggc 3720
cgttttctga ttatcgctca gtttgccaac tactttggtc ctttaacggt cgagatctgc 3780
taccatgcga taaatatctt gacagcgttt tggaaagcgc gattgagagg aaacgtccaa 3840
actatttgtt tattgggctt ataattgact aaaaatgggc cttgttaaca gaatctcttg 3900
tatttttttt cttttgaaat ttacatcact tttccataac aaacgaaatg caacaaactg 3960
taaaaaaact ttgtgttaga tggagaattt acagccattc gattggtttc aaaatatgtc 4020
aagagtctca atgatcggac ggttgcaaag aggacacgag cctctgcagc gcaagggcga 4080
attccagcac actggcggcc gttactagtg gatccgagct cggtaccaag cttggcgtaa 4140
tcatggagcc tgcttttttg tacaaacttg ccatgattac gccaagcttg catgccgtcg 4200
accagatctg atatctgcgg ccgcctcgag catatgctag aggatccccg ggtacccaac 4260
ttttctatac aaagttggat ggcatgcaag cttggcgtaa tcatggtcat agctgtttcc 4320
tactagatct gattgtcgtt tcccgccttc agtttaaact atcagtgttt gacaggatat 4380
attggcgggt aaacctaaga gaaaagagcg tttattagaa taatcggata tttaaaaggg 4440

cgtgaaaagg tttatccgtt cgtccatttg tatgtc                                4476

<210>  9
<211>  312
<212>  PRT
<213>  Chlorella zofingiensis

<400>  9

Met Ala Pro Asp Val Thr His Val Gln Pro Arg Val Gln Ser Pro Ala
1               5                   10                  15

Gly Pro Asp Asp Glu Asp Asp Ala Leu Ser Leu Trp Lys Ala Gln Tyr
            20                  25                  30

Pro Met Pro Glu Glu Lys Gly Thr Val Ser Lys Pro Gln Ala Ala Leu
        35                  40                  45

Lys Tyr Arg Pro Pro Arg Ser Asp Trp Lys Gly Val Ser Ile Ala Cys
    50                  55                  60

Thr Val Ile Thr Leu Trp Thr Ala Val Phe Tyr His Gly Cys Trp Gln
65                  70                  75                  80

Ile Lys Leu Thr Gly Pro Asp Lys Ser Ala Trp Trp Asp Val Val Ala
                85                  90                  95

Thr Phe Leu Ala Leu Glu Phe Leu Asn Thr Gly Leu Phe Ile Thr Thr
            100                 105                 110

His Asp Ala Met His Gly Thr Ile Ala Ile Arg Asn Arg Arg Leu Asn
        115                 120                 125

Asp Leu Leu Gly Asn Ile Ala Ile Ser Leu Tyr Ala Trp Phe Asp Tyr
    130                 135                 140

Asp Met Leu His Lys Lys His Trp Glu His His Asn Phe Thr Gly Leu
145                 150                 155                 160

Pro His Lys Asp Pro Asp Phe His Arg Gly Asp Pro Ala Leu His Lys
                165                 170                 175

Trp Phe Gly Arg Phe Met Trp Glu Tyr Ala Thr Pro Leu Gln Phe Ala
            180                 185                 190

Lys Ile Phe Ala Tyr Pro Phe Phe Leu Gln Ser Leu Arg Val Gln Tyr
        195                 200                 205

Pro Asn Leu Cys Val Phe Leu Ala Ala Ala Pro Leu Val Ser Ala Phe
    210                 215                 220

Arg Leu Phe Tyr Phe Gly Thr Tyr Leu Pro His Leu Pro Ser Asn Ala
225                 230                 235                 240

Gln Glu Thr Met Pro Trp Glu Lys Ser His Ser Ala Asp Asp Pro Arg
                245                 250                 255

Pro Leu Ser Phe Leu Lys Cys Tyr His Phe Asp Tyr His Trp Glu His
            260                 265                 270

His Arg Trp Pro Tyr Ala Pro Trp Trp Glu Leu Pro Val Cys Lys Arg
        275                 280                 285

Ile Thr Lys Thr Leu Asp Ala Ala Val Pro Gly Val Gln Ser Asp Gly
    290                 295                 300

Thr Lys Lys Ser Gln Leu Val Asn
305                 310

<210>  10
<211>  1110
<212>  DNA
<213>  Artificial

<220>
<223>  synthetic fragment TP-RbcS/CZ BKT

<400>  10
atggcctcta tgatctcgtc tagtgcagtg actacagtct caagagcatc cacagttcaa      60

tcagcagctg ttgctccgtt tggtggtctg aagtctatga ctggatttcc cgtgaagaaa     120

gtgaacacgg atataacgtc cattacctcg aatggaggta gagtgaagtg tatggcacct     180

gatgtgacac atgttcagcc tagagttcaa agtcctgctg gtccagatga tgaggatgac     240

gctttgtctc tttggaaagc ccaatatcct atgcctgagg aaaagggtac agtatcgaaa     300

ccacaagcag ctctgaagta cagaccacct agatcagatt ggaaaggcgt ttcaattgcc     360

tgtactgtta tcacgctttg gactgctgtg ttctatcatg gttgttggca aatcaaactg     420

actggaccag ataagagtgc atggtgggac gttgttgcaa ctttcctagc gttggaattt     480

ctcaatactg ggcttttcat taccactcat gatgctatgc atggaaccat tgctatccga     540

aatcgcagac tgaatgacct tcttgggaat attgcgatca gcttgtatgc ttggttcgac     600

tacgatatgc tgcacaagaa acattgggaa catcacaact ttacaggact tccacacaaa     660

gatccagact ttcacagagg agatcctgca ctacacaaat ggtttggcag atttatgtgg     720

gagtatgcta caccattgca attcgcgaaa atcttcgcct atcccttctt tctccaaagt     780

ctcagagtac agtacccaaa cctatgcgtc tttcttgcag ctgcaccact tgttagcgca     840

tttcgactct tttactttgg gacatacttg cctcatttgc catctaatgc gcaagaaaca     900

atgccttggg aaaagtccca ttcagctgat gatccaagac ctcttagctt cttgaaatgt     960

tatcactttg actatcattg ggagcatcat agatggcctt atgcaccttg gtgggaactt    1020

cctgtatgca aacggattac caagacacta gatgctgctg taccaggtgt ccaatcagat    1080

gggaccaaaa agagccaatt ggttaactag                                     1110

<210>  11
<211>  4411
<212>  DNA
<213>  Artificial

<220>
<223>  VC-SIW198-1

<400>  11
gtgattttgt gccgagctgc cggtcgggga gctgttggct ggctggtggc aggatatatt      60

gtggtgtaaa caaattgacg cttagacaac ttaataacac attgcggacg tctttaatgt     120

actgaattaa catccgtttg atacttgtct aaaattggct gatttcgagt gcatctatgc     180

ataaaaacaa tctaatgaca attattacca agcagctgat catgagcgga gaattaaggg 240
agtcacgtta tgacccccgc cgatgacgcg ggacaagccg ttttacgttt ggaactgaca 300
gaaccgcaac gttgaaggag ccactcagcc gcgggtttct ggagtttaat gagctaagca 360
catacgtcag aaaccattat tgcgcgttca aaagtcgcct aaggtcacta tcagctagca 420
aatatttctt gtcaaaaatg ctccactgac gttccataaa ttcccctcgg tatccaatta 480
gagtctcata ttcactctca atccaaataa tctcgacatg tctccggaga ggagaccagt 540
tgagattagg ccagctacag cagccgatat ggccgcggtt tgtgacatcg ttaaccatta 600
cattgagacg tctacagtga actttaggac agagccacaa acaccacaag agtggattga 660
tgacctagag aggttgcaag atagataccc ttggttggtt gctgaggttg agggtgttgt 720
ggctggtatt gcttacgctg ggccctggaa ggctaggaac gcttacgatt ggacagttga 780
gagtactgtt tacgtgtcac ataggcatca aaggttgggc ctaggatcta cattgtacac 840
acatttgctt aagtctatgg aggcgcaagg ttttaagtct gtggttgctg ttataggcct 900
tccaaacgat ccatctgtta ggttgcatga ggctttggga tacacagcgc ggggtacatt 960
gcgcgcggct ggatacaagc atggtggatg gcatgatgtt ggtttttggc aaagggattt 1020
tgagttgcca gctcctccaa ggccagttag gccagttacc cagatctgag tcgatcgacc 1080
gatcttgctg cgttcggata ttttcgtgga gttcccgcca cagacccgga tgatccccga 1140
tcgttcaaac atttggcaat aaagtttctt aagattgaat cctgttgccg gtcttgcgat 1200
gattatcata taatttctgt tgaattacgt taagcatgta ataattaaca tgtaatgcat 1260
gacgttattt atgagatggg tttttatgat tagagtcccg caattataca tttaatacgc 1320
gatagaaaac aaaatatagc gcgcaaacta ggataaatta tcgcgcgcgg tgtcatctat 1380
gttactagat cgggcctgag tcgttgtaaa acgacggcca gtgaattatc caactttgta 1440
taataaagtt gccatgatta cgccaagctt gcatgccgtc gaccagatct gatatctgcg 1500
gccgcctcga gcatatgcta gaggatcccc gggtacccca ctttgtacaa gaaagctggg 1560
tggtaccggg ccccccctcg agtttaaacc actccgccct acactcgtat atatatgcct 1620
aaacctgccc cgttcctcat atgtgatatt attatttcat tattaggtat aagatagtaa 1680
acgataagga aagacaattt attgagaaag ccatgctaaa atatagatag atatacctta 1740
gcaggtgttt attttacaac ataacataac atagtagcta gccagcaggc aggctaaaac 1800
atagtatagt ctatctgcat gcctagttaa ccaattggct ctttttggtc ccatctgatt 1860
ggacacctgg tacagcagca tctagtgtct tggtaatccg tttgcataca ggaagttccc 1920
accaaggtgc ataaggccat ctatgatgct cccaatgata gtcaaagtga taacatttca 1980
agaagctaag aggtcttgga tcatcagctg aatgggactt ttcccaaggc attgtttctt 2040
gcgcattaga tggcaaatga ggcaagtatg tcccaaagta aaagagtcga aatgcgctaa 2100
caagtggtgc agctgcaaga aagacgcata ggtttgggta ctgtactctg agactttgga 2160
gaaagaaggg ataggcgaag attttcgcga attgcaatgg tgtagcatac tcccacataa 2220
atctgccaaa ccatttgtgt agtgcaggat ctcctctgtg aaagtctgga tctttgtgtg 2280
gaagtcctgt aaagttgtga tgttcccaat gtttcttgtg cagcatatcg tagtcgaacc 2340
aagcatacaa gctgatcgca atattcccaa gaaggtcatt cagtctgcga tttcggatag 2400
caatggttcc atgcatagca tcatgagtgg taatgaaaag cccagtattg agaaattcca 2460
acgctaggaa agttgcaaca acgtcccacc atgcactctt atctggtcca gtcagtttga 2520

tttgccaaca accatgatag aacacagcag tccaaagcgt gataacagta caggcaattg 2580

aaacgccttt ccaatctgat ctaggtggtc tgtacttcag agctgcttgt ggtttcgata 2640

ctgtaccctt ttcctcaggc ataggatatt gggctttcca aagagacaaa gcgtcatcct 2700

catcatctgg accagcagga ctttgaactc taggctgaac atgtgtcaca tcaggtgcca 2760

tacacttcac tctacctcca ttcgaggtaa tggacgttat atccgtgttc actttcttca 2820

cgggaaatcc agtcatagac ttcagaccac caaacggagc aacagctgct gattgaactg 2880

tggatgctct tgagactgta gtcactgcac tagacgagat catagaggcc atttttctca 2940

cttctgtatg aattgcatct agatatctcg aagctagtga acaagaaaaa tgtacataca 3000

ctgactacta atttatagcc atcattactg ctgatttggc aaatggcgta atctcaaacc 3060

aaatcacgag gggtaataaa tgttagccac ataaattggt aagatcaaag gacgatattc 3120

agacatctta agcatcgtgg tgtgaggcga taattgtttt gggctttttg gctcttgttt 3180

atggcaagaa aatccaattt gacacatgct ggctaatttt caaaaagcat aaaggattag 3240

tcagggattc tgaaactagg agtgcaattt tacacacaga aaaatcacat aatcgtaacc 3300

tataatcacg atgtgattca atgccatgat tatatcttca aactgaaatc atagagctca 3360

ttaaattgtt gtcttttgtt atatattaat ggtacataat gccctttaaa tgcttttgta 3420

ctttgcgaaa ttttgaaata aacaaaatac attgtttctg tatgcacatg gaaagtacta 3480

ataaacgaaa aagaaagttt ctctgcaaaa aacaaaaaaa aatatattta gggatagaca 3540

gaaatcattg tttaggaaag ttgctcttta ttatctctac tacttctcca cgagaattag 3600

gcgccatgca aatgacaccg atgctagaaa cttcggcagt gtactattgt ctggccgttt 3660

tctgattatc gctcagtttg ccaactactt tggtccttta acggtcgaga tctgctacca 3720

tgcgataaat atcttgacag cgttttggaa agcgcgattg agaggaaacg tccaaactat 3780

ttgtttattg ggcttataat tgactaaaaa tgggccttgt taacagaatc tcttgtattt 3840

tttttctttt gaaatttaca tcacttttcc ataacaaacg aaatgcaaca aactgtaaaa 3900

aaactttgtg ttagatggag aatttacagc cattcgattg gtttcaaaat atgtcaagag 3960

tctcaatgat cggacggttg caaagaggac acgagcctct gcagcgcaag ggcgaattcc 4020

agcacactgg cggccgttac tagtggatcc gagctcggta ccaagcttgg cgtaatcatg 4080

gagcctgctt ttttgtacaa acttgccatg attacgccaa gcttgcatgc cgtcgaccag 4140

atctgatatc tgcggccgcc tcgagcatat gctagaggat ccccgggtac ccaacttttc 4200

tatacaaagt tggatggcat gcaagcttgg cgtaatcatg gtcatagctg tttcctacta 4260

gatctgattg tcgtttcccg ccttcagttt aaactatcag tgtttgacag gatatattgg 4320

cgggtaaacc taagagaaaa gagcgtttat tagaataatc ggatatttaa aagggcgtga 4380

aaaggtttat ccgttcgtcc atttgtatgt c 4411

<210> 12
<211> 328
<212> PRT
<213> Chlamydomonas reinhardtii

<400> 12

Met Gly Pro Gly Ile Gln Pro Thr Ser Ala Arg Pro Cys Ser Arg Thr
1               5                   10                  15

Lys His Ser Arg Phe Ala Leu Leu Ala Ala Ala Leu Thr Ala Arg Arg
            20                  25                  30

Val Lys Gln Phe Thr Lys Gln Phe Arg Ser Arg Arg Met Ala Glu Asp
        35                  40                  45

Ile Leu Lys Leu Trp Gln Arg Gln Tyr His Leu Pro Arg Glu Asp Ser
    50                  55                  60

Asp Lys Arg Thr Leu Arg Glu Arg Val His Leu Tyr Arg Pro Pro Arg
65                  70                  75                  80

Ser Asp Leu Gly Gly Ile Ala Val Ala Val Thr Val Ile Ala Leu Trp
                85                  90                  95

Ala Thr Leu Phe Val Tyr Gly Leu Trp Phe Val Lys Leu Pro Trp Ala
            100                 105                 110

Leu Lys Val Gly Glu Thr Ala Thr Ser Trp Ala Thr Ile Ala Ala Val
        115                 120                 125

Phe Phe Ser Leu Glu Phe Leu Tyr Thr Gly Leu Phe Ile Thr Thr His
    130                 135                 140

Asp Ala Met His Gly Thr Ile Ala Leu Arg Asn Arg Arg Leu Asn Asp
145                 150                 155                 160

Phe Leu Gly Gln Leu Ala Ile Ser Leu Tyr Ala Trp Phe Asp Tyr Ser
                165                 170                 175

Val Leu His Arg Lys His Trp Glu His His Asn His Thr Gly Glu Pro
            180                 185                 190

Arg Val Asp Pro Asp Phe His Arg Gly Asn Pro Asn Leu Ala Val Trp
        195                 200                 205

Phe Ala Gln Phe Met Val Ser Tyr Met Thr Leu Ser Gln Phe Leu Lys
    210                 215                 220

Ile Ala Val Trp Ser Asn Leu Leu Leu Leu Ala Gly Ala Pro Leu Ala
225                 230                 235                 240

Asn Gln Leu Leu Phe Met Thr Ala Ala Pro Ile Leu Ser Ala Phe Arg
                245                 250                 255

Leu Phe Tyr Tyr Gly Thr Tyr Val Pro His His Pro Glu Lys Gly His
            260                 265                 270

Thr Gly Ala Met Pro Trp Gln Val Ser Arg Thr Ser Ser Ala Ser Arg
        275                 280                 285

Leu Gln Ser Phe Leu Thr Cys Tyr His Phe Asp Leu His Trp Glu His
    290                 295                 300

His Arg Trp Pro Tyr Ala Pro Trp Trp Glu Leu Pro Lys Cys Arg Gln
305                 310                 315                 320

Ile Ala Arg Gly Ala Ala Leu Ala

                325

<210>  13
<211>  1158
<212>  DNA
<213>  Artificial

<220>
<223>  synthetic fragment TP-RbcS/CR BKT

<400>  13
atggcgagta tgatctcatc tagtgcagtt acaaccgttt caagagcttc aactgtgcaa      60

tctgctgcag tagctccatt tggtggcttg aaaagcatga caggttttcc tgtcaagaaa     120

gttaacaccg acattacgag cattacgtct aatggaggga gagtcaaatg tatgggacca     180

ggaatacagc caacaagtgc tcgtccttgt tctaggacta agcactctag gtttgccctt     240

ttagctgcag ctctaactgc taggagagtt aagcagttta ctaagcaatt ccggtcaaga     300

agaatggctg aggatatcct caaactgtgg cagagacaat atcaccttcc tagagaagat     360

tccgataaac gaaccttaag ggaaagagta cacctctata gaccacctag aagtgatcta     420

ggtggaattg ctgttgctgt tacagtgatt gctctttggg ctactttgtt cgtttacggt     480

ctttggtttg taaaacttcc ctgggcactt aaagttgggg aaacagcaac ttcttgggca     540

acaatagctg cagttttttt tagccttgag ttcctttata ctggcctctt cattacaacc     600

catgatgcaa tgcatggaac aatcgcactg agaaatcgca gattgaacga ctttcttggt     660

caacttgcca tctcacttta cgcatggttt gattactcgg ttctgcatcg aaagcattgg     720

gaacatcaca atcatactgg agaaccaaga gttgacccag attttcacag aggaaaccca     780

aatctagctg tttggtttgc gcaattcatg gtctcataca tgactctaag ccagtttctc     840

aagattgctg tttggtcgaa tcttctcctt cttgctggtg cacctctggc taaccaactc     900

ttgtttatga cagctgcacc aatactttcg gcattcagac tcttctatta cggtacgtat     960

gtacctcatc atccagaaaa gggtcataca ggagctatgc catggcaagt ttcaaggacg    1020

agttcagctt caagactcca gtcctttcta acctgctatc acttcgatct acattgggaa    1080

catcatcgat ggccttatgc tccttggtgg gaactgccta aatgcagaca aattgctcgc    1140

ggtgcagctt tagcttag                                                  1158

<210>  14
<211>  4471
<212>  DNA
<213>  Artificial

<220>
<223>  VC-SIW195-1

<400>  14
gtgattttgt gccgagctgc cggtcgggga gctgttggct ggctggtggc aggatatatt      60

gtggtgtaaa caaattgacg cttagacaac ttaataacac attgcggacg tctttaatgt     120

actgaattaa catccgtttg atacttgtct aaaattggct gatttcgagt gcatctatgc     180

ataaaaacaa tctaatgaca attattacca agcagctgat catgagcgga gaattaaggg     240

agtcacgtta tgacccccgc cgatgacgcg ggacaagccg ttttacgttt ggaactgaca     300

gaaccgcaac gttgaaggag ccactcagcc gcgggtttct ggagtttaat gagctaagca     360

catacgtcag aaaccattat tgcgcgttca aaagtcgcct aaggtcacta tcagctagca     420

aatatttctt gtcaaaaatg ctccactgac gttccataaa ttcccctcgg tatccaatta     480

gagtctcata ttcactctca atccaaataa tctcgacatg tctccggaga ggagaccagt 540
tgagattagg ccagctacag cagccgatat ggccgcggtt tgtgacatcg ttaaccatta 600
cattgagacg tctacagtga actttaggac agagccacaa acaccacaag agtggattga 660
tgacctagag aggttgcaag atagataccc ttggttggtt gctgaggttg agggtgttgt 720
ggctggtatt gcttacgctg ggccctggaa ggctaggaac gcttacgatt ggacagttga 780
gagtactgtt tacgtgtcac ataggcatca aaggttgggc ctaggatcta cattgtacac 840
acatttgctt aagtctatgg aggcgcaagg ttttaagtct gtggttgctg ttataggcct 900
tccaaacgat ccatctgtta ggttgcatga ggctttggga tacacagcgc ggggtacatt 960
gcgcgcggct ggatacaagc atggtggatg gcatgatgtt ggtttttggc aaagggattt 1020
tgagttgcca gctcctccaa ggccagttag gccagttacc cagatctgag tcgatcgacc 1080
gatcttgctg cgttcggata ttttcgtgga gttcccgcca cagacccgga tgatccccga 1140
tcgttcaaac atttggcaat aaagtttctt aagattgaat cctgttgccg gtcttgcgat 1200
gattatcata taatttctgt tgaattacgt taagcatgta ataattaaca tgtaatgcat 1260
gacgttattt atgagatggg tttttatgat tagagtcccg caattataca tttaatacgc 1320
gatagaaaac aaaatatagc gcgcaaacta ggataaatta tcgcgcgcgg tgtcatctat 1380
gttactagat cgggcctgag tcgttgtaaa acgacggcca gtgaattatc caactttgta 1440
taataaagtt gccatgatta cgccaagctt gcatgccgtc gaccagatct gatatctgcg 1500
gccgcctcga gcatatgcta gaggatcccc gggtacccca ctttgtacaa gaaagctggg 1560
tggtaccggg ccccccctcg agtttaaacc actccgccct acactcgtat atatatgcct 1620
aaacctgccc cgttcctcat atgtgatatt attatttcat tattaggtat aagatagtaa 1680
acgataagga aagacaattt attgagaaag ccatgctaaa atatagatag atatacctta 1740
gcaggtgttt attttacaac ataacataac atagtagcta gccagcaggc aggctaaaac 1800
atagtatagt ctatctgcat gctcgagcgg ccgcctaagc taaagctgca ccgcgagcaa 1860
tttgtctgca tttaggcagt tcccaccaag gagcataagg ccatcgatga tgttcccaat 1920
gtagatcgaa gtgatagcag gttagaaagg actggagtct tgaagctgaa ctcgtccttg 1980
aaacttgcca tggcatagct cctgtatgac ccttttctgg atgatgaggt acatacgtac 2040
cgtaatagaa gagtctgaat gccgaaagta ttggtgcagc tgtcataaac aagagttggt 2100
tagccagagg tgcaccagca agaaggagaa gattcgacca aacagcaatc ttgagaaact 2160
ggcttagagt catgtatgag accatgaatt gcgcaaacca aacagctaga tttgggtttc 2220
ctctgtgaaa atctgggtca actcttggtt ctccagtatg attgtgatgt tcccaatgct 2280
ttcgatgcag aaccgagtaa tcaaaccatg cgtaaagtga gatggcaagt tgaccaagaa 2340
agtcgttcaa tctgcgattt ctcagtgcga ttgttccatg cattgcatca tgggttgtaa 2400
tgaagaggcc agtataaagg aactcaaggc taaaaaaaac tgcagctatt gttgcccaag 2460
aagttgctgt ttccccaact ttaagtgccc agggaagttt tacaaaccaa agaccgtaaa 2520
cgaacaaagt agcccaaaga gcaatcactg taacagcaac agcaattcca cctagatcac 2580
ttctaggtgg tctatagagg tgtactcttt cccttaaggt tcgtttatcg gaatcttctc 2640
taggaaggtg atattgtctc tgccacagtt tgaggatatc ctcagccatt cttcttgacc 2700
ggaattgctt agtaaactgc ttaactctcc tagcagttag agctgcagct aaaagggcaa 2760

acctagagtg cttagtccta gaacaaggac gagcacttgt tggctgtatt cctggtccca 2820
tacatttgac tctccctcca ttagacgtaa tgctcgtaat gtcggtgtta actttcttga 2880
caggaaaacc tgtcatgctt ttcaagccac caaatggagc tactgcagca gattgcacag 2940
ttgaagctct tgaaacggtt gtaactgcac tagatgagat catactcgcc atttttctca 3000
cttctgtatg aattgcatct agatatctcg aagctagtga acaagaaaaa tgtacataca 3060
ctgactacta atttatagcc atcattactg ctgatttggc aaatggcgta atctcaaacc 3120
aaatcacgag gggtaataaa tgttagccac ataaattggt aagatcaaag gacgatattc 3180
agacatctta agcatcgtgg tgtgaggcga taattgtttt gggctttttg gctcttgttt 3240
atggcaagaa aatccaattt gacacatgct ggctaatttt caaaaagcat aaaggattag 3300
tcagggattc tgaaactagg agtgcaattt tacacacaga aaaatcacat aatcgtaacc 3360
tataatcacg atgtgattca atgccatgat tatatcttca aactgaaatc atagagctca 3420
ttaaattgtt gtcttttgtt atatattaat ggtacataat gccctttaaa tgcttttgta 3480
ctttgcgaaa ttttgaaata aacaaaatac attgtttctg tatgcacatg gaaagtacta 3540
ataaacgaaa aagaaagttt ctctgcaaaa aacaaaaaaa aatatattta gggatagaca 3600
gaaatcattg tttaggaaag ttgctcttta ttatctctac tacttctcca cgagaattag 3660
gcgccatgca aatgacaccg atgctagaaa cttcggcagt gtactattgt ctggccgttt 3720
tctgattatc gctcagtttg ccaactactt tggtccttta acggtcgaga tctgctacca 3780
tgcgataaat atcttgacag cgttttggaa agcgcgattg agaggaaacg tccaaactat 3840
ttgtttattg ggcttataat tgactaaaaa tgggccttgt taacagaatc tcttgtattt 3900
tttttctttt gaaatttaca tcacttttcc ataacaaacg aaatgcaaca aactgtaaaa 3960
aaactttgtg ttagatggag aatttacagc cattcgattg gtttcaaaat atgtcaagag 4020
tctcaatgat cggacggttg caaagaggac acgagcctct gcagcgcaag ggcgaattcc 4080
agcacactgg cggccgttac tagtggatcc gagctcggta ccaagcttgg cgtaatcatg 4140
gagcctgctt ttttgtacaa acttgccatg attacgccaa gcttgcatgc cgtcgaccag 4200
atctgatatc tgcggccgcc tcgagcatat gctagaggat ccccgggtac ccaacttttc 4260
tatacaaagt tggatggcat gcaagcttgg cgtaatcatg gtcatagctg tttcctacta 4320
gatctgattg tcgtttcccg ccttcagttt aaactatcag tgtttgacag gatatattgg 4380
cgggtaaacc taagagaaaa gagcgtttat tagaataatc ggatatttaa aagggcgtga 4440
aaaggtttat ccgttcgtcc atttgtatgt c 4471